(19) Europäisches Patentamt
European Patent Office
Office européen des brevets

(11) EP 1 043 307 B1

(12) **EUROPÄISCHE PATENTSCHRIFT**

(45) Veröffentlichungstag und Bekanntmachung des Hinweises auf die Patenterteilung:
**04.06.2003 Patentblatt 2003/23**

(21) Anmeldenummer: **00104477.5**

(22) Anmeldetag: **08.03.2000**

(51) Int Cl.[7]: **C07C 217/74**, C07C 215/42, C07C 215/64, C07C 215/70, C07D 317/58, C07D 307/52, C07C 323/32, C07D 333/20, A61K 31/235, A61K 31/137, C07D 295/08, C07C 229/38, C07C 255/59, C07C 215/28

(54) **3-Amino-3-arylpropan-1-ol-Derivate, deren Herstellung und Verwendung**

3-amino-3-arylpropan-1-ol-derivates, their preparation and their use

Dérivés 3-Amino-3-arylpropan-1-ol leur préparation et leur usage

(84) Benannte Vertragsstaaten:
**AT BE CH CY DE DK ES FI FR GB GR IE IT LI LU MC NL PT SE**
Benannte Erstreckungsstaaten:
**LT LV SI**

(30) Priorität: **07.04.1999 DE 19915601**

(43) Veröffentlichungstag der Anmeldung:
**11.10.2000 Patentblatt 2000/41**

(73) Patentinhaber: **Grünenthal GmbH**
**52078 Aachen (DE)**

(72) Erfinder:
- **Sundermann, Bernd, Dr.**
**52072 Aachen (DE)**
- **Kögel, Babette-Yvonne, Dr.**
**52379 Langerwehe-Hamich (DE)**
- **Hennies, Hagen-Heinrich, Dr.**
**52152 Simmerath (DE)**
- **Buschmann, Helmut, Dr.**
**52066 Aachen (DE)**

(56) Entgegenhaltungen:
**EP-A- 1 043 306**

- **MOELM, DETLEF ET AL: "Fragmentation reactions of quaternized.gamma.-amino alcohols. Diastereoselective synthesis of highly functionalized oxetanes and unsaturated aldehydes and ketones with a (Z)-C:C double bond" EUR. J. ORG. CHEM., Nr. 10, 1998, Seiten 2185-2191, XP000993116**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 043 307 B1

Printed by Jouve, 75001 PARIS (FR)

## Beschreibung

Die Erfindung betrifft substituierte 3-Amino-3-arylpropan-1-ole, der allgemeinen Formel I,

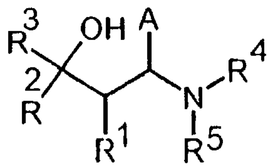

I

worin

$R^1$, $R^2$ ,jeweils unabhängig voneinander, $C_{1-6}$-Alkyl oder $R^1$ und $R^2$ zusammen einen $(CH_2)_{2-6}$-Ring bilden, der auch phenylsubstituiert sein kann,

$R^3$ $C_{3-6}$-Alkyl, $C_{3-6}$-Cycloalkyl, Aryl mit gegebenenfalls Heteroatomen im Ringsystem und den Substituenten $R^6$ bis $R^8$ am Arylring, oder ein substitiertes $C_{1-3}$-Alkylphenyl der Formel XII,

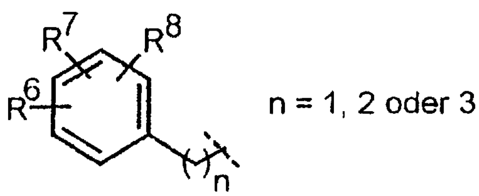

XII,

$R^4$, $R^5$ ,jeweils unabhängig voneinander, $C_{1-6}$Alkyl, $C_{3-6}$-Cycloalkyl, Phenyl, Benzyl, Phenethyl oder $R^4$ und $R^5$ zusammen einen $(CH_2)_{3-6}$- oder $CH_2CH_2OCH_2CH_2$-Ring bilden,

$R^6$ bis $R^8$ ,jeweils unabhängig voneinander, H, F, Cl, Br, $CHF_2$, $CF_3$, OH, $OCF_3$, $OR^{14}$, $NR^{15}R^{16}$, $SR^{14}$, Phenyl, $SO_2CH_3$, $SO_2CF_3$, $C_{1-6}$-Alkyl, CN, $COOR^{14}$, $CONR^{15}R^{16}$ oder $R^6$ und $R^7$ zusammen einen $OCH_2O$, $OCH_2CH_2O$, CH=CHO, $CH=C(CH_3)O$ oder $(CH_2)_4$-Ring bilden, worin

$R^{14}$ $C_{1-6}$-Alkyl, Phenyl, Benzyl, Phenethyl und

$R^{15}$, $R^{16}$ , jeweils unabhängig voneinander, H, $C_{1-6}$- Alkyl, Phenyl, Benzyl, Phenethyl, und

A ein Arylrest, der gegebenenfalls Heteroatome im Ringsystem aufweist und/oder substituiert sein kann,

bedeuten,
und deren Diastereomere oder Enantiomere in Form ihrer Basen oder Salze physiologisch verträglicher Säuren, wobei 1-Benzyl-2-(dimethylaminophenylmethyl)-cyclohexanol, dessen Diastereomere und dessen Enantiomere in Form ihrer Base und dessen Umsetzungsprodukt mit Methyljodid ausgenommen sind, sowie deren Herstellung und Verwendung als Arzneimittel.

Die Behandlung chronischer und nicht chronischer Schmerzzustände hat in der Medizin eine große Bedeutung, denn Schmerz gehört zu den Basissymptomen in der Klinik. Zur Zeit besteht ein weltweiter Bedarf an zusatzlicher, nicht ausschließlich opioider, aber gut wirksamer Schmerztherapie. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung fur den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische Opioide wie z.B. Morphin sind bei der Therapie starker bis stärkster Schmerzen gut wirksam. Ihr Einsatz wird jedoch durch die bekannten Nebenwirkungen z.B. Atemdepression, Erbrechen, Sedierung, Obstipation,

Sucht, Abhängigkeit und Toleranzentwicklung limitiert. Sie konnen daher nur unter besonderen Vorsichtsmaßnahmen wie z.B. speziellen Verordnungsvorschriften über einen langeren Zeitraum oder in höheren Dosierungen gegeben werden (Goodman, Gilman, The Pharmacological Basis of Therapeutics, Pergamon Press, New York 1990). Außerdem sind sie bei einigen Schmerzzuständen, insbesondere bei neuropathischen Schmerzen, weniger wirksam.

Die der Erfindung zugrundeliegende Aufgabe bestand darin, eine neue Strukturklasse analgetisch wirksamer Substanzen zu finden, die sich zur Schmerztherapie eignen. Weitere Aufgaben bestanden darin, Wirkstoffe zu finden, die sich auch zur Verwendung als Lokalanastetikum und/oder Antiarrhythmikum und/oder Antiemetikum und/oder Nootropikum (Neurotropikum) und/oder zur Behandlung/Therapie von cardiovaskulären Erkrankungen und/oder Harninkontinenz und/oder Diarrhöe und/oder Pruritus und/oder Alkoholund/oder Drogen- und/oder Medikamentenabhängigkeit und/oder Entzündungen eignen. In der Regel eignen die Substanzen sich auch zur Behandlung von Depressionen und/oder zur Vigilanzsteigerung und/oder Libidosteigerung.

Es wurde gefunden, daß die Verbindungsklasse der allgemeinen Formel I sich durch eine ausgeprägte analgetische Wirkung auszeichnet. Darüber hinaus zeigen die Verbindungen der allgemeinen Formel I deutliche Affinität zur Bindungsstelle 2 des Natriumkanals (BTX-Bindung), zur Benzothiazepin- und zur Phenylalkylamin-Bindungsstelle des L-Typ Kalziumkanals (Diltiazem- und Verapamil-Bindung) und hemmen die synaptosomale Noradrenalin-Wiederaufnahme (NA-Uptakehemmung). Dadurch ist die Verbindungsklasse der allgemeinen Formel I auch zur Verwendung als Lokalanästetikum und/oder Antiarrhythmikum und/oder Anti-, emetikum und/oder Nootropikum (Neurotropikum) und/oder zur Behandlung/Therapie von cardiovaskulären Erkrankungen und/oder Harninkontinenz und/oder Diarrhoe und/oder Pruritus und/oder Alkohol- und/oder Drogen- und/oder Medikamentenabhängigkeit und/oder Entzundungen geeignet. In der Regel eignet sich die Verbindungsklasse der allgemeinen Formel I auch zur Vigilanzsteigerung und/oder Libidosteigerung und/oder zur Behandlung von Depressionen.

Die Erfindung betrifft daher substituierte 3-Amino-3-arylpropan-1-ole der allgemeinen Formel I,

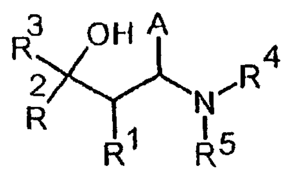

I

worin die Reste $R^1$ bis $R^5$ und A die oben angegebene Bedeutung haben und die entsprechenden Diastereomere oder Enantiomere in Form ihrer Basen oder Salze von physiologisch verträglichen Säuren, wobei 1-Benzyl-2-(dixnethylaininopheiiyline thyl) -cyclohexanol, dessen Diastereomere und dessen Enantiomere in Form ihrer Base und dessen Umsetzungsprodukt mit Methyljodid ausgenommen sind.

Bevorzugt sind Verbindungen der allgemeinen Formel I, in denen $R^1$ und $R^2$ zusammen einen $(CH_2)_{2-6}$-Ring, insbesondere einen $(CH_2)_4$-Ring, bilden, der gegebenenfalls phenylsubstituiert ist, $R^3$ bis $R^5$ und A die Bedeutung gemaß der Definition der allgemeinen Formel I haben oder

Verbindungen der allgemeinen Formel I, in denen $R^3$ ein substitiertes $C_{1-3}$-Alkylphenyl der Formel XII darstellt, $R^1$, $R^2$, $R^4$ und $R^5$ und A die Bedeutung gemäß der Definition der allgemeinen Formel I haben oder

Verbindungen der allgemeinen Formel I, in denen $R^3$ einen Arylrest mit gegebenenfalls Heteroatomen im Ringsystem und den Substituenten $R^6$ bis $R^8$ am Arylring clarstellt, $R^1$, $R^2$, $R^4$ und $R^5$ und A die Bedeutung gemäß der Definition der allgemeinen Formel I haben oder

Verbindungen der allgemeinen Formel I, in denen A einen Rest aus der Gruppe von substituiertem Phenyl der Formel XI

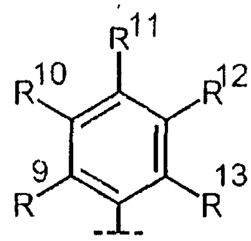

XI,

worin

$R^9$ bis $R^{13}$ ,jeweils unabhängig voneinander, H, F, Cl, Br, I, $CF_3$, OH, $OR^{14}$, $OCF_3$, $SR^{14}$, $SO_2CH_3$, $SO_2CF_3$, $C_{1-6}$-Alkyl, Phenyl, CN, $COOR^{14}$, $NO_2$ oder $R^9$ und $R^{10}$ oder $R^{10}$ und $R^{11}$ zusammen einen $OCH_2O$- oder $OCH_2CH_2O$-Ring bilden,

$R^{14}$ $C_{1-6}$-Alkyl, Phenyl, Benzyl, Phenethyl

oder A einen unsubstituierten oder substituierten Thiophen-Rest oder einen unsubstituierten oder substituierten Furan-Rest bedeutet und die Reste $R^1$ bis $R^5$ die Bedeutung gemäß der Definition der allgemeinen Formel I haben.

Weiterhin bevorzugt sind Verbindungen der allgemeinen Formel I, in denen $R^1$ und $R^2$ zusammen einen $(CH_2)_{2-6}$-Ring, insbesondere einen $(CH_2)_4$-Ring, bilden, der gegebenenfalls phenylsubstituiert ist, $R^3$ ein substitiertes $C_{1-3}$-Alkylphenyl der Formel XII darstellt, $R^4$ bis $R^5$ und A die Bedeutung gemäß der Definition der allgemeinen Formel I haben oder

Verbindungen der allgemeinen Formel I, in denen $R^1$ und $R^2$ zusammen einen $(CH_2)_{2-6}$-Ring, insbesondere einen $(CH_2)_4$-Ring, bilden, der gegebenenfalls phenylsubstituiert ist, $R^3$ einen Arylrest mit gegebenenfalls Heteroatomen im Ringsystem und den Substituenten $R^6$ bis $R^8$ am Arylring darstellt, $R^4$ bis $R^5$ und A die Bedeutung gemäß der Definition der allgemeinen Formel I haben oder

Verbindungen der allgemeinen Formel I, in denen $R^1$ und $R^2$ zusammen einen $(CH_2)_4$-Ring bilden, der gegebenenfalls phenylsubstituiert ist, A einen Rest aus der Gruppe von substituiertem Phenyl der Formel XI oder einen unsubstituierten oder substituierten Thiophen-Rest oder einen unsubstitujerten oder substituierten Furan-Rest bedeutet, $R^3$ ein substitiertes $C_{1-3}$-Alkylphenyl der Formel XII darstellt, $R^4$ bis $R^5$ die Bedeutung gemäß der Definition der allgemeinen Formel I haben oder

Verbindungen der allgemeinen Formel I, in denen $R^1$ und $R^2$ zusammen einen $(CH_2)_4$-Ring bilden, der gegebenenfalls phenylsubstituiert ist, A einen Rest aus der Gruppe von substituiertem Phenyl der Formel XI oder einen unsubstituierten oder substituierten Thiophen-Rest oder einen unsubstituierten oder substituierten Furan-Rest bedeutet, $R^3$ einen Arylrest mit gegebenenfalls Heteroatoinen im Ringsystem und den Substituenten $R^6$ bis $R^8$ am Arylring darstellt, $R^4$ bis $R^5$ die Bedeutung gemäß der Definition der allgemeinen Formel I haben oder

Bevorzugt sind auch Verbindungen der allgemeinen Formel I, in denen $R^1$ und $R^2$ zusammen einen $(CH_2)_4$-Ring bilden, A einen unsubstituierten oder substituierten Thiophen-Rest, $R^3$ ein substitiertes $C_{1-3}$-Alkylphenyl der Formel XII darstellt, $R^4$ bis $R^5$ die Bedeutung gemäß der Definition der allgemeinen Formel I bedeuten oder

Verbindungen der allgemeinen Formel I, in denen $R^1$ und $R^2$ zusammen einen $(CH_2\ )_4$-Ring bilden, A einen unsubstituierten oder substituierten Thiophen-Rest, $R^3$ einen Arylrest mit gegebenenfalls Heteroatomen im Ringsystem und den Substituenten $R^6$ bis $R^8$ am Arylring darstellt, $R^4$ bis $R^5$ die Bedeutung gemäß der Definition der allgemeinen Formel I bedeuten oder

Verbindungen der allgemeinen Formel I, in denen $R^1$ und $R^2$ zusammen einen $(CH_2)_4$-Ring bilden, A einen unsubstituierten oder substituierten Furan-Rest, $R^3$ ein substitiertes $C_{1-3}$-Alkylphenyl der Formel XII darstellt, $R^4$ bis $R^5$ die Bedeutung gemäß der Definition der allgemeinen Formel I bedeuten.

Verbindungen der allgemeinen Formel I, in denen R1 und $R^2$ zusammen einen $(CH_2)_4$-Ring bilden, A einen unsubstituierten oder substituierten Furan-Rest, $R^3$ einen Arylrest mit gegebenenfalls Heteroatomen im Ringsystem und den Substituenten $R^6$ bis $R^8$ am Arylring darstellt, $R^4$ bis $R^5$ die Bedeutung gemäß der Definition der allgemeinen Formel I bedeuten.

Zu weiteren bevorzugten Verbindungen zählen:

2-(-Dimethylaminophenylmethyl)-1-(3-methoxyphenyl)-cyclohexanol und das entsprechende Hydrochlorid

2-(-Dimethylaminophenylmethyl)-1-(3-fluorphenyl)cyclohexanol und das entsprechende Hydrochlorid

2-(Dimethylaminophenylmethyl)-1-phenylcyclohexanol und das entsprechende Hydrochlorid

3-[2-(Dimethylaminophenylmethyl)-1-hydroxycyclohexyl]-phenol und das entsprechende Hydrochlorid

2- (Dimethylaminophenylmethyl)-1-(4-methoxyphenyl)-cyclohexanol und das entsprechende Hydrochlorid

1- (4-Chlorphenyl)-2 -(dimethylaminophenylmethyl)cyclohexanol und das entsprechende Hydrochlorid

2-(Dimethylaminophenylmethyl)-1-(4-fluorphenyl)cyclohexanol und das entsprechende Hydrochlorid

2-(Dimethylaminophenylmethyl)-1-p-tolylcyclohexanol und das entsprechende Hydrochlorid

1-(3-Chlorphenyl)-2-[dimethylamino-(3-methoxyphenyl)-methyl]cyclohexanol und das entsprechende Hydrochlorid

1- (4-Dimethylaminophenyl)-2-[dimethylaminophenylmethyl]cyclohexanol und das entsprechende Hydrochlorid

1-Benzo[1,3]dioxol-4-yl-2-(dimethylaminophenylmethyl)-cyclohexanol und das entsprechende Hydrochlorid

1-(3,4-Dimethoxyphenyl)-2-(dimethylaminophenylmethyl)-cyclohexanol und das entsprechende Hydrochlorid

2-(Dimethylaminophenylmethyl)-1-(3-methoxybenzyl)-cyclohexanol und das entsprechende Hydrochlorid

1-Benzyl-2-(dimethylaminophenylmethyl)cyclohexanol; Hydrochlorid

2-(Dimethylaminophenylmethyl)-1-(4-fluor-3-trifluormethylphenyl)cyclohexanol und das entsprechende Hydrochlorid

2-(Dimethylaminophenylmethyl)-1-(4-trifluormethoxybenzyl) cyclohexanol und das entsprechende Hydrochlorid

2-(Dimethylaminophenylmethyl)-1-furan-3-ylcyclohexanol und das entsprechende Hydrochlorid

1-Butyl-2-(dimethylaminophenylmethyl)cyclohexanol und das entsprechende Hydrochlorid

1-(3,4-Dichlorphenyl)-2-(dimethylaminophenylmethyl)-cyclohexanol und das entsprechende Hydrochlorid

(+)-1-(3,4-Dichlorphenyl)-2-(dimethylaminophenylmethyl) cyclohexanol und das entsprechende Hydrochlorid

(-)-1-(3, 4-Dichlorphenyl)-2-(dimethylaminophenylmethyl)cyclohexanol und das entsprechende Hydrochlorid

4-[2-(Dimethylaminophenylmethyl)-1-hydroxycyclohexyl]-phenol und das entsprechende Hydrochlorid

2-(Dimethylaminophenylmethyl)-1-naphthalin-2-ylcyclohexanol und das entsprechende Hydrochlorid

2-[Dimethylamino-(4-trifluormethylphenyl)methyl]-1-(3-methoxybenzyl)cyclohexanol und das entsprechende Hydrochlorid

1-(4-Chlorbenzyl)-2-(dimethylaminophenylmethyl)-1-cyclohexanol und das entsprechende Hydrochlorid

2-(Dimethylaminophenylmethyl)-1-(2-fluorbenzyl)cyclohexanol und das entsprechende Hydrochlorid

2-(Dimethylaminophenylmethyl)-1-(4-fluorbenzyl)cyclohexanol und das entsprechende Hydrochlorid

1-(2,5-Dimethoxyphenyl)-2-(dimethylaminophenylmethyl)-cyclohexanol und das entsprechende Hydrochlorid

1-(2-Chlor-4-fluorbenzyl )-2-(dimethylaminophenylmethyl) cyclohexanol und das entsprechende Hydrochlorid

1-(4-tert.-Butylbenzyl)-2-(dimethylaminophenylmethyl)-cyclohexanol und das entsprechende Hydrochlorid

2-(Dimethylaminophenylmethyl)-1-(3-fluorbenzyl)cyclohexanol und das entsprechende Hydrochlorid

1-(2-Chlorbenzyl)-2- (dimethylaminophenylmethyl)cyclohexanol und das entsprechende Hydrochlorid

1-Benzo[1,3]dioxol-5-yl-2-[dimethylamino-(3-methoxyphenyl)methyl] cyclohexanol und das entsprechende Hydrochlorid

1-(3-Chlorbenzyl)-2-(dimethylaminophenylmethyl)cyclohexanol und das entsprechende Hydrochlorid

1-(2,4-Dichlorbenzyl)-2-(dimethylaminophenylmethyl)-cyclohexanol und das entsprechende Hydrochlorid

1-Benzyl-2-[dimethylaminophenyl-(3-phenoxyphenyl)-methyl]cyclohexanol und das entsprechende Hydrochlorid

1-Benzyl-2-[dimethylaminophenyl-(3-methoxyphenyl)-methyl] cyclohexanol und das entsprechende Hydrochlorid

2-(Dimethylaminophenylmethyl)-1-(3-trifluormethylbenzyl)cyclohexanol und das entsprechende Hydrochlorid

2-[Dimethylamino-(3-methoxyphenyl)methyl]-1-(3-methoxybenzyl)cyclohexanol und das entsprechende Hydrochlorid

2-[(2-Chlorphenyl)dimethylaminomethyl]-1-naphthalin-2-ylcyclohexanol und das entsprechende Hydrochlorid

1-Benzyl-2-[(3,4-dichlorphenyl)dimethylaminomethyl]-cyclohexanol und das entsprechende Hydrochlorid

2-[(3,4-Dichlorophenyl)dimethylaminomethyl]-1-phenethylcyclohexanol und das entsprechende Hydrochlorid

1-Benzyl-2- [dimethylamino-(4-fluorphenyl) methyl]]cyclohexanol und das entsprechende Hydrochlorid

2-[(3-Chlorphenyl) dimethylaminomethyl]-1-phenylcyclohexanol und das entsprechende Hydrochlorid

1-(2,4-Dichlorphenyl)-2-(3-dimethylaminomethyl)-1-cyclohexanol und das entsprechende Hydrochlorid

1-Benzyl-2-[(3-Chlorphenyl)dimethylaminomethyl]cyclohexanol und das entsprechende Hydrochlorid

1-Benzyl-2-[(2-chlorphenyl)dimethylaminomethyl]cyclohexanol und das entsprechende Hydrochlorid

1-(4-tert.-Butylbenzyl)-2-[(3,4-dichlorphenyl)di-methylarninomethyl]cyclohexanol und das entsprechende Hydrochlorid

2-[Dimethylamino-(4-fluorphenyl)methyl]-1-(3-trifluormethylbenzyl)cyclohexanol und das entsprechende Hydrochlorid

2-Dimethylaminophenylmethyl) bicyclohexyl-1-ol und das entsprechende Hydrochlorid

2-(Dimethylaminophenylmethyl)-1-(4-methoxybenzyl)-cyclohexanol und das entsprechende Hydrochlorid

1-(2,4-Difluorbenzyl)-2-(dimethylaminophenylmethyl)-cyclohexanol und das entsprechende Hydrochlorid

1-(4-tert.-Butylbenzyl)-2-[(3-chlorphenyl)dimethyl-aminomethyl]cyclohexanol und das entsprechende Hydrochlorid

2-[Dimethylamino-(3-phenoxyphenyl)methyl]-1-phenethylcyclohexanol und das entsprechende Hydrochlorid

2-[Dimethylamino-(3-phenoxyphenyl)methyl]-1-(3-tri-fluormethylbenzyl)cyclohexanol und das entsprechende Hydrochlorid

1-(2,5-Difluorbenzyl)-2-(dimethylaminophenylmethyl)-cyclohexanol und das entsprechende Hydrochlorid

1-(3,4-Difluorbenzyl)-2-(dimethylaminophenylmethyl)-cyclohexanol und das entsprechende Hydrochlorid

1-(2-Chlor-6-fluorbenzyl)-2-(dimethylaminophenylmethyl) cyclohexanol und das entsprechende Hydrochlorid

1-(2,3-Difluorbenzyl)-2-(dimethylaminophenylmethyl)-cyclohexanol und das entsprechende Hydrochlorid

1-Benzyl-2-[(4-chlorphenyl)dimethylaminomethyl]cyclohexanol und das entsprechende Hydrochlorid

1-Dimethylamino-3-ethyl-2-methyl-1,5-diphenylpentan-3-ol und das entsprechende Hydrochlorid

1-(2-Chlorbenzyl)-2-[(2-chlorphenyl)-dimethylaminomethyl]cyclohexanol und das entsprechende Hydrochlorid

1-Benzyl-2-[(4-bromphenyl)dimethylaminomethyl]cyclohexanol und das entsprechende Hydrochlorid

2-[(4-Chlorphenyl)dimethylaminomethyl]-1-(4-trifluormethylphenyl)cyclohexanol und das entsprechende Hydrochlorid

2-[(4-Chlorphenyl)dimethylaminomethyl]-1-(3-trifluor-methylbenzyl)cyclohexanol und das entsprechende Hydrochlorid

1-(4-tert.-Butylbenzyl)-2-[dimethylamino-(3-phenoxy-phenyl)methyl]cyclohexanol und das entsprechende Hydrochlorid

4-{Dimethylamino-[2-hydroxy-2-(4-trifluormethylphenyl)cyclohexyl]methyl}benzonitril und das entsprechende Hydrochlorid

2-(Dimethylamino-o-tolylmethyl)-1-phenylcyclohexano) und das entsprechende Hydrochlorid

1-Benzyl-2-(dimethylamino-o-tolylmethyl)cyclohexanol und das entsprechende Hydrochlorid

2- (Dimethylaminophenylmethyl)-1-(3-phenylpropyl )cyclohexanol und das entsprechende Hydrochlorid

2-[(2-Chlorphenyl)dimethylaminomethyl]-1-[2-(4-fluor-phenyl)ethyl]cyclohexanol und das entsprechende Hydrochlorid

2-[Dimethylaminothiopen-2-ylmethyl]-1-(3-trifluormethylbenzyl)cyclohexanol und das entsprechende Hydrochlorid

Methyl-4-[2-(dimethylaminophenylmethyl)-1-hydroxycyclohexyl]benzoat und das entsprechende Hydrochlorid

1-Benzyl-2-(dimethylaminophenylmethyl)-4-phenylcyclohexanol und das entsprechende Hydrochlorid

1-(4-Bromiphenyl)-2- (dimethylaminophenylmethyl)cyclohexanol und das entsprechende Hydrochlorid

2-(Dimethylaminophenylmethyl)-1-naphthalin-1-ylcyclohexanol und das entsprechende Hydrochlorid

2-(Dimethylaminophenylmethyl)-1-(2-methylsulfanylphenyl) cyclohexanol und das entsprechende Hydrochlorid

1-Benzyl-2-(dimethylaminonaphthalin-2-ylmethyl)cyclohexanol und das entsprechende Hydrochlorid

1-Benzyl-2-(dimethylaminopentafluorphenylmethyl)cyclohexanol und das entsprechende Hydrochlorid

1-Benzyl-2-(phenylpiperidin-1-ylmethyl)cyclohexanol und das entsprechende Hydrochlorid

2-(Dimethylaminophenylmethyl)-1-(4-trifluormethylphenyl)cyclohexanol und das entsprechende Hydrochlorid

3-(4-tert.-Butylbenzyl)-1-dimethylamino-2-methyl-1-phenylpentan-3-ol und das entsprechende Hydrochlorid

2-(Dimethylamino-o-tolylmethyl)-1-phenethylcyclohexanol und das entsprechende Hydrochlorid

1-(4- *tert.*-Butylbenzyl)-2-[dimethylaminothiophen-2-ylmethyl]cyclohexanol und das entsprechende Hydrochlorid

Erfindungsgemäße Verbindungen sind auch Verbindungen der allgemeinen Formel I als Diastereomere oder Enantiomere in Form ihrer Basen oder Salze von physiologisch vertraglichen Säuren.

In einer speziellen Ausführungsform der Erfindung werden die erfindungsgemäßen Verbindungen einschließlich der ausgenommenen Verbindungen als Gemisch der Enantiomeren in nicht äquimolaren Mengen als Wirkstoff in einem Arzneimittel, gegebenenfalls neben weiteren Wirkstoffen, verwendet. Vorzugsweise betragt dabei der Anteil eines Enantiomeren zwischen 5 und 45 Massenprozent.

Der Ausdruck "$C_{1-6}$-Alkyl" bedeutet im Rahmen der vorliegenden Erfindung geradkettige oder verzweigte Kohlenwasserstoffe mit 1 bis 6 Kohlenstoffatomen. Beispielhaft seien Methyl, Ethyl, Propyl, Isopropyl, n-Butyl, sek. -Butyl, tert.-Butyl, n-Pentyl, Neopentyl und n-Hexyl genannt.

Der Ausdruck "$C_{3-7}$-Cycloalkyl" bedeutet im Rahmen der vorliegenden Erfindung gesättigte cyclische Kohlenwasserstoffe oder geradkettige oder verzweigte Alkylreste,die gesättigte cyclische Kohlenwasserstoffe enthalten, mit insgesamt 3 bis 7 Kohlenstoffatomen. Beispielhaft seien Cyclopropyl, Cyclopropylmethyl, Methylcyclopropyl, Cyclobutyl, 1-Cyclopropyiethyl, 2-Cyclopropylethyl, Cyclopentyl, CyclopentyliriethyJ, Cyclohexyl oder Cycloheptyl erwähnt.

Der Ausdruck "Aryl" bedeutet im Rahmen der vorliegenden Erfindung gegebenenfalls ein- oder mehrfach substituierte vorzugsweise aromatische Ringsysteme, die gegebenenfalls Heteroatome im Ringsystem aufweisen können. Vorzugsweise sind die Arylreste ein- oder mehrfach mit den Resten $R^9$ bis $R^{13}$ substituiert. Die vorzugsweise 5- oder 6-gliedrigen ungesättigten, gegebenenfalls mit weiteren Ringen kondensierten, ggf. ein- oder mehrfach substituierten heterocyclischen Verbindungen können ein oder zwei Heteroatome, wie Stickstoff, Sauerstoff und/oder Schwefel im Ringsystem aufweisen.

Beispielhaft seien aus der Gruppe der Heteroaryle Furan, Thiophen, Pyrrol, Pyridin, Pyrimidin, Chinolin, Isochinolin, Phthalazin oder Chinazolin aufgeführt.

Des weiteren sind Verfahren zur Herstellung der Verbindung der allgemeinen Formel I ein Erfindungsgegenstand.

Diese Verfahren zur Herstellung der Verbindungen der allgemeinen Formel I mit Ausnahme des 1-Benzyl-2-(dimethylaminophenylmethyl)-cyclohexanol, dessen Diastereomeren und dessen Enantiomeren sind dadurch gekennzeichnet, daß man Mannich-Basen der Formel II mit geeigneten Nukleophilen, vorzugsweise metallorganischen Verbindungen $R^3Y$, in denen Y MgCl, MgBr, MgI oder Li bedeutet, bei Temperaturen zwischen -70°C und +110°C umsetzt

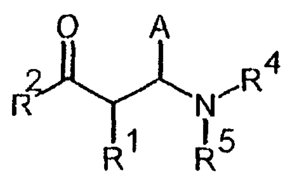

II

Die Umsetzung einer Mannichbase der Formel II mit einer Grignardverbindung $R^3Y$, in der Y MgCl, MgBr oder MgI bedeutet, oder mit einer lithiumorganischen Verbindung $R^3Li$ kann in einem aliphatischen Ether, beispielsweise Diethylether und/oder Tetrahydrofuran, einem Kohlenwasserstoff, beispielsweise Hexan oder Toluol, oder Gemischen aus Kohlenwasserstoffen und aliphatischen Ethern, bei Temperaturen zwischen -70°C und +110°C durchgeführt werden. Die Darstellung einer Grignard-Verbindung $R^3Y$ kann mit oder ohne Zusatz eines Mitführreagenzes, vorzugsweise 1,2-Dibromethan, erfolgen. Alternativ können aromatische Grignard-Verbindungen $R^3Y$ durch Umsetzung eines aromatischen Iodids $R^3I$ mit einer Organomagnesiumverbindung, beispielsweise Isopropylmagnesiumchlorid oder Diisopropylmagnesium, durch Iod-Magnesiumaustausch erhalten werden. Lithiumorganische Verbindungen $R^3Li$ lassen sich aus OrganohalogenverbindungenR$^3$Z, in denen Z Cl, Br oder I bedeutet, durch Umsetzung mit beispielsweise einer n-Butyllithium/Hexan-Lösung durch Halogen-Lithiumaustausch erhalten.

Bei der Umsetzung einer Mannichbase der Formel II mit einer metallorganischen Verbindung $R^3Y$ werden in Abhängigkeit von den Reaktionsbedingungen bevorzugt tertiäre Alkohol mit der relativen Konfiguration der Formel Ia erhalten, in denen die Aminoarylmethylgruppe *cis* zur Hydroxylgruppe angeordnet ist, wenn $R^1$ und $R^2$ ein Ringsystem bilden. Bei offenkettigen Systemen wird die analoge relative Stereochemie erhalten, die als *anti* zu spezifizieren ist. Die Verbindungen der allgemeinen Formel I lassen sich durch säulenchromatographische Trennung oder durch Kristallisation, auch ihrer Salze, beispielsweise der Hydrochloride, diastereomerenrein erhalten.

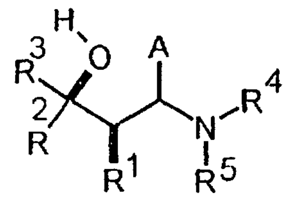

Ia

Die Mannichbasen der Formel II lassen sich durch Umsetzung von Enaminen der Formel III mit einem Imminiumsalz der Formel IV, in dem Y beispielsweise $Cl^-$, $AlCl_4^-$, $Br^-$ oder $I^-$ bedeutet, erhalten.

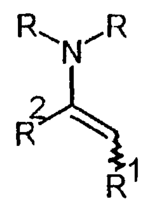

III

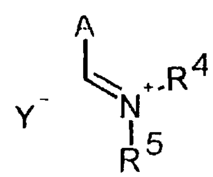

IV

Die Enamine werden nach literaturbekannten Verfahren aus Ketonen der Formel V und sekundären Aminen, beispielsweise Dimethylamin, Pyrrolidin, Piperidin oder Morpholin, hergestellt (Acta Chem. Scand. B 38 (1984) 49-53). Die Imminiumsalze werden nach literaturbekannten Verfahren durch Umsetzung von Aminalen der Formel VI mit Säurenchloriden, beispielsweise Acetylchlorid oder Thionylchlorid, hergestellt (Houben-Wey] - Methoden der Organischeu Chemie, E21b (1995) 1925-1929).

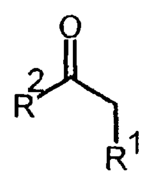

V

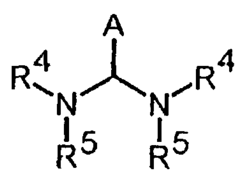

VI

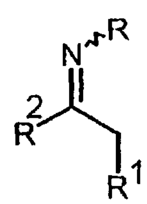

VII

Die Imminiumsalze der Formel IV müssen dabei nicht isoliert werden, sondern können in situ erzeugt und mit Enaminen der Formel III zu Mannichbasen der Formel II umgesetzt werden (Angew. Chem. 106 (1994) 2531-2533). Aufgrund der der Keto-Enol-Tautomerie analogen Enamin-Imin-Tautornerie sind statt der Enamine der Formel III auch Imine der Formel VII einsetzbar. Alternativ können Ketone der Formel V auch direkt mit Imminiumsalzen der Formel IV umgesetzt werden.

Mannichbasen der Formel II können aber auch durch Umsetzung von Enaminen der Formel III mit einem aromatischen Aldehyd der Formel VIII und einem sekundären Amin $HNR^4R^5$, auch in Form des korrespondierenden Hydrochlorids $HNR^4R^5$, HCl, in Gegenwart von Triethylamin, Chlortrimethylsilan und Natriumiodid direkt hergestellt werden (Synlett (1997) 974-976).

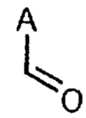

VIII

Die Mannichbasen der Formel II werden mit den oben beschriebenen Verfahren in Abhängigkeit von den Reaktionsbedingungen bevorzugt mit der relativen Konfiguration der Formel IIa erhalten, in denen die Aminogruppe *anti* zu $R^1$ angeordnet ist. Die Verbindungen der Formel IIa lassen sich durch Kristallisation, auch ihrer Salze, beispielsweise der Hydrochloride, oder durch chromatographische Trennung diastereomerenrein erhalten.

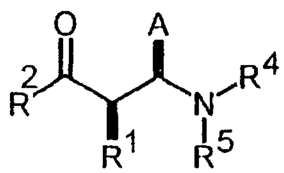

IIa

Weniger stereoselektiv verläuft dagegen die Darstellung von Mannichbasen der Formel II durch 1,4-Addition sekundärer Amine der Formel X an Enone der Formel IX, die aus der Aldolkondensation von Ketonen der Formel V mit aromatischen Aldehyden der Formel VIII erhalten werden (US 4,017,637). Diese Vorgehensweise eignet sich daher zur Darstellung der anderen möglichen Stereoisomeren.

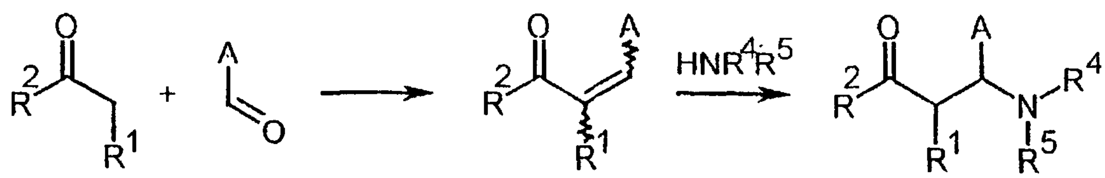

V VIII IX X II

Werden chirale Amine zur Darstellung von Enaminen der Formel III oder Iminen der Formel VII eingesetzt, so können in der nachfolgenden Mannichreaktion enantiomerenangereicherte bis enantiomerenreine Mannichbasen der Formel II erhalten werden (Houben-Weyl - Methoden der Organischen Chemie, E21b (1995) 1925-1929).

3-Amino-3-arylpropan-1-ol-Verbindungen der allgemeinen Formel I, in denen $R^3$ einen phenolischen Substituenten enthält, lassen sich beispielsweise aus den entsprechenden Methylether-Derivaten mit Diisobutylaluminiumhydrid in einem aromatischen Kohlenwasserstoff, beispielsweise Toluol, bei einer Temperatur zwischen 60°C und 130°C herstellen (Synthesis (1975) 617-630).

Die Verbindungen der Formel I lassen sich mit physiologisch verträglichen Säuren, beispielsweise Salzsaure, Bromwasser-stoffsäure, Schwefelsäure, Methansulfonsaure, Ameisensäure, Essigsaure, Oxalsäure, Bernsteinsaure, Weinsäure, Mandelsäure, Fumarsäure, Milchsäure, Zitronensäure, Glutaminsäure und/oder Asparaginsäure, in bekannter Weise in ihre Salze überfuhren. Vorzugsweise wird die Salzbildung in einem Lösungsmittel, beispielsweise Diethylether, Diisopropylether, Essigsäurealkylester, Aceton und/oder 2-Butanon durchgeführt. Zur Herstellung der Hydrochloride eignet sich darüber hinaus Trimethylchlorsilan in wäßriger Lösung.

Die der Formel I entsprechenden Substanzen sind toxikologisch unbedenklich, so daß sie sich als pharmazeutischer Wirkstoff in Arzneimittel eignen. Ein weiterer Gegenstand der Erfindung sind daher Arzneimittel enthaltend als Wirkstoff wenigstens eine Verbindung der allgemeinen Formel I. Vorzugsweise eignen sich die erfindungsgemäßen Arzneimittel als Analgetika.

Biochemische Untersuchungen haben ergeben, daß die Verbindungen der allgemeinen Formel I neben ihrer analgetischen Wirkung auch ausgeprägte Affinität zur Bindungstelle 2 des Natriumkanals (BTX-Bindung), zur Benzothiazepin- und zur Phenylalkylamin-Bindungsstelle des L-Typ Kalziumkanals (Diltiazem- und Verapamil-Bindung) zeigen und die synaptosomale Noradrenalin-Wiederaufnahme hemmen (NA-Uptakehemmung). Die erfindungsgemäßen Substanzen eignen sich daher neben der besonders bevorzugten Verwendung in der Schmerztherapie auch zur Verwendung als Lokalanastelikum und/oder Antiarrhythmikum und/oder Antiemetikum und/oder Nootropikum (Neurotropikum) und/oder zur Behandlung/Therapie von cardiovaskularen Erkrankungen und/oder Harninkontinenz und/oder Diarrhoe und/oder Pruritus und/oder Alkohol- und/oder Drogenund/oder Medikamentenabhangigkeit und/oder Entzündungen. In der Regel eignen die Verbindungen der allgemeinen Formel I sich auch zur Behandlung von Depressionen und/oder zur Vigilanzsteigerung und/oder Libidosteigerung.

Die erfindungsgemäßen Analgetika enthalten neben mindestens einem 3-Amino-3-arylpropan-1-ol-Derivat der Formel I Trägermaterialien, Füllstoffe, Lösungsmittel, Verdünnungsmittel, Farbstoffe und/oder Bindemittel. Die Auswahl der Hilfsstoffe sowie die einzusetzenden Mengen derselben hängt davon ab, ob das Arzneimittel oral, intravenös, intraperitoneal, intradermal, intramuskular, intranasal, buccal oder örtlich, zum Beispiel auf Infektionen an der Haut, der Schleimhaute und an den Augen, appliziert werden soll. Für die orale Applikation eignen sich Zubereitungen in Form von Tabletten, Dragees, Kapseln, Granulaten, Tropfen, Saften und Sirupen, für die parenterale, topische und inhalative Applikation Lösungen, Suspensionen, leicht rekonstituierbare Trockenzubereitungen sowie Sprays. Erfindungsgemäße Verbindungen der Formel I in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen Verbindungen der Formel 1 verzögert freisetzen.

Die an den Patienten zu verabreichende Wirkstoffmenge variiert in Abhängigkeit vom Gewicht des Patienten, von der Applikationsart, der Indikation und dem Schweregrad der Erkrankung. Ublicherweise werden 0,5 bis 500 mg/kg wenigstens eines 3-Amino-3-arylpropan-1-ol-Derivats der Formel 1 appliziert.

**Pharmakologische Untersuchungen**

**Analgesieprüfung im Writhing-Test an der Maus**

Die Untersuchung auf analgetische Wirksamkeit wurde i.m Phenylchinon-induzierten Writhing an der Maus (modifiziert nach I.C. Hendershot und J. Forsaith (1959) J. Pharmacol. Exp. Ther. 125, 237-240) durchgeführt. Dazu wurden männliche NMRI-Mäuse im Gewicht von 25 bis 30 g verwendet. Gruppen von 10 Tieren pro Substanzdosis erhielten 10 Minuten nach intravenöser Gabe der Prüfsubstanzen 0,3 ml/Maus einer 0,02%igen wäßrigen Lösung von Phenylchinon (Phenylbenzochinon, Fa. Sigma, Deisenhofen; Herstellung der Lösung unter Zusatz von 5 % Äthanol und Aufbewahrung im Wasserbad bei 45°C) intraperitoneal appliziert. Die Tiere wurden einzeln in Beobachtungskäfige gesetzt. Mittels eines Drucktastenzähler wurde die Anzahl der schmerzinduzierten Streckbewegungen (sogenannte Writhingreaktionen = Durchdrucken des Körpers mit Abstrecken der Hinterextremitäten) 5 bis 20 Minuten nach der Phenylchinon-Gabe ausgezählt. Als Kontrolle wurden Tiere mitgeführt, die nur physiologische Kochsalzlösung erhalten. Alle Substanzen wurden in der Standarddosierung von 10 mg/kg getestet. Die prozentuale Hemmung (%Hemmung) der Writhingreaktion durch eine Substanz wurde nach folgender Formel berechnet:

$$\% \text{ Hemmung} = 100 - \frac{\text{Writhingreaktionen der behandelten Tiere}}{\text{Writhingreaktionen der Kontrolltiere}} * 100$$

Für einige Substanzen wurde aus der dosisabhangigen Abnahme der Writhingreaktionen im Vergleich zu parallel untersuchten Phenylchinon-Kontrollgruppen mittels Regressionsanalyse (Auswerteprogramm Martens EDV Service, Eckental) die $ED_{50}$-Werte mit 95 % Vertrauensbereich der Writhingreaktion berechnet.

Alle untersuchten erfindungsgemäßen Verbindungen zeigten eine ausgeprägte analgetische Wirkung. Die Ergebnisse sind in Tabelle 1 zusammengefaßt.

Tabelle 1

| (Teil 1/2): Analgesieprufung im Writhing-Test an der Maus | | | |
|---|---|---|---|
| Beispiel | %Hemmung der Writhingreaktion bei 10 mg/kg intravenös | Beispiel | %Hemmung der Writhingreaktion bei 10 mg/kg intravenös |
| **1** | 85 | **29** | 96 |
| **2** | 88 | **30** | 85 |
| **3** | 83 | **31** | 76 |

Tabelle 1 (fortgesetzt)

| (Teil 1/2): Analgesieprufung im Writhing-Test an der Maus | | | |
|---|---|---|---|
| Beispiel | %Hemmung der Writhingreaktion bei 10 mg/ kg intravenös | Beispiel | %Hemmung der Writhingreaktion bei 10 mg/ kg intravenös |
| **4** | 75 | **32** | 100 |
| **5** | 100 | **33** | 84 |
| **6** | 83 | **34** | 99 |
| **7** | 85 | **35** | 81 |
| **8** | 81 | **36** | 100 |
| **9** | 73 | **37** | 93 |
| **10** | 97 | **38** | 85 |
| **11** | 99 | **39** | 94 |
| **12** | 95 | **40** | 72 |
| **13** | 100 | **41** | 88 |
| **14** | 70 | **42** | 76 |
| **15** | 73 | **43** | 80 |
| **16** | 75 | **44** | 100 |
| **17** | 100 | **45** | 82 |
| **18** | 100 | **46** | 68 |
| **+19** | 74 | **47** | 89 |
| **- 19** | 88 | **48** | 94 |
| **20** | 96 | **49** | 100 |
| **21** | 97 | **50** | 85 |
| **22** | 68 | **51** | 99 |
| **23** | 100 | **52** | 70 |
| **24** | 97 | **53** | 90 |
| **25** | 100 | **54** | 98 |
| **26** | 94 | **55** | 92 |
| **27** | 82 | **56** | 94 |
| **28** | 100 | **57** | 84 |

Tabelle 1

| (Teil 2/2): Analgesieprufung im Writhing-Test an der Maus | |
|---|---|
| Beispiel | % Hemmung der Writhingreaktion bei 10 mg/kg intravenös |
| **58** | 98 |
| **59** | 59 |
| **60** | 63 |
| **61** | 90 |
| **62** | 94 |
| **63** | 86 |

Tabelle 1 (fortgesetzt)

| (Teil 2/2): Analgesieprufung im Writhing-Test an der Maus | |
|---|---|
| Beispiel | % Hemmung der Writhingreaktion bei 10 mg/kg intravenös |
| **64** | 88 |
| **65** | 76 |
| **66** | 91 |
| **67** | 84 |
| **68** | 55 |
| **69** | 45 |
| **70** | 98 |
| **71** | 55 |
| **72** | 89 |
| **73** | 75 |
| **74** | 37 |
| **75** | 57 |
| **76** | 60 |
| **77** | 54 |
| **78** | 73 |
| **79** | 71 |
| **80** | 61 |
| **81** | 75 |
| **82** | 100 |

**Biochemische Untersuchungen**

**Untersuchungen zur Noradrenalin-Wiederaufnahmehemmung (NA-Uptakehemmung)**

Um diese in vitro Studien durchführen zu können, werden Synaptosomen aus Rattenhirnarealen frisch isoliert. Es findet jeweils eine sogenannte "$P_2$"-Fraktion Verwendung, die nach der Vorschrift von Gray und Whittaker (E.G. Gray und V.P. Whittaker (1962) J. Anat. 76, 79-88) präpariert wird. Für den NA-uptake werden diese vesikulären Partikel aus dem Hypothalamus männlicher Rattengehirne isoliert.

Folgende Kenndaten wurden für den NA-Transporter ermittelt:

$$\text{NA-Uptake : Km} = 0{,}32 \pm 0{,}11\ \mu\text{M}$$

(Jeweils N = 4, d.h. Mittelwerte ± SEM aus 4 unabhängigen Versuchsreihen, die in Dreifach-Parallelversuchen durchgeführt wurden).

Eine detaillierte Methodenbeschreibung kann der Litaratur entnommen werden (M.Ch. Frink, H.-H. Hennies, W. Englberger, M. Haurand und B. Wilffert (1996) Arzneim.-Forsch./Drug Res. 46 (III), 11, 1029-1036).

**Bindungsuntersuchungen am L-Kalziumkanal**

**Benzothiazepin-Bindungsstelle (Diltiazem-Bindung)**

Das biologische Membranmaterial wurde aus dem Cerebrocortex der Ratte isoliert. Als Ligand wurde [$^3$H]-cis-(+)-Diltiazem (5 nM im Ansatz) verwendet. Inkubation für 20 Minuten bei 25 °C. Als unspezifische Bindung ist die Radioaktivität definiert, die bei Anwesenheit von (±)-Diltiazem ($10^{-6}$ M im Ansatz) gemessen wird. Der nicht gebundene

Anteil des radioaktiven Liganden wird nach Abschluß der Inkubation mit Hilfe eines Filtrationsprozesses über Whatman Glasfiber GF/B-Membranen abgetrennt. Die Membranen werden anschließend nach einem Waschprozeß am β-Counter vermessen. Die Methode ist in Anlehnung an die Veröffentlichung von Schoemaker und Langer (H. Schoemaker und S.Z. Langer (1985) Eur. J. Pharmacol. 111, 273-277) erstellt worden. Der $K_D$-Wert für diese hoch affine Bindungsstelle wurde mit 4,10 ± 0,75 nM bestimmt (N = 3, d.h. Mittelwerte ± SEM aus 3 unabhangigen Versuchsreihen, die in Dreifach-Parallelversuchen durchgeführt worden sind).

**Phenylalkylamin-Bindungsstelle (Verapamil-Bindung)**

Das biologische Material (Ionenkanal-Partikel) ist in Anlehnung an die Publikation von Reynolds, Gould und Snyder (I.J. Reynolds, R.J. Gould und S.H. Snyder (1983) J. Pharmacol. 95, 319-321) hergestellt worden.

Als Radioligand wurde N-Methyl-[$^3$H]-Verapamil (2 nM im Ansatz) verwendet. Als unspezifische Bindung wird diejenige Radioaktivität definiert, die in Gegenwart von nicht radioaktivem Verapamil ($10^{-4}$ M im Ansatz) bestimmt wird. Inkubation bei 25 °C für 45 Minuten. Anschließende Filtration über Whatman GF/B-Filter mit angeschlossener Wasche. Bestimmung der auf dem Filter verbliebenen Radioaktivität (Ionenkanalbindung) am β-Counter.

Der $K_D$-Wert für diese Bindungsstelle wurde mit 138,6 nM bestimmt (N = 2, d.h. Mittelwerte aus 2 unabhängigen Versuchsreihen, die in Dreifach-Parallelversuchen durch-geführt worden sind).

**Bindungsuntersuchungen am Natriumkanal**

**Bindungsstelle 2 (BTX-Bindung):**

Die Bindungsstelle 2 des Natriumkanals ist die sogenannte Batrachotoxin-(BTX) Bindungsstelle. Als Ligand wurde [$^3$H]-Batrachotoxinin A20 α-Benzoat (10 nM im Ansatz) eingesetzt. Diese Ionenkanal-Partikel (Synaptosomen) wurden aus dem Ratten Cerebrocortex nach Gray und Whittaker (E.G. Gray und V.P. Whittaker (1962) J. Anat. 76, 79-88) angereichert. Als unspezifische Bindung ist die Radioaktivität definiert, die in Gegenwart von Veratridin (0,3 mM im Ansatz) gemessen wird. Inkubation bei. 37°C für 120 min. Die Assaybedingungen sind nach der Veröffentlichung von Pauwels, Leysen und Laduron (P.J. Pauwels, J.E. Leysen und P.M. Laduron (1986) Eur. J. Pharmacol. 124, 291-298) durchgeführt worden.

Der $K_D$-Wert für diese Bindungsstelle liegt bei 24,63 ± 1,56 nM. (N = 3, d.h. Mittelwerte ± SEM aus 3 unabhängigen Versuchsreihen, die in Dreifach-Parallelversuchen durchgeführt worden sind).

**Auswertung**

Neben der prozentualen Hemmung cler Testsysteme bei fixen Testsubstanzkonzentrationen (NA-Uptake: 1 μM im Ansatz; Ionenkanal Assays: 10 μM im Ansatz), wurden Dosisabhängigkeiten überprüft. Hierbei werden $IC_{50}$-Werte erhalten, die gemäß der "Cheng-Prusoff Gleichung" (Y.C. Cheng und W.H. Prusoff (1973) Biochem. Pharmacol. 22, 3099-3108) in Inhibitorkonstanten ($K_i$) umgerechnet werden können. Die $IC_{50}$ Werte wurden mit Hilfe des Computer-Programms "Figure P" (Version 6.0, Biosoft, Cambridge, England) erhalten. Km-Werte wurden gemäß Lineweaver und Burk (H. Lineweaver und D. Burk (1934) J. Am. Chem. Soc. 56, 658-666) berechnet. Um $K_D$-Werte darzustellen, ist das Computer-Programm "Ligand" (Version 4, Biosoft, England) angewendet worden.

Die Ergebnisse der biochemischen Untersuchungen sind in Tabelle 2 zusammengefaßt.

Tabelle 2

| (Teil 1/3): Biochemie | | | | |
|---|---|---|---|---|
| Beispiel | NA-Uptake-hemmung bei 1 μM | Verapamil-Bindung bei 10 μM | Diltiazen-Bindung bei 10 μM | BTX-Bindung bei 10 μM |
| **1** | 41 | 76 | 65 | 77 |
| **2** | 52 | 76 | 52 | 80 |
| **3** | 41 | 68 | 50 | 78 |
| **4** | 49 | 57 | 46 | 70 |
| **5** | 70 | 56 | 54 | 97 |
| **6** | 51 | 60 | 96 | 90 |

Tabelle 2 (fortgesetzt)

| (Teil 1/3): Biochemie | | | | |
|---|---|---|---|---|
| Beispiel | NA-Uptake-hemmung bei 1 μM | Verapamil-Bindung bei 10 μM | Diltiazen-Bindung bei 10 μM | BTX-Bindung bei 10 μM |
| **7** | 38 | 56 | 78 | 86 |
| **8** | 50 | 56 | 68 | 88 |
| **9** | 62 | | 91 | 89 |
| **10** | 48 | 61 | 69 | 88 |
| **11** | 54 | 61 | 85 | 79 |
| **12** | 87 | 69 | 51 | 69 |
| **13** | 95 | | 88 | 94 |
| **14** | 100 | | 88 | 89 |
| **15** | 44 | 87 | 97 | 94 |
| **16** | 89 | 91 | 100 | 98 |
| **17** | 57 | 72 | 54 | 70 |
| **18** | 84 | 75 | 81 | 95 |
| **19** | 50 | 81 | | 97 |
| **+19** | 62 | 82 | 85 | 92 |
| **- 19** | 36 | 83 | 85 | 96 |
| **20** | 68 | | 69 | 68 |
| **21** | 17 | 71 | 85 | 94 |
| **22** | 91 | 80 | 72 | 98 |
| **23** | 99 | | 100 | 94 |
| **24** | 98 | | 93 | 92 |
| **25** | 98 | | 85 | 91 |
| **26** | 5 | | 87 | 85 |
| **27** | 89 | | 96 | 97 |
| **28** | 63 | | 89 | 100 |

Tabelle 2

| (Teil 2/3): Biochemie | | | | |
|---|---|---|---|---|
| Beispiel | NA-Uptake-hemmung bei 1 μM | Verapamil-Bindung bei 10 μM | Diltiazem-Bindung bei 10 μM | BTX-Bindung bei 10 μM |
| **29** | 96 | | 72 | 88 |
| **30** | 98 | | 98 | 96 |
| **31** | 43 | | 90 | 85 |
| **32** | 100 | | 90 | 94 |
| **33** | 83 | | 86 | 98 |
| **34** | 96 | | 100 | 100 |
| **35** | 100 | | 87 | 92 |

Tabelle 2 (fortgesetzt)

| (Teil 2/3): Biochemie | | | | |
|---|---|---|---|---|
| Beispiel | NA-Uptake-hemmung bei 1 µM | Verapamil-Bindung bei 10 µM | Diltiazem-Bindung bei 10 µM | BTX-Bindung bei 10 µM |
| **36** | 91 | | | 98 |
| **37** | 100 | | 94 | 90 |
| **38** | 17 | | 67 | 97 |
| **39** | 61 | | 76 | 97 |
| **40** | 58 | | 86 | 98 |
| **41** | 100 | | 87 | 94 |
| **42** | 82 | | 50 | 89 |
| **43** | 14 | | 73 | 94 |
| **44** | 99 | | 71 | 95 |
| **45** | 89 | | 67 | 96 |
| **46** | 35 | | 93 | 96 |
| **47** | 100 | | 84 | 100 |
| **48** | 29 | | 86 | 96 |
| **49** | 100 | | 93 | 90 |
| **50** | 100 | | 93 | 95 |
| **51** | 10 | | 77 | 99 |
| **52** | 38 | | 98 | 99 |
| **53** | 49 | | 92 | 97 |
| **54** | 96 | | 76 | 98 |
| **55** | 38 | | 76 | 94 |
| **56** | 92 | | 88 | 99 |
| **57** | 99 | | 83 | 97 |
| **58** | 0 | | 80 | 100 |

Tabelle 2

| (Teil 3/3): Biochemie | | | | |
|---|---|---|---|---|
| Beispiel | NA-Uptake-hemmung bei 1 µM | Verapamil-Bindung bei 10 µM | Diltiazem-Bindung bei 10 µM | BTX-Bindung bei. 10 µM |
| **59** | 26 | | 64 | 86 |
| **60** | 56 | | 79 | 95 |
| **61** | 100 | | 89 | 98 |
| **62** | 80 | | 83 | 93 |
| **63** | 94 | | 95 | 99 |
| **64** | 36 | | 87 | 100 |
| **65** | 0 | | 72 | 78 |
| **66** | 15 | | 65 | 89 |

Tabelle 2 (fortgesetzt)

| (Teil 3/3): Biochemie | | | | |
|---|---|---|---|---|
| Beispiel | NA-Uptake-hemmung bei 1 μM | Verapamil-Bindung bei 10 μM | Diltiazem-Bindung bei 10 μM | BTX-Bindung bei. 10 μM |
| **67** | 83 | | 98 | 97 |
| **68** | 49 | | 88 | 98 |
| **69** | 76 | | 93 | 96 |
| **70** | 74 | | 91 | 93 |
| **71** | 88 | | 74 | 81 |
| **72** | 64 | | 97 | 99 |
| **73** | 61 | | 89 | 93 |
| **74** | 24 | | 83 | 93 |
| **75** | 0 | | 89 | 93 |
| **76** | 80 | | 73 | 90 |
| **77** | 27 | | 27 | 56 |
| **78** | 84 | | 95 | 96 |
| **79** | 58 | | 67 | 93 |
| **80** | 18 | | 100 | 100 |
| **81** | 66 | | 83 | 99 |
| **82** | 0 | | 85 | 98 |

**Beispiele**

Die folgenden Beispiele dienen zur näheren Erläuterung des erfindungsgemäßen Verfahrens.
Die Ausbeuten der hergestellten Verbindungen sind nicht optimiert.
Alle Temperaturen sind unkorrigiert.
Die Angabe Ether bedeutet Diethylether.
Als stationäre Phase für die Säulenchromatographie wurde Kieselgel 60 (0.040 - 0.063 mmm) der Firma E. Merck, Darmstadt, eingesetzt.
Die dünnschicht-chromatographischen Untersuchungen wurden mit HPTLC-Fertigplatten, Kieselgel 60 F 254, der Firma E. Merck, Darmstadt, durchgeführt.
Die Racemattrennungen wurden auf einer Chiracel OD Säule 250 x 4,6 mm mit Vorsäule der Firma Daicel durchgeführt.
Die Mischungsverhältnisse der Laufmittel für alle chromatographischen Untersuchungen sind stets in Volumen/Volumen angegeben.
RT bedeutet Raumtemperatur, Vol.% Volumenprozent, $m^2$ Massenprozent und %ee Enantiomerenüberschuß in Prozent.

**Beispiel 1**

2-(Dimethylaminophenylmethyl)-1-(3-methoxyphenyl)cyclohexanol, Hydrochlorid

1. Stufe

Benzylidendimethylammoniumchlorid

10 g ( 56 mmol ) N,N,N' ,N'-Tetramethyl-C-phenylmethandiamin (J. Am. Chem. Soc. 77 (1955) 1114-1116) wurden in 100 ml Ether gelöst und im Eisbad auf 0°C gekühlt. Es wurden unter Stickstoff 4,0 ml (56 mmol) Acetylchlorid zugetropft. Nach den ersten Tropfen fiel ein weißes Salz aus, die Temperatur erhöhte sich leicht. Nach 15 Stunden

bei RT wurde abdekantiert, der Feststoff dreimal mit je 100 ml Ether gewaschen, über eine Schutzgasfritte unter SLickstoff filtriert und im Ölpumpenvakuum bis zur Gewichtskonstanz getrocknet. Auf diese Weise wurden 7,7 g Benzylidendimethylammoniumchlorid (80,9% der Theorie) erhalten.

2. Stufe

2- (Dimethylaminophenylmethyl)cyclohexanon

7,1 ml (44 mmol) 1-(Pyrrolidino)-1-cyclohexen wurden in 45 ml Dichlormethan gelöst und unter Stickstoff mit einem Trockeneis/Isopropanol-Bad auf -70°C gekuhlt. Unter Ruhren wurden 7,5 g (44 mmol) Benzylidendimethylammoniumchlorid aus Stufe 1 zugegeben, die Mischung innerhalb von zwei bis drei Stunden auf -30°C erwärmt und 15 Stunden bei dieser Temperatur gelagert. Zur Aufarbeitung wurden 60 ml. halbkonzentrierte Salzsäure zugegeben und 5 Minuten nachgerührt. Bei RT wurde mit 50 ml Ether gewaschen, die wässrige Phase mit 440 ml Ammoniaklösung (25 Vol.%) versetzt und schnell dreimal mit je 150 ml Ether extrahiert. Die vereinigten organischen Extrakte wurden über Natriumsulfat getrocknet, filtriert und am Rotationsverdampfer ohne Wärmezufuhr (500 bis 10 mbar) eingeengt. Auf diese Weise wurden 10,1 g Rohbase (99,5% der Theorie) erhalten. 9,81 g (42,4 mmol) der Rohbase wurden in 83 ml 2-Butanon gelöst und nacheinander 0,76 ml (42,2 mmol) Wasser und 5,36 ml (42,4 mmol) Chlortrimethylsilan zugegeben. Der Ansatz wurde 15 Stunden bei RT aufbewahrt, der ausgefallene Feststoff abgesaugt, mit kleinen Portionen Ether gewaschen und im Ölpumpenvakuum bis zur Gewichtskonstanz getrocknet. Auf diese Weise wurden 8,92 g des Hydrochlorids von 2-(Dimethylaminophenylmethyl)cyclohexanon (78, 6% der Theorie) erhalten.

3. Stufe

2-(Dimethylaminophenylmethyl)-1-(3-methoxyphenyl)cyclohexanol, Hydrochlorid

1,08 g (44,5 mmol) Magnesiumspäne wurden in 10 ml Tetrahydrofuran p.a. gerührt. Es wurden 5,57 ml (44,5 mmol) 1 3-Bromanisol, gelost in 40 ml Tetrahydrofuran, so zugetropft, daß das Reaktionsgemisch leicht siedete. Nach beendeter Zugabe wurde eine Stunde bei RT nachgeruhrt. Aus 11 g (41,1 mmol) des nach Stufe 2 erhaltenen Hydrochlorids von 2-(Dimethylaminophenylmethyl)cyclohexanon wurde mit 100 ml Wasser und 10 ml Natronlauge (32 m%) die Base freigesetzt, dreimal mit je 100 ml Ether extrahiert, die vereinigten organischen Extrakte über Natriumsulfat getrocknet, filtriert und am Rotationsverdampfer ohne Wärmezufuhr (500 bis 10 mbar) eingeengt. 8,57 g (37 mmol) dieser Base wurden in 10 ml Tetrahydrofuran gelöst, zum Grignard-Ansatz getropft und 15 Stunden bei RT gerührt. Zur Aufarbeitung wurden 40 ml gesättigte Ammoniumchloridlosung bei Eisbadkühlung zugetropft und bei RT dreimal mit je 100 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 12,4 g Rohbase (99,0% der Theorie) erhalten. Die Rohbase wurde in 125 ml 2-Butanon gelöst und nacheinander 0,33 ml (18,3 mmol) Wasser und 4,63 ml (36,5 mmol) Chlortrimethylsilan zugegeben. Der Ansatz wurde 15 Stunden bei RT aufbewahrt, der ausgefallene Feststoff abgesaugt, mit kleinen Portionen Ether gewaschen und im Ölpumpenvakuum bis zur Gewichtskonstanz getrocknet. Auf diese Weise wurden 8,27 g 2-(Dimethylaminophenylmethyl)-1-(3-methoxyphenyl) cyclohexanol, Hydrochlorid (59,4% der Theorie) mit einem Schmelzpunkt von 227 - 229°C erhalten.

**Beispiel 2**

2-(Dimethylaminophenylmethyl)-1-(3-fluorphenyl)cyclohexanol, Hydrochlorid

0, 87 g (36,0 mmol) Magnesiumspäne wurden in 10 ml Ether p.a. gerührt. Es wurden 4, 02 ml (36, 0 mmol) 3-Bromfluorbenzol, gelöst in 30 ml Ether, so zugetropft, daß das Reaktionsgemisch leicht siedete. Nach beendeter Zugabe wurde eine Stunde bei RT nachgerührt. 7,0 g (30 mmol) des nach Beispiel 1 hergestellten 2-(Dimethylaminophenylmethyl)cyclohexanons wurden in 10 ml Ether gelösl, bei Eisbadkühlung zum Grignard-Ansatz getropft und 15 Stunden bei RT gerührt. Zur Aufarbeitung wurden 40 ml gesättigte Aminoniumchloridlöswig bei Eisbadkühlung zugegeben und bei RT dreimal mit je 100 ml Essigsäureethylester extrahiert. Die vereinigten organichen Extrakte wurden über Natriumsulfat getrocknet, filtriert und am Rotationsverdampfer (500 bis 10 mbar) eingeengt. Es wurden 9,27 g Rohbase (93,6% der Theorie) erhalten. Aus der Rohbase wurden nach Beispiel 1 (3. Stufe) mit Chlortrimethylsilan/ Wasser in 2-Butanon 6,04 g 2-(Dimethylaminophenylmethyl)-1-(3-fluorphenyl) cyclohexanol, Hydrochlorid (54,8% der Theorie) erhalten, das sich beim Erhitzen ab 140°C zersetzt.

**Beispiel 3**

2-(Dimethylaminophenylmethyl)-1-phenylcyclohexanol, Hydrochlorid

0,87 g (36,0 mmol) Magnesiumspäne wurden in 10 ml Ether p.a. gerührt. Es wurden 3,8 ml (36,0 mmol) Brombenzol, gelöst in 30 ml Ether, so zugetropft, daß das Reaktionsgemisch leicht siedete. Nach beendeter Zugabe wurde eine Stunde bei RT nachgeruhrt . 7,0 g (30 mmol) des nach Beispiel 1 hergestellten 2-(Dimethylaminophenylmethyl)-cyclohexanons wurden in 10 ml Ether gelöst, bei Eisbadkuhlung zum Grignard-Ansatz getropft und 15 Stunden bei RT gerührt. Zur Aufarbeitung wurden 40 ml gesättigte Ammoniumchloridlösung bei Eisbadkühlung zugegeben und bei RT dreimal mit je 100 ml Essigsaureethylester extrahiert. Die vereinigten organischen Extrakte wurden über Natriumsulfat getrocknet, filtriert und am Rotationsverdampfer (500 bis 10 mbar) eingeengt. Es wurden 8,99 g Rohbase (96,0 % der. Theorie) erhalten. Aus der Rohbase wurden nach Beispiel 1 (3. Stufe) mit Ciilortrimetliylsilaii/Wasser in 2-Butanon 6, 85 g 2-(Dimethylaminophenylmethyl)-1-phenylcyclohexanol, Hydrochlorid (65,4 % der Theorie) erhalten, das sich beim Erhitzen ab 140°C zersetzt.

**Beispiel 4**

3-[2-(Dimethylaminophenylmethyl)-1-hydroxycyclohexyl]-phenol, Hydrochlorid

1. Stufe

(3-Bromphenoxy)trimethylsilan

Zu 49,3 g (0,285 mol) 3-Bromphenol wurden unter Stickstoff 23,4 g (0,145 mol) Hexamethylsilazan getropft, die Lösung langsam auf 150°C erhitzt und 1 Stunde bis zum Ende der Gasentwicklung geruhrt. Zur Reinigung wurde bei 6 inbar destilliert, die Hauptfraktion siedete bei 79 °C. Auf diese Weise wurden 66,7 g (3-Bromphenoxy)trimethylsilan (95,5% der Theorie) erhalten.

2. Stufe

3-[2-(Dimethylaminophenylmethyl)-1-hydroxycyclohexyl]-phenol, Hydrochlorid

1,25 g (51,6 mmol) Magnesiumspäne wurden in 10 ml Ether p.a. gerührt. Es wurden 12,7 g (51, 6 mmol) (3-Bromphenoxy)trimethylsilan aus Stufe 1, gelöst in 30 ml Ether, so zugetropft, daß das Reaktionsgemisch leicht siedete. Nach beendeter Zugabe wurde eine Stunde bei RT nachgerührt. 10, 0 g (43,0 mmol) des nach Beispiel 1 hergestellten 2- (Dimethylaminophenylmethylcyclohexanons wurden in 10 ml Ether gelöst, bei Eisbadkühlung zum Grignard-Ansatz getropft und 15 Stunden bei RT gerührt. Zur Aufarbeitung wurden 150 ml Salzsaure (1 M) zugegeben, es entstanden zwei Phasen und ein in Aceton lösliches Öl. Die wässrige Phase und das in Aceton gelöste Öl wurden mit Natriumhydrogencarbonat leicht alkalisch gestellt (pH ca. 8) und bei RT dreimal mit je 100 ml Essigsäureethylester extrahiert. Die vereinigten organischen Extrakte wurden uber Natriumsulfat getrocknet, filtriert und am Rotationsverdampfer (500 bis 10 mbar) eingeengt. Es wurden 7,51 g Rohbase erhalten, die auf eine 5,5 x 50 cm Säule, gefüllt mit Kieselgel, gegeben wurde. Die Elution mit Essigsäureethylester/Methanol 24:1 ergab 0,85 g Base, aus der nach Beispiel 1 (3. Stufe) mit Chlortrimethylsilan/Wasser in 2-Butanon ein Hydrochlorid gefällt wurde. Hieraus wurde mit 10 ml Wasser und Natriumhydrogencarbonat die Base freigesetzt (pH ca. 8), dreimal mit je 30 ml Ether extrahiert, die vereinigten organischen Extrakte über Natriumsulfat getrocknet, filtriert und am Rotationsverdampfer (500 bis 10 mbar) eingeengt. Die erhaltenen 670 mg Base wurden auf eine 3 x 17 cm Säule, gefullt mit Kieselgel, gegeben. Die Elution mit Essigsäureethylester/n-Hexan 2:3 ergab 580 mg Base, aus der mit 0,16 ml Salzsäure (32 m%) und 5 ml Aceton 0,53 g des 3-[2-(Dimethylaminophenylmethyl)-1-hydroxycyclohexyl]phenol, Hydrochiorids erhalten wurden (3,4% der Theorie), das sich beim Erhitzen ab 140°C zersetzt.

**Beispiel 5**

2-(Dimethylaminophenylmethyl)-1- (4-methoxyphenyl)cyclohexanol, Hydrochlorid

0,88 g (36,3 mmol) Magnesiumspäne wurden in 10 ml Tetrahydrofuran p.a. gerührt. Es wurden 4,55 ml (36,3 mmol) 4-Bromanisol, gelost in 30 ml Tetrahydrofuran, so zugetropft, daß das Reaktionsgemisch leicht siedete. Nach beendeter Zugabe wurde eine Stunde bei RT nachgeruhrt. 7,0 g (30,3 mmol) des nach Beispiel 1 hergestellten 2-(Dimethylaminophenylmethyl) cyclohexanons wurden in 10 ml Tetrahydrofuran gelöst, bei Eisbadkühlung zum Grignard-Ansatz getropft und 15 Stunden bei RT gerührt. Zur Aufarbeitung wurden 40 ml gesättigte Ammoniumchloridlösung bei Eisbadkühlung zugegeben und bei RT dreimal mit je 100 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 10,5 g Rohbase (102% der Theorie) erhalten. Aus der Rohbase wurden nach Beispiel 1 (3. Stufe) mit Chlortrimethylsilan/Wasser in 2-Butanon 4,24 g 2-(Di-methylaminophenylmethyl)-1-(4-methoxyphenyl) cyclohexanol, Hydrochlorid (37,2% der Theorie) erhalten, das sich beim Erhitzen ab 150°C zersetzt.

**Beispiel 6**

1-(4-Chlorphenyl )-2-(dimethylaminophenylmethyl)cyclohexanol, Hydrochlorid

0,63 g (25,9 mmol) Magnesiumspäne wurden in 10 ml Ether p.a. gerührt. Es wurden 4,97 g (25,9 mmol) 4-Bromchlorbenzol, gelöst in 30 ml Ether, so zugetropft, daß das Reaktionsgemisch leicht siedete. Nach beendeter Zugabe wurde eine Stunde bei RT nachgerührt. 5,0 g (21,6 mmol) des nach Beispiel 1 hergestellten 2-(Dimethylaminophenylmethyl)cyclohexanons wurden in 10 ml Ether gelöst, bei Eisbadkühlung zum Grignard-Ansatz getropft und 15 Stunden bei RT gerührt. Zur Aufarbeitung wurden 40 ml gesättigte Ammoniumchloridlösung bei Eisbadkuhlung zugegeben und bei RT dreimal mit je 100 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 7,24 g Rohbase (97,4% der Theorie) erhalten. Aus der Rohbase wurden nach Beispiel 1 (3. Stufe) mit Chlortrimethylsilan/Wasser /Wasser in 2-Butanon 5,43 g 1-(4-Chlorphenyl)-2-(dimethylaminophenylmethyl)cyclohexanol, Hydrochlorid (66,0 % der Theorie) erhalten, das sich beim Erhitzen ab 175°C zersetzt.

**Beispiel 7**

2-(Dimethylaminophenylmethyl)-1-(4-fluorphenyl)cyclohexanol, Hydrochlorid

0,50 g (20,7 mmol) Magnesiumspäne wurden in 10 ml Ether p . a . gerührt. Es wurden 2,28 ml (20,7 mmol) 4-Bromfluorbenzol, gelöst in 20 ml Ether, so zugetropft, daß das Reaktionsgemisch leicht siedete. Nach beendeter Zugabe wurde eine Stunde bei RT nachgeruhrt. 4,0 g (17,3 mmol) des nach Beispiel 1 hergestellten 2-(Dimethylaminophenylmethyl)cyclohexanons wurden in 10 ml Ether gelöst, bei Eisbadkuhlung zum Grignard-Ansatz getropft und 15 Stunden bei RT gerührt. Zur Aufarbeitung wurden 30 ml gesättigte Ammoniumchloridlösung bei Eisbadkühlung zugegeben und bei RT dreimal mit je 80 ml Essigsäureethylester extrahiert.

Die vereinigten organischen Extrakte wurden über Natriumsulfat getrocknet, filtriert und am Rotationsverdampfer (500 bis 10 mbar) eingeengt. Es wurden 5,50 g Rohbase (97,2% der Theorie) erhalten. Aus der Rohbase wurden nach Beispiel 1 (3. Stufe) mit Chlortrimethylsilan/Wasser in 2-Butanon 3,61 g 2-(Dimethylaminophenylmethyl)-1-(4-fluorphenyl)cyclohexanol, Hydrochlorid (57,4% der Theorie) erhalten, das sich beim Erhitzen ab 150°C zersetzt.

**Beispiel 8**

2-(Dimethylaminophenylmethyl)-1-p-tolylcyclohexanol, Hydrochlorid

0,50 g (20,7 mmol) Magnesiumspäne wurden in 10 ml Ether p.a. gerührt. Es wurden 2,55 ml (20,7 mmol) 4-Bromtoluol, gelöst in 20 ml Ether, so zugetropft, daß das Reaktionsgemisch leicht siedete. Nach beendeter Zugabe wurde eine Stunde bei RT nachgerührt. 4,0 g (17,3 mmol) des nach Beispiel 1 hergestellten 2-(Dimethylaminophenylmethyl)-cyclohexanons wurden in 10 ml Ether gelöst, bei Eisbadkühlung zum Grignard-Ansatz getropft und 15 Stunden bei RT gerührt. Zur Aufarbeitung wurden 30 ml gesättigte Ammoniumchloridlösung bei Eisbadkühlung zugegeben und bei RT dreimal mit je 80 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 5,35 g Rohbase (95,7% der Theorie) erhalten. Aus der Rohbase wurden nach Beispiel 1 (3. Stufe) mit Chlortrimethylsilan/Wasser in 2-Butanon 1, 73 g 2- (Dimethylaminophenylmethyl) -1-p-tolylcyclohexanol, Hydrochlorid (27,8% der Theorie) mit einem Schmelzpunkt von 168 - 169°C erhalten.

**Beispiel 9**

1-(3-Chlorphenyl)-2-(dimethylamino-(3-methoxyphenyl)-methyl]cyclohexanol, Hydrochlorid

1. Stufe

C-(3-Methoxyphenyl)-N,N,N',N'-tetramethylmethandiamin

18,3 3 ml (0,15 mol) 3-Anisaldehyd wurden mit 38 ml (0,30 mol) Dimethylaminlösung (40 m% in Wasser) unter Rühren fur fünf Stunden auf 50°C erwärmt und anschließend für 15 Stunden bei RT weitergerührt. Zur Aufarbeitung wurden 20 ml gesättigte Kaliumcarbonat-Lösung und festes Kaliumcarbonat zugegeben, bis ein pH von ca. 9 erreicht war. Es wurde dreimal mit je 200 ml Essigsäureethylester extrahiert. Die vereinigten organischen Extrakte wurden über Kaliumcarbonat getrocknet, filtriert und am Rotationsverdampfer (500 bis 10 mbar) eingeengt. Auf diese Weise wurden 27,0 g C-(3-Methoxyphenyl)-N,N,N',N'-tetramethylmethandiamin (86,3% der Theorie) erhalten.

2. Stufe

(3-Methoxybenzyliden)dimethylammoniumchlorid

30 g (144 mmol) C- (3-Methoxyphenyl) -N, N, N' , M' -tetramethylmethandiamin aus Stufe 1 wurden in 200 ml Ether gelöst und im Eisbad (Methanol/Eis 1:1) auf -10°C gekühlt. Unter Stickstoff wurden 10,3 ml (144 mmol) Acetylchlorid zugetropft. Es fiel ein weißes Salz aus, die Temperatur erhöhte sich leicht. Nach 15 Stunden bei RT wurde abdekantiert, der Feststoff dreimal mit je 100 ml Ether gewaschen, über eine Schutzgasfritte unter Stickstoff filtriert und im Ölpumpenvakuum bis zur Gewichtskonstanz getrocknet. Auf diese Weise wurden 19,8 g (3-Methoxybenzyliden)dimethylammoniumchlorid (68,8% der Theorie) erhalten.

3. Stufe

2-[Dimethylamino-(3-methoxyphenyl)methyl]cyclohexanon

15,3 ml (95 mmol) 1-(Pyrrolidino)-1-cyclohexen wurden in 100 ml Dichlormethan gelöst und unter Stickstoff mit einem Trockeneis/Isopropanol-Bad auf -70°C gekühlt. Unter Rühren wurden 19 g (95 mmol) (3-Methoxybenzyliden) dimethylammoniumchlorid aus Stufe 2 zugegeben, die Mischung innerhalb von zwei bis drei Stunden auf -30°C erwärmt und 15 Stunden bei dieser Temperatur gelagert. Zur Aufarbeitung wurden 60 ml halbkonzentrierte Salzsäure zugegeben und 5 Minuten nachgerührt. Bei RT wurde mit 50 ml Ether extrahiert, die wässrige Phase mit 100 ml Ammoniaklösung (25 Vol.%) versetzt und schnell dreimal mit je 200 ml Ether extrahiert. Die vereinigten organischen Extrakte wurden über Natriumsulfat getrocknet, filtriert und am Rotationsverdampfer ohne Wärmezufuhr (500 bis 10 mbar) eingeengt. Auf diese Weise wurden 19,1 g Rohbase (76,6% der Theorie) erhalten. Aus der Rohbase wurden nach Beispiel 1 (2. Stufe) mit Chlortrimethylsilan/Wasser in 2-Butanon 18,0 g des Hydrochlorids von 2-[Dimethylamino-(3-methoxyphenyl)methyl]-cyclohexanon (63,7% der Theorie) mit einem Schmelzpunkt von 142°C erhalten.

4. Stufe

1-(3-Chlorphenyl)-2-[dimethylamino-(3-methoxyphenyl)-methyl]cyclohexanol , Hydrochlorid

0,55 g (22,4 mmol) Magnesiumspäne wurden in 10 ml Ether p.a. gerührt. Es wurden 2,6 ml (22,4 mmol) 3-Bromchlorbenzol, gelöst in 20 ml Ether, so zugetropft, daß das Reaktionsgemisch leicht siedete. Nach beendeter Zugabe wurde eine Stunde bei RT nachgerührt. Aus 6 g (20,1 mmol) des nach Stufe 3 erhaltenen Hydrochlorids von 2-[Dimethylamino-(3-methoxyphenyl)methyl]cyclohexanon wurde mit 60 ml Wasser und 5 ml Natronlauge (32 m%) die Base freigesetzt, dreimal mit je 60 ml Ether extrahiert, die vereinigten organischen Extrakte uber Natriumsulfat getrocknet, filtriert und am Rotationsverdampfer ohne Wärmezufuhr (500 bis 10 mbar) eingeengt. 4,9 g (18,7 mmol) dieser Base wurden in 10 ml Ether gelöst, bei Eisbadkühlung zum Grignard-Ansatz getropft und 15 Stunden bei RT gerührt. Zur Aufarbeitung wurden 30 ml gesättigte Ammoniumchloridlösung bei Eisbadkühlung zugegeben und bei RT dreimal mit je 80 ml Essigsaureethylester extrahiert. Die vereinigten organischen Extrakte wurden über Natriumsulfat getrocknet, filtriert und am Rotationsverdampfer (500 bis 10 mbar) eingeengt. Es wurden 6,51 g Rohbase (100% der Theorie) erhalten. Aus der Rohbase wurden nach Beispiel 1 (3. Stufe) mit Chlortrimethylsilan/Wasser in 2-Butanon 5,0 g 1-(3-Chlorphenyl)-2-[dimethylamino-(3-methoxyphenyl)methyl]cyclohexanol, Hydrochlorid (70,1% der Theorie) mit einem Schmelzpunkt von 131 - 133°C erhalten.

**Beispiel 10**

1-(4-Dimethylaminophenyl)-2-[dimethylaminophenylmethyl]-cyclohexanol, Hydrochlorid

0,50 g (20,7 mmol) Magnesiumspäne wurden in 5 ml Tetrahydrofuran p.a. geruhrt. Es wurden 4,14 g (20,7 mmol) 4-Brom-N,N-dimethylanilin, gelöst in 10 ml Tetrahydrofuran, so zugetropft, daß das Reaktionsgemisch leicht siedete. Nach beendeter Zugabe wurde eine Stunde bei 55 °C nachgerührt. 4,0 g (17,3 mmol) des nach Beispiel 1 hergestellten 2-(Dimethylaminophenylmethyl) cyclohexanons wurden in 10 ml Tetrahydrofuran gelöst, bei Eisbadkühlung zum Grignard-Ansatz getropft und 15 Stunden bei RT gerührt. Zur Aufarbeitung wurden 40 ml gesattigte Ammoniumchloridlösung bei Eisbadkuhlung zugegeben und bei RT dreimal mit je 100 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 6,24 g Hohbase (102% der Theorie) erhalten, aus der nach Beispiel 1 (3. Stufe) mit Chlortrimethylsilan/Wasser in 2-Butanon ein Hydrochlorid gefallt wurde. Hieraus wurde mit 30 ml Wasser und 4 ml Natronlauge (32 m%) die Base freigesetzt, dreimal mit je 30 ml Ether extrahiert, die vereinigten organischen Extrakte über Natriumsulfat getrocknet, filtriert und am Rotationsverdampfer (500 bis 10 mbar) eingeengt. Es wurden 1,90 g Rohbase (26,9% der Theorie) erhalten. Aus der Pohbase wurden nach Beispiel 1 (3. Stofe) mit Chiortrimetbylsilan/ Wasser in 2-Butanon 0,88 g 1-(4-Dimethylaminophenyl)-2-[dimethyl-aminophenylmethyl] cyclohexanol, Hydrochlorid (10, 9%. der Theorie) mit einem Schmelzpunkt von 124 - 125°C erhalten.

**Beispiel 11**

1-Benzo[1, 3]dioxol-4-yl-2-(dimethylaminophenylmethyl)-cyclohexanol, Hydrochlorid

2,61 g (13 mmol) 4-Brom-1,2-methylendioxybenzol wurden in 10 ml Tetrahydrofuran gelöst und unter Stickstoff mit einem Trockeneis/Isopropanol-Bad auf -70°C gekühlt. Unter Rühren wurden 9,35 ml (15 mmol) n-Butyllithium (1,6 M in Hexan) so zugetropft, daß die Temperatur nicht über -60°C stieg. Es wurde 30 Minuten nachgerührt und dann 3,0 g (13 mmol) des nach Beispiel 1 hergestellten 2-(Dimethylaminophenylmethyl)cyclohexanons, gelöst in 10 ml Tetrathydrofuran, bei Eisbadkuhlung zugetropft und innerhalb zwei Stunden auf RT erwärmt. Zur Aufarbeitung wurden 20 ml gesättigte Ammoniumchloridlösung bei. Eisbadkuhlung zugegeben und bei RT dreimal mit je 70 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 4, 67 g Rohbase (102% der Theorie) erhalten. Aus der Rohbase wurden nach Beispiel 1 (3. Stufe) mit Chlortrimethylsilan/Wasser in 2-Butanon 3,05 g 1-Benzo [1,3]-dioxol-4-yl-2-(dimethylaminophenylmethyl)cyclohexanol, Hydrochlorid (60, 3% der Theorie) mit einem Schmelzpunkt von 209°C erhalten.

**Beispiel 12**

(3,4-Dimethoxyphenyl)-2-(dimethylaminophenylmethyl)cyclohexanol, Hydrochlorid

2,82 g (13 mmol) 3-Broinveratol wurden in 20 ml Tetrahydrofuran gelöst irid unter Stickstoff mit einem Trockeneis/Isopropanol-Bad auf -70°C gekühlt. Unter Rühren wurden 9,35 ml (15 mmol) n-Butyllithium (1,6 M in Hexan) so zugetropft, daß die Temperatur nicht über -60°C stieg. Es wurde 30 Minuten nachgerührt und dann 3,0 g (13 mmol) des nach Beispiel 1 hergestellten 2-(Dimethylaminophenylmethyl) cyclohexanons, gelost in 10 ml Tetrahydrofuran, bei Eisbadkühlung zugetropft und innerhalb zwei Stunden auf RT erwärmt. Zur Aufarbeitung wurden 20 ml gesättigte Ammoniumchloridlösung bei Eisbadkühlung zugegeben und bei RT dreimal mit je 70 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 5,09 g Rohbase (106 % der Theorie) erhalten. Aus der Rohbase wurden nach Beispiel 1 (3. Stufe) mit Chlortrimethylsilan/Wasser in 2-Butanon 3,73 g (3, 4-Dimethoxyphenyl)-2-(dimethyl-aminophenylmethyl)cyclohexanol, Hydrochlorid (70,8 % der Theorie) mit einem Schmelzpunkt von 205 - 207°C erhalten.

**Beispiel 13**

2-(Dimethylaminophenylmethyl)-1-(3-methoxybenzyl)cyclohexanol, Hydrochlorid

0,63 g (25,9 nmol) Magnesiuinspäne wurden in 10 ml Ether p.a. gerührt . Es wurden 5,21 g (25,9 mmol) 3-Methoxybenzylbromid, gelöst in 20 ml Ether, so zugetropft, daß das Reaktionsgemisch leicht siedete. Nach beendeter Zugabe wurde eine Stunde bei RT nachgerührt. 5,0 g (21,6 mmol) des nach Beispiel 1 hergestellten 2-(Dimethylaminophenylmethyl) cyclohexanons wurden in 10 ml Ether gelöst, bei Eisbadkühlung zum Grignard-Ansatz getropft und

15 Stunden bei RT gerührt. Zur Aufarbeitung wurden 40 ml gesättigte Ammoniumchloridlösung bei Eisbadkühlung zugegeben und bei RT dreimal mit je 80 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 7,53 g Rohbase (98,6 % der Theorie) erhalten. Aus der Rohbase wurden nach Beispiel 1 (3. Stufe) mit Chlortrimethylsilan/Wasser in 2-Butanon 4,45 g 2-(Dimethylaminophenylmethyl-1-(3-methoxybenzyl)cyclohexanol, Hydrochlorid ( 52,8% der Theorie) erhalten, das sich beim Erhitzen ab 160°C zersetzt.

**Beispiel 14**

1-Benzyl-2-(dimethylaminophenylmethyl)cyclohexanol, Hydrochlorid

0,63 g (25,9 mmol) Magnesiumspäne wurden in 10 ml Ether p.a. gerührt. Es wurden 4,43 g (25,9 mmol) Benzylbromid, gelöst in 20 ml Ether, so zugetropft, daß das Reaktionsgemisch leicht siedete. Nach beendeter Zugabe wurde eine Stunde bei RT nachgerührt. 5,0 g (21,6 mmol) des nach Beispiel 1 hergestellten 2-(Dimethylaminophenylmethyl)-cyclohexanons wurden in 10 ml Ether gelöst, bei Eisbadkuhlung zum Grignard-Ansatz getropft und 15 Stunden bei RT gerührt. Zur Aufarbeitung wurden 40 ml gesattigte Ammoniumchloridlosung bei Eisbadkuhlung zugegeben und bei RT dreimal mit je 80 ml Essigsaureethylester extrahiert. Die vereinigten organischen Extrakte wurden über Natriumsulfat getrocknet, filtriert und am Rotationsverdampfer (500 bis 10 mbar) eingeengt. Es wurden 6,84 g Rohbase (97,9% der Theorie) erhalten. Aus der Rohbase wurden nach Beispiel 1 (3. Stufe) mit Chlortrimethylsilan/Wasser in 2-Butanon 1,61 g 1-Benzyl-2- (dimethylaminophenylmethyl) cyclohexanol, Hydrochlorid (20,7% der Theorie) mit einem Schmelzpunkt von 223 - 225°C erhalten.

**Beispiel 15**

2-(Dimetylaminophenylmethyl)-1-(4-fluor-3-trifluormethylphenyl)cyclohexanol, Hydrochlorid

0,25 g (10,3 mmol) Magnesiumspäne wurden in 15 ml Ether p.a. gerührt. Es wurden 2,2 g (10,3 mmol) 5-Brom-2-fluorbenzotrifluorid, gelöst in 15 ml Ether, so zugetropft, daß das Reaktionsgemisch leicht siedete. Nach beendeter Zugabe wurde eine Stunde bei RT nachgerührt. 1,80 g (8,57 mmol) des nach Beispiel 1 hergestellten 2-(Dimethylaminophenylmethyl)cyclohexanons wurden in 15 ml Ether gelösl, bei Eisbadkühlung zum Grignard-Ansatz getropft und 15 Stunden bei RT gerührt. Zur Aufarbeitung wurden 20 ml gesättigte Ammoniumchloridlosung bei Eisbadkühlung zugegeben und bei RT dreimal mit je 40 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 3,9 g Rohbase (126% der Theorie) erhalten. Aus der Rohbase wurden nach Beispiel 1 (3. Stufe) mit Chlortrimethylsilan/Wasser in 2-Butanon 2,14 g 2-(Dimethylaminophenylmethyl)-1-(4-fluor-3-trifluormethylphenyl)cyclohexanol, Hydrochlorid (63,7% der Theorie) mit einem Schmelzpunkt von 234 - 237°C erhalten.

**Beispiel 16**

2-(Dimethylaminophenylmethyl)-1-(4-trifluormethoxybenzyl)-cyclohexanol, Hydrochlorid

0,10 g (4,2 mmol) Magnesiumspäne wurden in 10 ml ELher p.a. gerührt. Es wurden 1,0 g (4,2 mmol) 4-(Trifluormethoxy)benzylbromid, gelöst in 10 ml Ether, so zugetropft, daß das Reaktionsgemisch leicht siedete. Nach beendeter Zugabe wurde eine Stunde bei RT nachgerührt. 0,8 g (3,5 nunol) des nach Beispiel 1 hergestellten 2-(Dimethylaminophenylmethyl) cyclohexanons wurden in 10 ml Ether gelöst, bei Eisbadkühlung zum Grignard-Ansatz getropft und 15 Stunden bei RT geruhrt. Zur Aufarbeitung wurden 20 ml gesättigte Ammoniumchloridlösung bei Eisbadkuhlung zugegeben und bei RT dreimal mit je 30 ml Essigsaureethylester extrahiert. Die vereinigten organischen Extrakte wurden über Natriumsulfat getrocknet, filtriert und am Rotationsverdampfer (500 bis 10 mbar) eingeengt. Es wurden 1,65 g Rohbase (121%der Theorie) erhalten. Aus der Rohbase wurden nach Beispiel 1 (3. Stufe) mit Chlortrimethylsilan/Wasser in 2-Butanon 0,64 g 2-(Dimethylaminophenylmethyl)-1-(4-trifluormethoxybenzyl)cyclohexanol, Hydrochlorid (41,7% der Theorie) erhalten, das sich beim Erhitzen ab 178°C zersetzt.

**Beispiel 17**

2-(Dimethylaminophenylmethy)-1-furan-3-ylcyclohexanol, Hydrochlorid

1,0 g (6,8 mmol) 3-Bromfuran wurden in 10 ml Tetrahydrofuran gelöst und unter Stickstoff mit einem Trokkeneis/Isopropanol.-Bad auf -70 °C gekuhlt. Unter Ruhren wurden 5,1 ml (8,1 mmol) n-Butyllithium (1,6 M in Hexan)

so zugetropft, daß die Temperatur nicht über -60°C stieg. Es wurde 30 Minuten nachgerührt und dann 1, 57 g (6,8 mmol) des nach Beispiel 1 hergestellten 2-(Dimethylaminophenylmethyl)cyclohexanons, gelöst in 10 ml Tetrahydrofuran, bei Eisbadkühlung zugetropft und innerhalb zwei Stunden auf RT erwärmt. Zur Aufarbeitung wurden 20 ml gesättigte Ammoniumchloridlosung bei Eisbadkühlung zugegeben und bei RT dreimal mit je 50 ml Essigsäureethylester extrahiert. Die vereinigten organischen Extrakte wurden über Natriumsulfat getrocknet, filtriert und am Rotationverdampfer (500 bis 10 mbar) eingeengt. Es wurden 2,15 g Rohbase (106% der Theorie) erhalten, aus der nach Beispiel 1 (3. Stufe) mit Chlortrimethylsilan/Wasser in 2-Butanon ein Hydrochlorid gefällt wurde. Hieraus wurde mit 20 ml Wasser und 3 ml Natronlauge (32 m%) die Base freigesetzt, dreimal mit je 30 ml Ether extrahiert, die vereinigten organischen Extrakte über Natriumsulfat gerocknet, filtriert und am Rotationsverdampfer (500 bis 10 mbar) eingeengt. Es wurden 1,29 g Rohbase (63,4% der Theorie) erhalten, die auf eine 4 x 30 cm Säule, gefüllt mit Kieselgel, gegeben wurde. Die Elution mit Diisopropylether/Methanol 4,7:0,3 ergab 0,33 g Base, aus der nach Beispiel 1 (3. Stufe) mit Chlortrimethylsilan/Wasser in 2-Butanon 0,28 g 2-(Dimethylaminophenylmethyl)-1-furan-3-ylcyclohexanol, Hydrochlorid (12,4%. der Theorie) erhalten wurden, das sich beim Erhitzen ab 130°C zersetzt.

**Beispiel 18**

1-Butyl-2-(dimethylaminophenylmethyl)cyclohexanol, Hydrochlorid

2,43 g (7,2 mmol)(1-Bromnaphthalin-2-yloxy)-tert.-butyldimethylsilan wurden in 10 ml Tetrahydrofuran gelost und im Trockeneis/Isopropanol-Bad auf -70°C gekühlt. Unter Rühren und Stickstoff wurden 5,4 ml (8,6 mmol) n-Butyllithium (1,6 M in Hexan) zugetropft und 30 Minuten nachgerührt. 2,7 g (7,2 mmol) des nach Beispiel 1 hergestellten 2-(Dimethylaminophenylmethyl)cyclohexanons, gelöst in 10 ml Tetrahydrofuran, wurden zugetropft und der Ansatz innerhalb von zwei Stunden auf RT erwärmt. Zur Aufarbeitung wurden 20 ml gesattigte Ammoniumchloridlösung bei Eisbadkühlung zugegeben und bei RT dreimal mit je 50 ml Essigsäureethylester extrahiert. Die vereinigten organischen Extrakte wurden uber Natriumsulfat getrocknet, filtriert und am Rotationsverdampfer (500 bis 10 mbar) eingeengt. Es wurden 4,65 g Rohbase erhalten (223% der Theorie), die mit 10 ml n-Hexan, 9 ml Methanol und 4,7 ml Salzsaure (2 N) versetzt wurden. Die wässrige Phase wurde abgetrennt, das Methanol am Rotationsverdampfer (500 bis 10 mbar) abdestilliert, die Lösung mit Natriumcarbonatlösung (1 M) alkalisch gestellt (pH ca. 9) und bei RT dreimal mit je 50 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 2,34 g Rohbase (53,4% der Theorie) erhalten, die auf eine 3,5 x 15 cm Saule, gefüllt mit kieselgel, gegeben wurde. Die Elution mit Essigsäureethylester/n-Hexan 2: 3 ergab 0,23 g Base, aus der nach Beispiel 1 (3. Stufe) mit Chlortrirnethylsilan/Wasser in 2-Butanon 0,13 g 1-Butyl-2-(dimethylaminophenylmethyl)cyclohexanol, Hydrochlorid (5,5 % der Theorie) erhalten wurden, das sich beim Erhitzen ab 110°C zersetzt.

**Beispiel 19**

(-)-1-(3,4-Dichlorphenyl)-2-(dimethylaminophenylimethyl)-cyclohexanol, Hydrochlorid und

(+)-1-(3,4-Dichlorphenyl)-2-(dimethylaminophenylmethyl)-cyclohexanol, Hydrochlorid

0,78 g (32,1 mmol) Magnesiumspäne wurden in 10 ml Ether p.a. gerührt. Es wurden 7,13 g (31,0 mmol) 1-Brom-3,4-dichlorbenzol, gelost in 20 ml Ether, so zugetropft, daß das Reaktionsgemisch leicht siedete. Nach beendeter Zugabe wurde eine Stunde bei RT nachgerührt. 6,0 g (26,0 mmol) des nach Beispiel 1 hergestellten 2-(Dimethylaminophenylmethyl) cyclohexanons wurden in 15 ml Ether gelöst, bei Eisbadkuhlung zum Grignard-Ansatz getropft und 15 Stunden bei RT gerührt. Zur Aufarbeitung wurden 40 ml gesättigte Ammoniumchloridlösung bei Eisbadkühlung zugegeben und bei RT dreimal mit je 100 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 10, 4 g Rohbase (106% der Theorie) erhalten.

1,96 g (5,2 mmol) dieser Base wurden in 20 ml 2-Butanon gelost, 0,78 g L(+)-Weinsaure (5,2 mmol) zugegeben, unter Erwärmen gelöst und eine Woche bei 4°C gelagert, wobei ein weißer Niederschlag entstand. Dieser wurde abfiltriert, mehrmals mit wenig 2-Butanon und Ether gewaschen, in 20 ml Wasser gelöst, mit 2 ml Natronlauge (32 m%) die Base freigesetzt und dreimal mit je 30 ml Essigsäureethylester extrahiert. Die vereinigten organischen Extrakte wurden über Natriumsulfat getrocknet, filtriert, und am Rotationsverdampfer (500 bis 10 mbar) eingeengt. Es wurden 0,69 g (1,8 mmol) Rohbase erhalten. Die optische Reinheit wurde per HPLC bestimmt. Die Rohbase wurde in Hexan-Isopropanol - Diethylamin (990 : 10 : 1) gelöst (0,1 Vol.%), hiervon 20 µl eingespritzt und mit dem Fließmittel Hexan/Isopropanol/Diethylamin (990 : 10 : 1) bei einem Fluß von 1 ml/min über eine Säule Chiracel OD 250 x 4,6 mm mit Vorsäule (Firma Daicel) eluiert. Detektiert wurde bei einer Wellenlänge von 254 nm. Der Reinheitsgrad lag bei 98,8

%ee. Aus der Rohbase wurden nach Beispiel 1 (3. Stufe) mit Chlortrimethylsilan/Wasser in 2-Butanon 0,32 g (0,77 mmol) (-)-1-(3, 4-Dichlorphenyl)-2-(dimethylaminophenylmethyl)cyclohexanol, Hydrochlorid (29, 7% der Theorie) mit einem Drehwert von $[\alpha]_D^{20}$ = -27,9 (c = 1,097 in Methanol) erhalten.

Die Mutterlauge des abfiltrierten Base/Weinsaure-Gemisches wurde eingeengt und wie bei dem zuvor erhaltenen Niederschlag die Base mit Natronlauge freigesetzt und anschließend ein Hydrochlorid mit Chlortrimethylsilan/Wasser in 2-Butanon gefällt. Hieraus wurde wiederum mit Natronlauge die Base freigesetzt und 0,62 g (1,6 mmol) Rohbase erhalten. Diese Rohbase wurde in 6 ml 2-Butanon gelöst, 0,25 g (1,6 mmol) D(-)-Weinsaure zugegeben, die entstandene Fallung abfiltriert, mehrmals mit wenig 2-Butanon und Ether gewaschen, in 20 ml Wasser gelöst, mit 2 ml Natronlauge (32 m%) die Base freigesetzt und dreimal mit je 30 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 0,33 g (0,87 mmol) Rohbase erhalten. Die optische Reinheit wurde wie oben per HPLC bestimmt. Der Reinheitsgrad lag bei 98,5 %ee. Aus der Rohbase wurden nach Beispiel 1 (3. Stufe) mit Chlortrimethylsilan/Wasser in 2-Butanon 0,12 g (0,29 mmol) (+)-1-(3,4-Dichlorphenyl)-2-(dimethylaminophenylmethyl)cyclohexanol, Hydrochlorid (11,1% der Theorie) mit einem Drehwert von $[\alpha]_D^{20}$= +27, 3 (c = 1,081 in Methanol) erhalten.

**Beispiel 20**

4-[2-(Dimethylaminophenylmethyl)-1-hydroxycyclohexyl]-phenol, Hydrochlorid

0,38 g (15,5 mmol) Magnesiumspäne wurden in 5 ml Tetrahydrofuran p.a. gerührt. Es wurden 3,81 g (15,5 mmol) 1-(4-Bromphenoxy)-1-ethoxyethan, gelöst in 5 ml Tetrahydrofuran, so zugetropft, daß das Reaktionsgemisch leicht siedete. Nach beendeter Zugabe wurde eine Stunde bei 55°C nachgerührt. 3,0 g (13,0 mmol) des nach Beispiel 1 hergestellten 2- 2-(Dimethylaminophenylmethyl)cyclohexanons wurden in 15 ml Tetrahydrofuran gelöst, bei Eisbadkühlung zum Grignard-Ansatz getropft und 15 Stunden bei RT gerührt. Zur Aufarbeitung wurden 30 ml gesättigte Ammoniumchloridlösung bei Eisbadkühlung zugegeben und bei RT dreimal mit je 80 ml Essigsaureethylester extrahiert. Die vereinigten organischen Extrakte wurden über Natriumsulfat getrocknet, filtriert und am Rotationsverdampfer (500 bis 10 mbar) eingeengt. Es wurden 5,25 g Rohbase erhalten, die in 20 ml Ether gelöst und mit 10 ml Salzsäure (1 N) versetzt wurde. Die wässrige Phase wurde abgetrennt und soviel Natriumhydrogencarbonat zugegeben, bis ein pH von ca. 8 erreicht war. Es wurde dreimal mit je 20 ml Ether extrahiert, die vereinigten organischen Extrakte über Natriumsulfat getrocknet, filtriert und am Rotationsverdampfer (500 bis 10 mbar) eingeengt. Es wurden 1,96 g Rohbase (46,3% der Theorie) erhalten, aus der mit 0,49 ml Salzsaure (32 m%) und 20 ml Aceton 1,8 g des 4-[2-(Dimethylaminophenyl-methyl-1-hydroxycyclohexyl]phenol, Hydrochlorid erhalten wurden (38,6% der Theorie), das sich beim Erhitzen ab 140°C zersetzt.

**Beispiel 21**

2-(Dimethylaminophenylmethyl)-1-naphthalin-2-ylcyclohexanol, Hydrochlorid

Zu 1,37 g (14,2 mmol) trockenem Magnesiumchlorid, gelöst in 35 ml Tetrahydrofuran, wurden unter Stickstoff 1,01 g (25,9 mmol) Kalium gegeben und unter Rühren auf 65°C erwarmt. Die Suspension wurde drei Stunden zum Rückfluß erhitzt, 2,99 g (14,0 mmol) 2-Bromnaphthalin, gelöst in 10 ml Tetrahydrofuran, zugetropft, 1,5 Stunden weitergerührt, auf RT abgekuhlt, anschließend 2,65 g (13,0 mmol) des nach Beispiel 1 hergestellten 2-(Dimethylaminophenylmethyl) cyclohexanons, gelöst in 10 ml Tetrahydrofuran, zugetropft und 15 Stunden bei RT gerührt. Zur Aufarbeitung wurden 20 ml gesättigte Ammoniumchloridlösung bei Eisbadkühlung zugegeben und bei RT dreimal mit je 80 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 4,2 g Rohbase (102% der Theorie) erhalten, aus der nach Beispiel 1 (3. Stufe) mit Chlortrimethylsilan/Wasser in 2-Butanon ein Hydrochlorid gefällt wurde. Hieraus wurde mit 20 ml Wasser und 3 ml Natronlauge (32 m%) die Base freigesetzt, dreimal mit je 30 ml Ether extrahiert, die vereinigten organischen Extrakte über Natriumsulfat getrocknet, filtriert und am Rotationsverdampfer (500 bis 10 mbar) eingeengt. Aus der Rohbase wurden nach Beispiel 1 (3. Stufe) mit Chlortrimethylsilan/Wasser in 2-Butanon 0,42 g 2-(Dimethylaminophenylmethyl)-1-naphthalin-2-ylcyclohexanol, Hydrochlorid (9,2% der Theorie) erhalten, das sich beim Erhitzen ab 240°C zersetzt.

**Beispiel 22**

2-[Dimethylamino-(4-trifluormethylphenyl)methyl]-1-(3-methoxybenzyl)cyclohexanol, Hydrochlorid

1. Stufe

N,N,N',N'-Tetramethyl-C-(4-trifluormethylphenyl)methandiamin

Zu 55 g (0,315 mol) 4-(Trifluormethyl)benzaldehyd wurden unter Rühren und Eisbadkühlung 81 ml (0,632 mol) Diniethylaminlosung (40 m% in Wasser) gegeben und 15 Stunden bei RT weitergerührt. Zur Aufarbeitung wurden 40 ml gesattigte Kaliumcarbonatlösung und festes Kaliumcarbonat zugegeben, bis ein pH von ca. 9 erreicht war. Es wurde dreimal mit je 300 ml Essigsäureethylester extrahiert. Die vereinigten organischen Extrakte wurden über Kaliumcarbonat getrocknet, filtriert und am Rotationsverdampfer (500 bis 10 mbar) eingeengt. Auf diese Weise wurden 68,3 g N,N,N',N'-Tetramethyl-C-(4-trifluormethylphenyl)methandiamin (87,8% der Theorie) erhalten.

2. Stufe

2-[Dimethylamino-(4-trifluormethylphenyl)methyl]cyclohexanon

63 g (256 mmol) N,N,N',N'-Tetramethyl-C-(4-trifluormethylphenyl)methandiamin aus Stufe 1 wurden in 450 ml Ether gelöst und im Eisbad auf 0°C gekühlt. Unter Stickstoff wurden 18,3 ml (256 mmol) Acetylchlorid zugetropft und 15 Stunden bei RT gerührt. Der Ansatz wurde mit einem Trockeneis/Isopropanol-Bad auf -70 °C gekühlt, 38,7 g (256 mmol) 1-(Pyrrolidino)-1-cyclohexen, gelöst in 300 ml Dichlormethan, zugetropft, die Mischung innerhalb von drei Stunden auf -30°C erwärmt und 15 Stunden bei dieser Temperatur gelagert. Zur Aufarbeitung wurden 200 ml halbkonzentrierte Salzsäure zugegeben und 5 Minuten nachgeruhrt-Die Phasen wurden getrennt und die wässrige Lösung bei RT mit 150 ml Ether extrahiert, mit 400 ml Ammoniaklösung (5 Vol.%) versetzt und schnell dreimal mit je 400 ml Ether extrahiert. Die vereinigten organischen Extrakte wurden über Natriumsulfat getrocknet, filtriert und am Rotationsverdampfer ohne Wärmezufuhr (500 bis 10 mbar) eingeengt. Auf diese Weise wurden 63,1 g Rohbase (82,4% der Theorie) erhalten. Aus der Rohbase wurden nach Beispiel 1 (2. Stufe) mit Chlortrimethylsilan/Wasser in 2-Butanon 51,4 g des Hydrochlorid von 2-[Dimethylamino-(4-trifluormethylphenyl)methyl]cyclohexanon (59,8% der Theorie) mit einem Schmelzpunkt von 139 - 140°C erhalten.

3. Stufe

2-[Dimethylamino-(4-trifluormethylphenyl)methyl]-1-(3-methoxybenzyl)cyclohexanol, Hydrochlorid

0,29 g (12,0 mmol) Magnesiumispäne wurden in 5 ml Ether p.a. gerührt. Es wurden 2,42 g (12,0 mmol) 3-Methoxybenzylbromid, gelost in 10 ml Ether, so zugetropft, daß das Reaktionsgemisch leicht siedete. Nach beendeter Zugabe wurde eine Stunde bei RT nachgerührt. 3,0 g (10,0 mmol) des nach Stufe 2 hergestellten 2-[Dimethylamino-(4-trifluormethylphenyl)methyl]cyclohexanons wurden in 5 ml. Ether gelöst, bei Eishadkühlung zum Grignard-Ansatz getropft und 15 Stunden bei RT gerührt. Zur Aufarbeitung wurden 30 ml gesattigte Ammoniumchloridlösung bei Eisbadkühlung zugegeben und bei RT dreimal mit je 50 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 5,60 g Rohbase (133% der Theorie) erhalten. Aus der Rohbase wurden nach Beispiel 1 (3. Stufe) mit Chlortrimethylsilan/Wasser in 2-Butanon 3,26 g 2-[Dimethylamino-(4-trifluormethylphenyl)methyl]-1-(3-methoxybenzyl)-cyclohexanol, Hydrochlorid (70,9% der Theorie) erhallen, das sich beim Erhitzen ab 133°C zersetzt.

**Beispiel 23**

1-(4-Chlorbenzyl)-2-(dimethylaminophenylmethyl)-1-cyclohexanol, Hydrochlorid

0,38 g (15,6 mmol) Magnesiumspäne wurden in 15 ml Ether p.a. gerührt. Es wurden 1,99 g (15,6 mmol) 4-Chlorbenzylbromid, gelöst in 15 ml Ether, so zugetropft, daß das Reaktionsgemisch leicht siedete. Nach beendeter Zugabe wurde eine Stunde bei RT nachgeruhrt. 3,0 g (13,0 mmol) des nach Beispiel 1 hergestellten 2-(Dimethylaminophenylmethyl)cyclohexanons wurden in 15 ml Ether gelost, bei Eisbadkühlung zum Grignard-Ansatz getropft und 15 Stunden bei RT gerührt. Zur Aufarbeitung wurden 30 ml gesättigte Ammoniumchloridlösung bei Eisbadkühlung zugegeben und bei RT dreimal mit je 60 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden

4,48 g Rohbase (96,5% der Theorie) erhalten. Aus der Rohbase wurden nach Beispiel 1 (3. Stufe) mit Chlortrimethylsilan/Wasser in 2-Butanon 1,74 4 g 1-(4-Chlorbenzyl)-2-(dimethylaminophenylmethyl)-1-cyclohexanol, Hydrochlorid (34,0% der Theorie) erhalten, das sich beim Erhitzen ab 208°C zersetzt.

**Beispiel 24**

2-(Dimethylaminophenylmethyl)-1-(2-fluorbenzyl)cyclohexanol, Hydrochlorid

0,38 g (15,6 mmol) Magnesiumspäne wurden in 15 ml Ether p. a . gerührt. Es wurden 1,85 g (15,6 mmol) 2-Fluorbenzylbromid, gelöst in 15 ml Ether, so zugetropft, daß das Reaktionsgemisch leicht siedete. Nach beendeter Zugabe wurde eine Stunde bei RT nachgerührt. 3,0 g (13,0 mmol) des nach Beispiel 1 hergestellten 2-(Dimethylaminophenylmethyl)cyclohexanons wurden in 15 ml Ether gelöst, bei Eisbadkühlung zum Grignard-Ansatz getropft und 15 Stunden bei RT gerührt. Zur Aufarbeitung wurden 30 ml gesättigte Ammoniumchloridlösung bei Eisbadkühlung zugegeben und bei RT dreimal mit je 60 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 3,50 g Rohbase (79,0% der Theorie) erhalten. Aus der Rohbase wurden nach Beispiel 1 (3. Stufe) mit Chlortrimethylsilan/Wasser in 2-Butanon 1,75 g 2-(Dimethylaminophenylmethyl)-1-(2-fluorbenzyl)cyclohexanol, Hydrochlorid (35,7% der Theorie) erhalten, das sich beim Erhitzen ab 175°C zersetzt.

**Beispiel 25**

2-(Dimethylaminophenylmethyl)-1-(4-fluorbenzyl)cyclohexanol, Hydrochlorid

0,38 g (15,6 mmol) Magnesiumspane wurden in 15 ml Ether p.a. gerührt. Es wurden 1,87 g (15,6 mmol) 4-Fluorbenzylbromid, gelöst in 15 ml Ether, so zugetropft, daß das Reaktionsgemisch leicht siedete. Nach beendeter Zugabe wurde eine Stunde bei RT nachgeruhrt. 3,0 g (13,0 mmol) des nach Beispiel 1 hergestellten 2-(Dimethylaminophenylmethyl)cyclohexanons wurden in 15 ml Ether gelöst, bei Eisbadkühlung zum Grignard-Ansatz getropft und 15 Stunden bei RT gerührt. Zur Aufarbeitung wurden 30 ml gesättigte Ammoniumchloridlösung bei Eisbadkuhlung zugegeben und bei RT dreimal mit je 60 ml Essigsaureethylester extrahiert. Die vereinigten organischen Extrakte wurden über Natriumsulfat getrocknet, filtriert und am Rotationsverdampfer (500 bis 10 mbar) eingeengt. Es wurden 4,51 g Rohbase (102% der Theorie) erhalten. Aus der Rohbase wurden nach Beispiel 1 (3. Stufe) mit Chlortrimethylsilan/Wasser in 2-Butanon 2,59 g 2-(Dimethylaminophenylmethyl)-1-(4-fluorbenzyl)cyclohexanol, Hydrochlorid (52,8% der Theorie) erhalten, das sich beim Erhitzen ab 203°C zersetzt.

**Beispiel 26**

1-(2,5-Dimethoxyphenyl)-2-(dimethylaminophenylmethyl)-cyclohexanol, Hydrochlorid

0,38 g (15,6 mmol) Magnesiumspäne wurden in 15 ml Tetrahydrofuran p.a. gerührt. Es wurden 3,39 g (15,6 mmol) 1-Brom-2,5-dimethoxybenzol, gelöst in 15 ml Tetrahydrofuran, so zugetropft, daß das Reaktionsgemisch leicht siedete. Nach beendeter Zugabe wurde 1,5 Stunden bei 65°C nachgerührt. 3,0 g (13,0 mmol) des nach Beispiel 1 hergestellten 2-(Dimethylaminophenylmethyl)cyclohexanons wurden in 15 ml Tetrahydrofuran gelöst, bei Eisbadkühlung zum Grignard-Ansatz getropft und 15 Stunden bei RT gerührt. Zur Aufarbeitung wurden 30 ml gesattigte Ammoniumchloridlösung bei Eisbadkühlung zugegeben und bei RT dreimal mit je 60 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 5,17 g Rohbase (108% der Theorie) erhalten. Aus der Rohbase wurden nach Beispiel 1 (3. Stufe) mit Chlortrimethylsilan/Wasser in 2-Butanon 4,43 g 1-(2,5-Dimethoxyphenyl)-2-(dimethylaminophenylmethyl)cyclohexanol, Hydrochlorid (84,2% der Theorie) mit einem Schmelzpunkt über 240°C erhalten.

**Beispiel 27**

1-(2-Chlor-4-fluorbenzyl)-2-(dimethylaminophenylmethyl)-cyclohexanol, Hydrochlorid

0,38 g (15,6 mmol) Magnesiumspäne wurden in 10 ml Ether p . a . gerührt. Es wurden 2,79 g (15,6 mmol) 2-Chlor-4-fluorbenzylbromid, gelost in 10 ml Ether, so zugetropft, daß das Reaktionsgemisch leicht siedete. Nach beendeter Zugabe wurde eine Stunde bei RT nachgerührt. 3,0 g (13,0 mmol) des nach Beispiel 1 hergestellten 2-(Dimethylaminophenylmethyl)cyclohexanons wurden in 15 ml Ether gelost, bei Eisbadkuhlung zum Grignard-Ansatz getropft und 15 Stunden bei RT gerührt. Zur Aufarbeitung wurden 30 ml gesättigte Ammoniumchloridlosung bei Eisbadkühlung zugegeben und bei RT dreimal mit je 60 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 4,52 g Rohbase (92,8% der Theorie) erhalten. Die Rohbase wurde in 45 ml 2-Butanon und etwas Essigsäureethylester gelöst, nacheinander 0,11 ml (6,0 mmol) Wasser und 1,52 ml (12,0 mmol) Chlortrimethylsilan zugegeben und der Ansatz 15 Stunden bei RT aufbewahrt. Die Lösemittel wurden am Rotationsverdampfer (500 bis 10 mbar) abdestil-liert, der Rückstand in 20 ml Ether aufgenommen, der zuruckbleibende Feststoff abfiltriert, mit kleinen Portionen Ether gewaschen und im Ölpumpenvakuum bis zur Gewichtskonstanz getrocknet. Auf diese Weise wurden 4,45 g 1-(2-Chlor-4-fluorbenzyl)-2-(dimethylaminophenylmethyl)cyclohexanol, Hydrochlorid (83,2% der Theorie) erhalten, das sich beim Erhitzen ab 100°C zersetzt.

**Beispiel 28**

1-(4-tert.-Butylbenzyl)-2-(dimethylaminophenylmethyl)-cyclohexanol, Hydrochlorid

0,33 g (13,5 mmol) Magnesiumspäne wurden in 10 ml Ether p.a. gerührt. Es wurden 2,46 g (13,5 mmol) 4-tert. -Butylbenzylchlorid, gelöst in 10 ml Ether, so zugetropft, daß das Reaktionsgemisch leicht siedete. Nach beendeter Zugabe wurde eine Stunde bei RT nachgerührt. 3,0 g (13,0 mmol) des nach Beispiel 1 hergestellten 2-(Dimethylaminophenylmethyl)cyclohexanons wurden in 10 ml Ether gelöst, bei Eisbadkühlung zum Grignard-Ansatz getropft und 15 Stunden bei RT gerührt. Zur Aufarbeitung wurden 30 ml gesättigte Ammoniumchloridlösung bei Eisbadkuhlung zugegeben und bei RT dreimal mit je 60 ml Essigsäureethylester extrahiert. Die vereinigten organi-schen Extrakte wurden uber Natriumsulfat getrocknet, filtriert und am Rotationsverdampfer (500 bis 10 mbar) eingeengt. Es wurden 4,18 g Rohbase (98,1% der Theorie) erhalten. Aus der Rohbase wurden nach Beispiel 1 (3. Stufe) mit Chlortrimethylsilan/Wasser in 2-Butanon 2,16 g 1-(4-tert.-Butylbenzyl)-2-(dimethylaminophenylmethyl)cyclohexanol, Hydrochlorid (46,2% der Theorie) mit einem Schmelzpunkt von 227 - 229°C erhalten.

**Beispiel 29**

2-(Dimethylaminophenylmethyl)-1-(3-fluorbenzyl ) cyclohexanol, Hydrochlorid

0,38 g (15,6 mmol) Magnesiumspane wurden in 10 ml Ether p.a. gerührt. Es wurden 1,89 g (15,6 mmol) 3-Fluorbenzylbromid, gelöst in 10 ml Ether, so zugetropft, daß das Reaktionsgemisch leicht siedete. Nach beendeter Zugabe wurde eine Stunde bei RT nachgeruhrt. 3,0 g (13,0 mmol) des nach Beispiel 1 hergestellten 2-(Dimethylaminophenylmethyl)cyclohexanons wurden in 15 ml Ether gelöst, bei Eisbadkuhlung zum Grignard-Ansatz getropft und 15 Stunden bei RT gerührt. Zur Aufarbeitung wurden 30 ml gesättigte Ammoniumchloridlösung bei Eisbadkühlung zugegeben und bei RT dreimal mit je 60 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 4,59 g Rohbase (104 % der Theorie) erhalten, aus der nach Beispiel 1 (3. Stufe) mit Chlortrimethylsilan /Wasser in 2-Butanon ein Hydrochlorid gefällt wurde. Hieraus wurde mit 40 ml Wasser und 5 ml Natronlauge (32 m%) die Base freigesetzt, dreimal mit je 40 ml Ether extrahiert, die vereinigten organischen Extrakte über Natriumsulfat getrocknet, filtriert und am Rotationsverdampfer (500 bis 10 mbar) eingeengt. Es wurden 3,42 g Rohbase (77,2% der Theorie) erhalten. Aus der Rohbase wurden nach Beispiel 1 (3. Stufe) mit Chlortrimethylsilan/Wasser in 2-Butanon 2,72 g 2-(Dimethylaminophenylmethyl)-1-(3-fluorbenzyl)cyclohexanol, Hydrochlorid (55,5% der Theorie) mit einem Schmelzpunkt von 146 - 147°C erhalten.

**Beispiel 30**

1-(2-Chlorbenzyl)-2-(dimethylaminophenylmethyl)cyclohexanol, Hydrochlorid

0,38 g (15,6 mmol) Magnesiumspäne wurden in 15 ml Ether p.a. gerührt. Es wurden 2,0 ml (15,6 mmol) 2-Chlorbenzylchlorid, gelöst in 15 ml Ether, so zugetropft, daß das Reaktionsgemisch leicht siedete. Nach beendeter Zugabe wurde eine Stunde bei RT nachgerührt. 3,0 g (13,0 mmol) des nach Beispiel 1 hergestellten 2-(Dimethylaminophenylmethyl)cyclohexanons wurden in 15 ml Ether gelöst, bei Eisbadkühlung zum Grignard-Ansatz getropft und 15 Stunden bei RT gerührt. Zur Aufarbeitung wurden 30 ml gesättigte Ammoniumchloridlösung bei. Eisbadkühlung zugegeben und bei RT dreimal mit je 60 ml Essigsäureethylester extrahiert. Die vereinigten organischen Extrakte wurden über Natriumsulfat getrocknet, filtriert und am Rotations-verdampfer (500 bis 10 mbar) eingeengt. Es wurden 4,50 g Rohbase (97,0% der Theorie) erhalten, aus der nach Beispiel 1 (3. Stufe) mit Chlortrimethylsilan/Wasser in 2-Butanon ein Hydrochlorid gefällt wurde. Hieraus wurde mit 40 ml Wasser und 5 ml Natronlauge (32 m%) die Base freigesetzt, dreimal mit je 40 ml Ether extrahiert, die vereinigten organischen Extrakte über Natriumsulfat getrocknet,

filtriert und am Rotationsverdampfer (500 bis 10 mbar) eingeengt. Man erhielt 2,90 g Rohbase, die auf eine 3,5 x 15 cm Säule, gefüllt mit Kieselgel, gegeben wurden. Die Elution mit Essigsäureethylester/n-Hexan 2:5 ergab 1,59 g Base, aus der nach Beispiel 1 (3. Stufe) mit Chlortrimethylsilan/Wasser in 2-Butanon 1,75 g 1-(2-Chlorbenzyl)-2-(dimethylaminophenyl-methyl)cyclohexanol, Hydrochlorid (34,2% der Theorie) erhalten, das sich beim Erhitzen ah 130°C zersetzt.

**Beispiel 31**

1-Benzo[1,3]dioxol-5-yl-2-[dimethylamino-(3-methoxyphenyl)methyl]cyclohexanol, Hydrochlorid

2, 61 g (13,0 mmol) 4-Brom-1,2-methylendioxybenzol wurden in 10 ml Tetrahydrofuran gelöst und unter Stickstoff mit einem Trockeneis/Isopropanol -Bad auf -70 °C gekuhlt. Unter Rühren wurden 7,9 ml (13,0 mmol) n-Butyllithium (1,6 M in Hexan) so zugetropft, daß die Temperatur nicht über -60°C stieg. Es wurde 30 Minuten nachgerührt und dann 3,0 g (10,8 mmol) des nach Beispiel 9 hergestellten 2-(Dimethylamino-(3-methoxyphenyl)methyl]cyclohexanons, gelöst in 10 ml Tetrahydrofuran, bei Eisbadkühlung zugetopft und innerhalb zwei Stunden auf RT erwärmt. Zur Aufarbeitung wurden 20 ml gesättigte Ammoniumchloridlösung bei Eisbadkühlung zugegeben und bei RT dreimal mit je 20 ml Essigsaureethylester extrahiert. Die vereinigten organischen Extrakte wurden über Natriumsulfat getrocknet, filtriert und am Rotationsverdampfer (500 bis 10 mbar) eingeengt. Es wurden 4,10 g Rohbase (98,8% der Theorie) erhalten, aus der nach Beispiel 1 (3. Stufe) mit Chlortrimethylsilan/Wasser in 2-Butanon 1,96 g 1-Benzo[1,3]dioxol-5-yl-2-[dimethylamino-(3-methoxyphenyl)methyl]cyclohexanol, Hydrochlorid (43,2% der Theorie) erhalten, das sich beim Erhitzen ab 109°C zersetzt.

**Beispiel 32**

1-(3-Chlorbenzyl)-2-(dimethylaminophenylmethyl)cyclohexanol, Hydrochlorid

0,38 g (15,6 mmol) Magnesiumspane wurden in 15 ml Ether p.a. gerührt. Es wurden 2,0 ml (15,6 mmol) 3-Chlorbenzylchlorid, gelöst in 15 ml Ether, so zugetropft, daß das Reaktionsgemisch leicht siedete. Nach beendeter Zugabe wurde eine Stunde bei RT nachgeruhrt. 3,0 g (13,0 mmol) des nach Beispiel 1 hergestellten 2-(Dimethylaminophenylmethyl) cyclohexanons wurden in 15 ml Ether gelöst;, bei Eisbadkühlung zum Grignard-Ansatz getropft und 15 Stunden bei RT gertihrt. Zur Aufarbeitung wurden 30 ml gesattigte Aniinoniumchloridlösung bei Eisbadkühlung zugegeben und bei RT dreimal mit je 60 ml Essigsäureethylester extrahiert. Die vereinigten organischen Extrakte wurden über Natriumsulfat getrocknet, filtriert und am Rotationsverdampfer (500 bis 10 mbar) eingeengt. Es wurden 4,55 g Rohbase (98,0% der Theorie) erhalten, aus der nach Beispiel 1 (3. Stufe) mit Chlortrimethylsilan/Wasser in 2-Butanon ein Hydrochlorid gefällt wurde. Hieraus wurde mit 40 ml Wasser und 5 ml Natronlauge (32 m%) die Base freigesetzt, dreimal mit je 40 ml Ether extrahiert, die vereinigten organischen Extrakte über Natriumsulfat getrocknet, filtriert und am Rotationsverdampfer (500 bis 10 mbar) eingeengt. Man erhielt 2,87 g Rohbase, die mit 5 ml Essigsäureethylester/n-Hexan 2:5 versetzt wurden. Der unlösliche Ruckstand wurde abfiltriert und getrocknet. Man erhielt 2,11 g Base, aus der nach Beispiel 1 (3. Stufe) mit Chlortrimethylsilan/Wasser in 2-Butanon 1,68 g 1-(3-Chlorbenzyl)-2-(dimethylaminophenylmethyl)cyclohexanol, Hydrochlorid (32,8 % der Theorie) mit einem Schmelzpunkt von 185 - 188°C gefällt wurde.

**Beispiel 33**

1-(2,4-Dichlorbenzyl)-2-(dimethylaminophenylmethyl)cyclohexanol, Hydrochlorid

0,38 g (15,6 mmol) Magnesiumspäne wurden in 10 ml Ether p.a. gerührt. Es wurden 3,04 g (15,6 mmol) 2,4-Dichlorbenzylchlorid, gelöst in 10 ml Ether, so zugetropft, daß das Reaktionsgemisch leicht siedete. Nach beendeter Zugabe wurde eine Stunde bei RT nachgerührt. 3,0 g (13,0 mmol) des nach Beispiel 1 hergestellten 2-(Dimethylaminophenylmethyl)cyclohexanons wurden in 15 ml Ether gelost, bei Eisbadkuhlung zum Grignard-Ansatz getropft und 15 Stunden bei RT gerührt. Zur Aufarbeitung wurden 30 ml gesattigte Ammoniumchloridlösung bei Eisbadkühlung zugegeben und bei RT dreimal mit je 60 ml Essigsäureethylester extrahiert. Die vereinigten organischen Extrakte wurden über Natriuinsulfat getrocknet, filtriert und am Rotationsverdampfer (500 bis 10 mbar) eingeengt. Es wurden 2,14 g Rohbase (97,0% der Theorie) erhalten, aus der nach Beispiel 1 (3. Stufe) mit Chlortrimethylsilan//Wasser in 2-Butanon ein Hydrochlorid gefällt wurde. Hieraus wurde mit 20 ml Wasser und 3 ml Natronlauge (32 m%) die Base freigesetzt, dreimal mit je 20 ml Ether extrahiert, die vereinigten organischen Extrakte über Natriumsulfat getrocknet, filtriert und am Rotationsverdampfer (500 bis 10 mbar) eingeengt. Es wurden 1,19 g Rohbase erhalten, aus der man nach Beispiel 1 (3. Stufe) mit Chlortrimethylsilan/Wasser in 2-Butanon 0,45 g 1-(2,4-Dichlorbenzyl)-2-(dimethylaminophenylmethyl)cyclohexanol, Hydrochlorid (8,1% der Theorie) erhielt, das sich beim Erhitzen ab 140°C zersetzt.

**Beispiel 34**

1-Benzyl-2-[dimethylaminophenyl-(3-phenoxyphenyl)methyl]-cyclohexanol, Hydrochlorid

1. Stufe

2-[Dimethylamino-(3-phenoxyphenyl)methyl]cyclohexanon

Zu 67 ml (66,6 mmol) Natriumiodidlösung (1 M in Acetonitril), mit einem Eisbad auf 0°C gekühlt, wurden unter Rühren 2,47 g (30,3 mmol) frisch getrocknetes Dimethylamin-Hydrochlorid gegeben, 8,4 ml (60,5 mmol) Triethylamin und 8,4 ml (66,6 mmol) Chlortrimethylsilan zugetropft und eine Stunde bei RT nachgerührt. Bei Eiskühlung wurden 6,0 ml (30,3 mmol) 3-Phenoxybenzaldehyd zugegeben und noch eine Stunde bei RT weitergerührt. Es wurde erneut mit einem Eisbad auf 0°C gekühlt, 4,58 g (30,3 mmol) 1-(Pyrrolidino)-1-cyclohexen zugegeben und zwei Stunden bei RT weitergerührt. Zur Aufarbeitung wurde der Ansatz bei Eiskühlung mit 45 ml halkonzentrierter Salzsäure versetzt, 10 Minuten gerührt, zweimal mit je 45 ml Ether gewaschen und mit 115 ml verdünnter Ammoniaklösung (5 Vol. %) alkalisch gestellt (pH ca. 9). Es wurde dreimal mit je 45 ml Ether extrahiert, die vereinigten organischen Extrakte über Natriumsulfat getrocknet, filtriert und am Rotationsverdampfer (500 bis 10 mbar) ohne Wärmezufuhr eingeengt. Auf diese Weise wurden 7,41 g Rohbase (75,7 % der Theorie) erhalten. Aus der Rohbase wurden nach Beispiel 1 (2. Stufe) mit Chlortrimethylsilan/Wasser in 2-Butanon 4,83 g des Hydrochlorids von 2-[Dimethylamino-(3-phenoxyphenyl)-methyl]cyclohexanon (44,4% der Theorie) erhalten.

2. Stufe

1-Benzyl-2-[dimethylaminophenyl-(3-phenoxyphenyl)methyl]-cyclohexanol, Hydrochlorid

0,27 g (11,1 mmol) Magnesiumspäne wurden in 5 ml Ether p.a. gerührt. Es wurden 1,90 g (11,1 mmol) Benzylbromid, gelöst in 10 ml Ether, so zugetropft, daß das Reaktionsgemisch leicht siedete. Nach beendeter Zugabe wurde eine Stunde bei RT nachgeruhrt. Aus 3,7 g (11,4 mmol) des nach Stufe 1 erhaltenen Hydrochlorids von 2-[Dimethylamino-(3-phenoxyphenyl)methyl]cyclohexanon wurde mit 30 ml Wasser und 5 ml Ammoniaklösung (25 Vol.%) die Base freigesetzt, dreimal mit je 30 ml Ether extrahiert, die vereinigten organischen Extrakte über Natriumsulfat getrocknet, filtriert und am Rotationsverdampfer ohne Wärmezufuhr (500 bis 10 mbar) eingeengt. 3,0 g (9,3 mmol) dieser Base wurden in 10 ml Ether gelöst, bei Eisbadkühlung zum Grignard-Ansatz getropft und 15 Stunden bei RT gerührt. Zur Aufarbeitung wurden 15 ml gesättigte Ammoniumchloridlösung bei Eisbadkühlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 3,51 g Rohbase (91,1% der Theorie) erhalten, aus der nach Beispiel 1 (3. Stufe) mit Chlortrimethylsilan/Wasser in 2-Butanon ein Hydrochlorid gefällt wurde. Hieraus wurde mit 30 ml Wasser und 5 ml Ammoniaklosung (25 Vol. %) die Base freigesetzt, dreimal mit je 15 ml Etlier extrahiert, die vereinigten organischen Extrakte uber Natriumsulfat getrocknet, filtriert und am Rotationsverdampfer (500 bis 10 mbar) eingeengt. Es wurden 2,55 g Rohbase (65,9% der Theorie) erhalten, die auf eine 5 x 33 cm Säule, gefüllt mit Kieselgel, gegeben wurde. Die Elution mit Essigsäureethylester /n-Hexan 1:4 ergab 1,92 g Base, aus der nach Beispiel 1 (3. Stufe) mit Chlortrimethylsilan/Wasser in 2-Butanon 0,51 g 1-Benzyl-2-[dimethylaminophenyl-(3-phenoxyphenyl)-methyl] cyclohexanol, Hydrochlorid (12,1% der Theorie) mit einem Schmelzpunkt von 189 - 190°C erhalten wurden.

**Beispiel 35**

1-Benzyl-2-[dimethylaminophenyl]-(3-methoxyphenyl)methyl]-cyclohexanol, Hydrochlorid

0,32 g (13,0 mmol) Magnesiumspane wurden in 10 ml Ether p.a. gerührt. Es wurden 2,22 g (13,0 mmol) Benzylbromid, gelöst in 10 ml Ether, so zugetropft, daß das Reaktionsgemisch leicht siedete. Nach beendeter Zugabe wurde eine Stunde bei RT nachgerührt. 3,0 g (10,8 mmol) des nach Beispiel 9 hergestellten 2-[Dimethylamino-(3-methoxyphenyl)-methyl]cyclohexanons wurden in 10 ml Ether gelöst, bei Eisbadkühlung zum Grignard-Ansatz getropft und 15 Stunden bei RT gerührt. Zur Aufarbeitung wurden 15 ml gesättigte Ammoniumchloridlösung bei Eisbadkuhlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 3,58 g Rohbase (93,7% der Theorie) erhalten, aus der nach Beispiel 1 (3. Stufe) mit Chlortrimethylsilan/Wasser in 2-Butanon ein Hydrochlorid gefällt wurde. Hieraus wurde mit 30 ml Wasser und 5 ml Ammoniaklösung (25 Vol.%) die Base freigesetzt, dreimal mit je 15 ml Ether extrahiert, die vereinigten organischen Extrakte über Natriumsulfat getrocknet, filtriert und am Rotationsverdampfer (500 bis 10 mbar) eingeengt. Es wurden 3,2 g Rohbase (76,2% der Theorie) erhalten, die auf eine 5 x 33 cm Säule, gefüllt mit Kieselgel, gegeben wurde. Die Elution mit Essigsäureethylester/n-Hexan 1:4 ergab 1,69 g Base, aus der nach Beispiel 1 (3. Stufe) mit Chlortrimethylsilan/Wasser in 2-Butanon 1,60 g 1-Benzyl-2-[dimethylaminophenyl-(3-methoxyphenyl)methyl]cyclohexanol, Hydrochlorid (38,0% der Theorie) mit einem Schmelzbereich von 101 - 115°C erhalten wurden.

**Beispiel 36**

2-(Dimethylaminophenylmethyl)-1-(3-trifluormethylbenzyl)-cyclohexanol, Hydrochlorid

0,33 g (13,5 mmol) Magnesiumspane wurden in 10 ml Ether p.a. gerührt. Es wurden 2,62 g (13,5 mmol) 3-Chlormetlylbenzotrifluorid, gelöst in 10 ml Ether, so zugetropft, daß das Reaktionsgemisch leicht siedete. Nach beendeter Zugabe wurde eine Stunde bei RT nachgerührt. 2, 60 g (11,2 mmol) des nach Beispiel 1 hergestellten 2-(Dimethylaminophenylmethyl)cyclohexanons wurden in 15 ml Ether gelost, bei Eisbadkühlung zum Grignard-Ansatz getropft und 15 Stunden bei RT gerührt. Zur Aufarbeitung wurden 30 ml gesättigte Ammoniumchloridlosung bei Eisbadkühlung zugegeben und bei RT dreimal mit je 60 ml Essigsaureethylester extrahiert. Die vereinigten organischen Extrakte wurden uber Natriumsulfat getrocknet, filtriert und am Rotationsverdampfer (500 bis 10 mbar) eingeengt. Es wurden 4,49 g Rohbase (104% der Theorie) erhalten, aus der nach Beispiel 1 (3. Stufe) mit Chlortrimethylsilan/Wasser in 2-Butanon ein Hydrochlorid gefällt wurde. Hieraus wurde mit 30 ml Wasser und 5 ml Ammoniaklösung (25 Vol.%) die Base freigesetzt, dreimal mit je 30 ml Ether extrahiert, die vereinigten organischen Extrakte über Natriumsulfat getrocknet, filtriert und am Rotationsverdampfer (500 bis 10 mbar) eingeengt. Es wurden 3,49 g Rohbase erhalten, aus der man nach Beispiel 1 (3. Stufe) mit Chlortrimethylsilan/Wasser in 2-Butanon 1,80 g 2-(Dimethylaminophenylmethyl)-1-(3-trifluormethylbenzyl)cyclohexanol, Hydrochlorid (37,4% der Theorie) mit einem Schmelzpunkt von 184 - 186°C erhielt.

**Beispiel 37**

2-[Dimethylamino-(3-methoxyphenyl)methyl]-1-(3-methoxybenzyl) cyclohexanol, Hydrochlorid

0,32 g (13,0 mmol) Magnesiumspäne wurden in 10 ml Ether p.a. gerührt. Es wurden 2,61 g (13,0 mmol) 3-Methoxybenzylbromid, gelöst in 10 ml Ether, so zugetropft, daß das Reaktionsgemisch leicht siedete. Nach beendeter Zugabe wurde eine Stunde bei RT nachgerührt. 3,0 g (10,8 mmol) des nach Beispiel 9 hergestellten 2-[Dimethylamino-(3-methoxyphenyl)methyl]cyclohexanons wurden in 10 ml Ether gelöst, bei Eisbadkühlung zum Grignard-Ansatz getropft und 15 Stunden bei RT gerührt. Zur Aufarbeitung wurden 15 ml gesättigte Ammoniumchloridlösung bei Eisbadkühlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 3,87 g Rohbase (101% der Theorie) erhalten, aus der nach Beispiel 1 (3. Stufe) mit Chlortrimethylsilan/Wasser in 2-Butanon ein Hydrochlorid gefällt wurde. Hieraus wurde mit 30 ml Wasser und 5 ml Ammoniaklösung (25 Vol.%) die Base freigesetzt, dreimal mit je 15 ml Ether extrahiert, die vereinigten organischen Extrakte über Natriumsulfat getrocknet, filtriert und am Rotationsverdampfer (500 bis 10 mbar) eingeengt. Es wurden 2,34 g Rohbase (61,2 % der Theorie) erhalten, aus der nach Beispiel 1 (3. Stufe) mit Chlortrimethylsilan/Wasser in 2-Butanon 2,04 g 2-[Dimethylamino-(3-methoxyphenyl)methyl]-1-(3-methoxybenzyl)-cyclohexanol, Hydrochlorid (48,4% der Theorie) erhalten wurden, das sich beim Erhitzen ab 75°C zersetzt.

**Beispiel 38**

2-[(2-Chlorphenyl)dimethylaminomethyl]-1-naphthalin-2-yl-cyclohexanol, Hydrochlorid

1. Stufe

2-[(2-Chlorphenyl)dimethylaminomethyl]cyclohexanon

Zu 471 ml (469 mmol) Natriumiodidlösung (1 M in Acetonitril) , mit einem Eisbad auf 0°C gekühlt, wurden unter Rühren 17,4 g (213 mmol) frisch getrocknetes Dimethylamin-Hydrochlorid gegeben, 60 ml (427 mmol) Triethylamin und 60 ml (469 mmol) Chlortrimethylsilan zugetropft und eine Stunde bei RT nachgerührt. Bei Eiskühlung wurden 24 ml (213 mmol) 2-Chlorbenzaldehyd zugegeben und noch eine Stunde bei RT weitergeruhrt. Es wurde erneut mit

einem Eisbad auf 0°C gekühlt, 34 ml (213 mmol) 1-(Pyrrolidino)-1-cyclohexen zugegeben und zwei Stunden bei RT weitergeruhrt. Zur Aufarbeitung wurde der Ansatz bei Eiskühlung mit 300 ml halkonzentrierter Salzsaure versetzt, 10 Minuten gerührt, zweimal mit je 300 ml Ether gewaschen und mit 770 ml verdünnter Ammoniaklösung (5 Vol.%) alkalisch gestellt (pH ca. 9) . Es wurde dreimal mit je 300 ml Ether extrahiert, die vereinigten organischen Extrakte über Natriumsulfat getrocknet, filtriert und am Rotationsverdampfer (500 bis 10 mbar) ohne Wärmezufuhr eingeengt. Auf diese Weise wurden 38,3 g Rohbase (67,5% der Theorie) erhalten. Aus der Rohbase wurden nach Beispiel 1 (2. Stufe) mit Chlortrimethylsilan/Wasser in 2-Butanon 33,6 g des Hydrochlorids von 2-[(2-Chlorphenyl)dimethylaminomethyl]-cyclohexanon (52,0% der Theorie) erhalten.

2. Stufe

2-[(2-Chlorphenyl)dimethylaminomethyl]-1-naphthalin-2-yl-cyclohexanol, Hydrochlorid.

0,27 g (11,1 mmol) Magnesiumspäne wurden in 5 ml Ether p a. gerührt. Es wurden 2,32 g ( 11,1 mmol) 2-Bromnaphthalin, gelöst in 10 ml Ether, so zugetropft, daß das Reaktionsgemisch leicht siedete. Nach beendeter Zugabe wurde eine Stunde bei RT nachgerührt. Aus 3,0 g (11, 2 mmol) des nach Stufe 1 erhaltenen Hydrochlorids von 2-[(2-Chlorphenyl)dimethylaminomethyl]cyclohexanon wurde mit 30 ml Wasser und 5 ml Ammoniaklösung (25 Vol.%) die Base freigesetzt, dreimal mit je 30 ml Ether extrahiert, die vereinigten organischen Extrakte über Natriumsulfat getrocknet, filtriert und am Rotationsverdampfer ohne Wärmezufuhr (500 bis 10 mbar) eingeengt. 2,50 g (9,3 mmol) dieser Base wurden in 10 ml Ether gelost, bei Eisbadkühlung zum Grignard-Ansatz getropft und 15 Stunden bei RT gerührt. Zur Aufarbeitung wurden 15 ml gesattigte Ammoniumchloridlösung bei Eisbadkühlung zugegeben und bei RT dreimal mit je 15 ml Ether extrahiert, Die vereinigten organischen Extrakte wurden über Natriuinsulfat getrocknet, filtriert und am Rotationsverdampfer (500 bis 10 mbar) eingeengt. Es wurden 4,05 g Rohbase (110 % der Theorie) erhalten, aus der nach Beispiel 1 (3. Stufe) mit Chlortrimethylsilan/Wasser in 2-Butanon ein Hydrochlorid gefallt wurde. Hieraus wurde mit 30 ml Wasser und 5 ml Ammoniak-lösung (25 Vol.%) die Base freigesetzt, dreimal mit je 15 ml Ether extrahiert, die vereinigten organischen Extrakte über Natriumsulfat getrocknet, filtriert und am Rotationsverdampfer (500 bis 10 mbar) eingeengt. Es wurden 2,2 g Rohbase (60,0 % der Theorie) erhalten, die auf eine 3 x 26 cm Säule, gefüllt mit Kieselgel, gegeben wurde. Die Elution mit Essigsäureethylester/n-Hexan 1:4 ergab 0,95 g Base, aus der nach Beispiel 1 (3. Stufe) mit Chlortrimethylsilan/Wasser in 2-Butanon 0, 47 g 2-[(2-Chlorphenyl)dimethylaminomethyl]-1-naphthalin-2-ylcyclohexanol, Hydrochlorid (11,3% der Theorie) mit einem Schmelzpunkt über 230°C erhalten wurden.

**Beispiel 39**

1-Benzyl-2-[(3,4-dichlorphenyl) dimethylaminomethyl] cyclohexanol, Hydrochlorid

1. Stufe

2-[(3,4-Dichlorphenyl)dimethylaminomethyl]cyclohexanon

Zu 214 ml (214 mmol) Natriumiodidlösung (1 M in Acetonitril), mit einem Eisbad auf 0°C gekühlt, wurden unter Ruhren 7,92 g (97,1 mmol) frisch getrocknetes Dimethylamin-Hydrochlorid gegeben, 27 ml (194 mmol) Triethylamin und 27 ml (214 mmol) Chlortrimethylsilan zugetropft und eine Stunde bei RT nachgerührt. Bei Eiskuhlung wurden 17,0 g (97,1 mmol) 3,4-Dichlorbenzaldehyd zugegeben und noch eine Stunde bei RT weitergerührt. Es wurde erneut mit einem Eisbad auf 0 °C gekühlt, 14,7 g (97,1 mmol) 1-(Pyrrolidino)-1-cyclohexen zugegeben und zwei Stunden bei RT weitergerührt. Zur Aufarbeitung wurde der Ansatz bei Eiskühlung mit 130 ml halkonzentrierter Salzsäure versetzt, 10 Minuten gerührt, zweimal mit je 125 ml Ether gewaschen und mit 300 ml verdünnter Ammoniaklösung (5 Vol.%) alkalisch gestellt (pH ca. 9). Es wurde dreimal mit je 125 ml Ether extrahiert, die vereinigten organischen Extrakte über Natriumsulfat getrocknet, filtriert und am Rotationsverdampfer (500 bis 10 mbar) ohne Wärmezufuhr eingeengt. Auf diese Weise wurden 26,6 g Rohbase (91, % der Theorie) erhalten. Aus der Rohbase wurden nach Beispiel 1 (2. Stufe) mit Chlortrimethylsilan/Wasser in 2-Butanon 26,7 g des Hydrochlorids von 2-[(3,4-Dichlorphenyl)dimethylaminomethyl] cyclohexanon (81,8% der Theorie) erhalten.

2. Stufe

1-Benzyl-2-[(3,4-dichlorphenyl)dimethylaminomethyl]cyclohexanol, Hydrochlorid

Aus 3,5 g (10,4 mmol) des nach Stufe 1 erhaltenen Hydrochlorids von 2- [(3,4-Dichlorphenyl)dimethylaminomethyl]-cyclohexanon wurde mit 30 ml Wasser und 10 ml Ammoniaklosung (25 Vol.%) die Base freigesetzt, dreimal mit je 30 ml Ether extrahiert, die vereinigten organischen Extrakte über Natriumsulfat getrocknet, filtriert und am Rotationsverdampfer ohne Wärmezufuhr (500 bis 10 mbar) eingeengt. 3,0 g (10,0 mmol) dieser Base wurden in 10 ml Tetrahydrofuran gelöst, bei Eisbadkuhlung zu 6,0 ml (12,0 mmol) Benzylmagnesiumchlorid (2 M Losung in Tetrahydrofuran) getropft und 15 Stunden bei RT gerührt. Zur Aufarbeitung wurden 15 ml gesättigte Ammoniumchloridlosung bei Eisbadkühlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 3,05 g Rohbase (77, 9% der Theorie) erhalten, aus der nach Beispiel 1 (3. Stufe) mit Chlortrimethylsilan/Wasser in 2-Butanon 2,88 g 1-Benzyl-2-[(3,4-dichlorphenyl)dimethylaminomethyl]cyclohexanol, Hydrochlorid (67, 1% der Theorie) erhalten wurden, das sich beim Erhitzen ab 200°C zersetzt.

**Beispiel 40**

2-[(3,4-Dichlorphenyl)dimethylaminomethyl]-1-phenethylcyclohexanol, hydrochlorid

3,0 g (10,0 mmol) des nach Beispiel 39 hergestellten 2-[(3,4-Dichlorphenyl)dimethylaminomethyl]cyclohexanons wurden in 10 ml Tetrahydrofuran gelost, bei Eisbadkühlung zu 12,0 ml (12,0 mmol ) Phenethylmagnesiumchlorid (1 M Lösung in Tetrahydrofuran) getropft und 15 Stunden bei RT gerührt. Zur Aufarbeitung wurden 15 ml gesättigte Ammoniumchloridlösung bei Eisbadkühlung zugegeben und bei RT dreimal mit je 15 ml Ether extrahiert. Die vereinigten organischen Extrakte wurden uber Natriumsulfat getrocknet, filtriert und am Rotationsverdampfer (500 bis 10 mbar) eingeengt. Man erhielt 3,88 g Rohbase (95,5% der Theorie) , aus der nach Beispiel 1 (3. Stufe) mit Chlortrimethylsilan/Wasser in 2-Butanon nach Zugabe von 30 ml n-Hexan ein Öl erhalten wurde. Nach dekantieren der Lösemittel wurde das Öl in 5 ml Wasser und 20 ml Ether gerührt, die entstandene Fällung abfiltriert und getrocknet. Auf diese Weise wurden 3,04 g 2-[(3,4-Dichlorphenyl)dimethylaminomethyl]-1-phenethylcyclohexanol, Hydrochlorid (68,7% der Theorie) erhalten, das sich beim Erhitzen ab 130°C zersetzt.

**Beispiel 41**

1-Benzyl-2-[dimethylamino-(4-fluorphenyl)methyl]cyclohexanol, Hydrochlorid

1. Stufe

2-[Dimethylamino-(4-fluorphenyl)methyl]cyclohexanon

Zu 532 ml (532 mmol) Natriunnodidlösung (1 M in Acetonitril), mit einem Eisbad auf 0°C gekuhll, wurden unter Rühren 19,7 g (242 mmol) frisch getrocknetes Dimethylamin-Hydrochlorid gegeben, 67 ml (483 mmol) Triethylamin und 67 ml (532 mmol) Chlortrimethylsilan zugetropft und eine Stunde bei RT nachgeruhrt. Bei Eiskühlung wurden 30,0 g (242 mmol) 4-Fluorbenzaldehyd zugegeben und noch eine Stunde bei RT weitergerührt. Es wurde erheut mit einem Eisbad auf 0°C gekuhlt, 36,6 g (242 mmol) 1-(Pyrrolidino)-1-cyclohexen zugegeben und zwei Stunden bei RT weitergerührt. Zur Aufarbeitung wurde der Ansatz bei Eiskühlung mit 300 ml halkonzentrierter Salzsäure versetzt, 10 Minuten gerührt, zweimal mit je 250 ml Ether gewaschen und mit 750 ml verdünnter Ammoniaklösung (5 Vol.%) alkalisch gestellt (pH ca. 9). Es wurde dreimal mit je 250 ml Ether extrahiert, die vereinigten organischen Extrakte über Natriumsulfat getrocknet, filtriert und am Rotationsverdampfer (500 bis 10 mbar) ohne Wärmezufuhr eingeengt. Auf diese Weise wurden 51,0 g Rolhbase (84,6% der Theorie) erhalten. Aus der Rohbase wurden nach Beispiel 1 (2. Stufe) mit Chlortrimethylsilan/Wasser in 2-Butanon 41,7 g des Hydrochlorids von 2-[Dimethylamino-(4-fluorphenyl)methyl]-cyclohexanon (60,3% der Theorie) erhalten.

2. Stufe

1-Benzyl-2-[dimethylamino-(4-fluorphenyl)methyl]cyclohexanol, Hydrochlorid

Aus 3,2 g (11,2 mmol) des nach Stufe 1 erhaltenen Hydrochlorids von 2-[Dimethylamino- (4-fluorphenyl)methyl]cyclohexanon wurde mit 30 ml Wasser und 10 ml Ammoniaklösung (25 Vol.%) die Base freigesetzt, dreimal mit je 30 ml Ether extrahiert, clie vereinigten organischen Extrakte über Natriumsulfat getrocknet, filtriert und am Rotationsverdampfer ohne Wärmezufuhr (500 bis 10 mbar) eingeengt. 2,69 g (10,8 mmol) dieser Base wurden in 10 ml Tetrahydrofuran gelöst, bei Eisbadkühlung zu 6,5 ml (12,9 mmol) Benzylmagnesiumchlorid (2 M Lösung in Tetrahydrofuran) getropft und 15 Stunden bei RT gerührt. Zur Aufarbeitung wurden 15 ml gesättigte Ammoniumchloridlösung bei Eisbadkühlung zugegeben und bei RT dreimal mit je 15 ml Ether extrahiert. Die vereinigten organischen Extrakte

wurden uber Natriumsulfat getrocknet, filtriert und am Rotationsverdampfer (500 bis 10 mbar) eingeengt. Es wurden 3,42 g Rohbase (84,1 % der Theorie) erhalten, aus der nach Beispiel 1 (3. Stufe) mit Chlortrimethylsilan/Wasser in 2-Butanon ein Hydrochlorid gefallt wurde. Hieraus wurde mit 30 ml Wasser und 5 ml Ammoniaklosung (25 Vol.%) die Base freigesetzt, dreimal mit je 15 ml Ether extrahiert, die vereinigten organischen Extrakte über Natriumsulfat getrocknet, filtriert und am Rotationsverdampfer (500 bis 10 mbar) eingeengt. Es wurden 2,89 g Rohbase (78,6% der Theorie) erhalten, aus der nach Beispiel 1 (3. Stufe) mit Chlortrimethylsilan/Wasser in 2-Butanon 2,58 g 1-Benzyl-2 [dimethylamino-(4-fluorphenyl)-methyl]cyclohexanol, Hydrochlorid (63,3 % der Theorie) mit einem Schmelzpunkt von 178°C erhalten wurden.

**Beispiel 42**

2-[(3-Chlorphenyl)dimethylaminomethyl]-1-phenylcyclohexanol, Hydrochlorid

1. Stufe

2-[(3-Chlorphenyl)dimethylaminomethyl]cyclohexanon

Zu 94 ml (94 mmol) Natriumiodidlösung (1 M in Acetonitril), mit einem Eisbad auf 0°C gekühlt, wurden unter Rühren 3,48 g (42,7 mmol) frisch getrocknetes Dimethylamin-Hydrochlorid gegeben, 12 ml (85,4 mmol) Triethylamin und 12 ml (94 mmol) Chlortrimethylsilan zugetropft und eine Stunde bei RT nachgerührt. Bei Eiskühlung wurden 4,8 ml (42,7 mmol) 3-Chlorbenzaldehyd zugegeben und noch eine Stunde bei RT weitergerührt. Es wurde erneut mit einem Eisbad auf 0°C gekühlt, 6,9 ml (42, 7 mmol) 1-(Pyrrolidino)-1-cyclohexen zugegeben und zwei Stunden bei RT weitergerührt. Zur Aufarbeitung wurde der Ansatz bei Eiskuhlung mit 60 ml halkonzentrierter Salzsaure versetzt, 10 Minuten gerührt, zweimal mit je 60 ml Ether gewaschen und mit 150 ml verdunnter Ammoniaklösung (5 Vol.% alkalisch gestellt (pH ca. 9). Es wurde dreimal mit je 60 ml Ether extrahiert, die vereinigten organischen Extrakte über Natriumsulfat getrocknet, filtriert und am RotationsVerdampfer (500 bis 10 mbar) ohne Wärmezufuhr eingeengt. Es wurden 8,97 g Rohbase (79,1% der Theorie) erhalten, aus der nach Beispiel 1 (3. Stufe) mit Chlortrimethylsilan/Wasser in 2-Butanon ein Hydrochlorid gefällt wurde. Hieraus wurde mit 90 ml Wasser und 15 ml Ammoniaklösung (25 Vol.%) die Base freigesetzt, dreimal mit je 50 ml Ether extrahiert, die vereinigten organischen Extrakte über Natriumsulfat getrocknet, filtriert und am Rotationsverdampfer ohne Wäremezufuhr (500 bis 10 mbar) eingeengt. Es wurden 7,05 g Rohbase (62,1% der Theorie) erhalten, aus der nach Beispiel 1 (2. Stufe) mit Chlortrimethyl silan/Wasser in 2-Butanon 7,38 g des Hydrochlorids von 2-[(3-Chlorphenyl)dimethylaminomethyl]cyclohexanon) 57,2% der Theorie) erhalten wurden.

2. Stufe

2-[(3-Chlorphenyl)dimethylaminomethyl]-1-phenylcyclohexanol, Hydrochlorid

Aus 2,5 g (8,27 mmol) des nach Stufe 1 erhaltenen Hydrochlorids von 2-[(3-Chlorphenyl)dimethylaminomethyl]cyclohexanon wurde mit 30 ml Wasser und 5 ml Ammoniaklösung (25 Vol.% ) die Base freigesetzt, dreimal mit je 30 ml Ether extrahiert, die vereinigten organischen Extrakte über Natriumsulfat getrocknet, filtriert und am Rotationsverdampfer ohne Wärmezufuhr (500 bis 10 mbar) eingeengt. 2,0 g (7,5 mmol) dieser Base wurden in 5 ml Tetrahydrofuran gelöst, bei Eisbadkühlung zu 4,5 ml (9,0 mmol) Phenylinagnesiumchiorid (2 M Lösung in Tetrahydrofuran) getropft und 15 Stunden bei RT gerührt. Zur Aufarbeitung wurden 10 ml gesättigte Amoniumchloridlosung bei Eisbadkühlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 2,30 g Rohbase (88,8% der Theorie) erhalten, aus der nach Beispiel 1 (3. Stufe) mit Chlortrimethylsilan/Wasser in 2-Butanon 2, 18 g 2-[(3-Chlorphenyl)dimethylaminomethyl]-1-phenylcyclohexanol, Hydrochlorid (76, 2% der Theorie) erhalten wurden, das sich beim Erhitzen ab 139°C zersetzt.

**Beispiel 43**

1-(2,4-Dichlorphenyl)-2-(3-dimethylaminophenylmethyl)-1-cyclohexanol, Hydrochlorid

0,76 g (31,2 mmol) Magnesiumspäne wurden in 10 ml Ether p.a. gerührt. Es wurde ein Gemisch aus 1,34 ml (15,6 mmol) Dibromethan und 3,52 g 1-Brom-2,4-dichlorbenzol, gelöst in 10 ml Ether, so zugetropft, daß das Reaktionsgemisch leicht siedete. Nach beendeter Zugabe wurde eine Stunde bei RT nachgerührt. 3,0 g (15,6 mmol) des nach Beispiel 1 hergestellten 2-(Dimethylaminophenylmethyl)cyclohexanons wurden in 10 ml Ether gelöst, bei Eisbadkühlung zum Grignard-Ansatz getropft und 15 Stunden bei RT gerührt. Zur Aufarbeitung wurden 20 ml gesättigte Ammoniumchloridlösung bei Eisbadkühlung zugegeben und bei RT dreimal mit je 50 ml Essigsaureethylester extrahiert. Die vereinigten organischen Extrakte wurden über Natriumsulfat getrocknet, filtriert und am Rotationsverdampfer (500 bis 10 mbar) eingeengt. Es wurden 4,92 g Rohbase (100 % der Theorie) erhalten, aus der nach Beispiel 1 (3. Stufe) mit Chlortrimethylsilan/Wasser in 2-Butanon ein Hydrochlorid gefällt wurde. Hieraus wurde mit 40 ml Wasser und 5 ml Natronlauge (32 m%) die Base freigesetzt, dreimal mit je 40 ml Ether extrahiert, die vereinigten organischen Extrakte über Natriumsulfat getrocknet, filtriert und am Rotationsverdampfer (500 bis 10 mbar) eingeengt. Es wurden 4,53 g Rohbase erhalten, die auf eine 3,5 x 30 cm Säule, gefüllt mit Kieselgel, gegeben wurde. Die Elution mit Essigsaureethylester/n-Hexan 1:4 ergab 2,74 g Base, aus der nach Beispiel 1 (3. Stufe) mit Chlortrimethylsilan/Wasser in 2-Butanon 2,46 g 1-(2, 9-Dichlorphenyl)-2-(3-dimethylaminophenylmethyl)-1-cyclohexanol, Hydrochlorid (45, 6% der Theorie) mit einem Schmelzpunkt von 192 - 195°C erhalten wurden.

**Beispiel 44**

1-Benzyl-2 [(3-Chlorphenyl)dimethylaminomethyl] cyclohexanol, Hydrochlorid

2,0 g (7,5 mmol) des nach Beispiel 42 hergestellten 2-[(3-Chlorphenyl)dimethylaminomethyl]cyclohexanons wurden in 10 ml Tetrahydrofuran gelöst, bei Eisbadkuhlung zu 4,5 ml (9,0 mmol) Benzylmagnesiumchlorid (2 M Losung in Tetrahydrofuran) getropft und 15 Stunden bei RT gerührt. Zur Aufarbeitung wurden 10 ml gesättigte Ammoniumchloridlösung bei Eisbadkülilutig zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 2,61 g Rohbase (97,0% der Theorie) erhalten, aus der nach Beispiel 1 (3. Stufe) mit Chlortrimethylsilan/Wasser in 2-Butanon 1, 24 g 1-Benzyl-2-[(3-Chlorphenyl)dimethylaminomethyl]cyclohexanol, Hydrochlorid (41,8% der Theorie) mit einem Schmelzpunkt von 161 - 163°C erhalten wurden.

**Beispiel 45**

1-Benzyl-2-[(2-chlorphenyl)dimethylaminomethyl]cyclohexanol, Hydrochlorid

0,27 g (11,3 mmol) Magnesiumspane wurden in 5 ml Ether p.a. gerührt. Es wurden 1,93 g (11,3 mmol) Benzybromid, gelöst in 10 ml Ether, so zugetropft, daß das Reaktionsgemisch leicht siedete. Nach beendeter Zugabe wurde eine Stunde bei RT nachgerührt. 2,5 g (9,4 mmol) des nach Beispiel 38 hergestellten 2-[(2-Chlorphenyl)dimethylaminomethyl]cyclohexanons wurden in 10 ml Ether gelöst, bei Eisbadkühlung zum Grignard-Ansatz getropft und 15 Stunden bei RT gerührt. Zur Aufarbeitung wurden 20 ml gesättigte Ammoniumchloridlösung bei Eisbadkühlung zugegeben und bei RT dreimal mit je 20 ml Ether extrahiert. Die vereinigten organischen Extrakte wurden über Natriumsulfat getrocknet, filtriert und am Rotationsverdampfer (500 bis 10 mbar) eingeengt. Es wurden 3,18 g Rohbase (94,4% der Theorie) erhalten, aus der nach Beispiel 1 (3. Stufe) mit Chlortrimethylsilan/Wasser in 2-Butanon ein Hydrochlorid gefallt wurde. Hieraus wurde mit 20 ml Wasser und 5 ml Ammoniaklösung (25 Vol .%) die Base freigesetzt, dreimal mit je 20 ml Ether extrahiert, die vereinigten organischen Extrakte über Natriumsulfat getrocknet, filtriert und am Rotationsverdampfer (500 bis 10 mbar) eingeengt. Es wurden 1,93 g Rohbase erhalten, die auf eine 3 x 25 cm Säule, gefüllt mit Kieseigel, gegeben wurde. Die Elution mit Essigsäureethylester/n-Hexan 1 :4 ergab 0,92 g Base, aus der nach Beispiel 1 (3. Stufe) mit Chlortrimethylsilan/Wasser in 2-Butanon 0, 43 g 1-Benzyl-2-[(2-chlorphenyl) dimethylaminomethyl]cyclohexanol, Hydrochlorid (11,5% der Theorie) mit einem Schmelzpunkt von 170°C erhalten wurden.

**Beispiel 46**

1-(4-tert.-Butylbenzyl)-2-[(3,4-dichlorphenyl)dimethylaminomethyl]cyclohexanol, Hydrochlorid

0,24 g (9,9 mmol) Magnesiumspane wurden in 5 ml Ether p.a. gerührt. Es wurden 1,81 g (9,9 mmol) 4-tert. -Butylbenzylchlorid, gelöst in 5 ml Ether, so zugetropft, daß das Reaktionsgemisch leicht siedete. Nach beendeter Zugabe wurde eine Stunde bei RT nachgerührt. 2,48 g (8,3 mmol) des nach Beispiel 39 hergestellten 2-[(3,4-Dichlorphenyl)-dimethylaminomethyl]cyclohexanons wurden in 10 ml Ether gelöst, bei Eisbadkühlung zum Grignard-Ansatz getropft und 15 Stunden bei RT geruhrt. Zur Aufarbeitung wurden 15 ml gesättigte Ammoniumchloridlösung bei Eisbadkühlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 3,69 g Rohbase (99,6% der Theorie) erhalten, aus der man nach Beispiel 1 (3. Stufe) mit Chlortrimethylsilan/Wasser in 2-Butanon 1,54 g 1-(4-tert.-Butylbenzyl)-2-[(3,4-dichlorphenyl)dimethylaminomethyl]cyclohexanol, Hydrochlorid (38,3% der

Theorie) erhielt, das sich beim Erhitzen ab 210°C zersetzt.

**Beispiel 47**

2-[Dimethylamino-(4-fluorpheny])methyl]-1-(3-trifluormethylbenzyl)cyclohexanol, Hydrochlorid

0,29 g (12,1 mmol) Magnesiumspäne wurden in 5 ml Ether p.a. gerührt. Es wurden 2,35 g (12,1 mnol) 3-Chlorbenzotrifluorid, gelöst in 5 ml Ether, so zugetropft, daß das Reaktionsgemisch leicht siedete. Nach beendeter Zugabe wurde eine Stunde bei RT nachgeruhrt. 2,51 g (10,1 mmol) des nach Beispiel 41 hergestellten 2-[Dimethylamino-(4-fluorphenyl)methyl]cyclohexanons wurden in 5 ml Ether gelöst, bei Eisbadkühlung zum Grignard-Ansatz getropft und 15 Stunden bei RT gerührt. Zur Aufarbeitung wurden 15 ml gesättigte Ammoniumchloridlosung bei Eisbadkuhlung zugegeben und bei RT dreimal mit je 15 ml Ether extrahiert. Die vereinigten organischen Extrakte wurden uber Natriumsulfat getrocknet, filtriert und am Rotationsverdampfer (500 bis 10 mbar) eingeengt. Es wurden 4,15 g Rohbase (101% der Theorie) erhalten, aus der man nach Beispiel 1 (3. Stufe) mit Chlortrimethylsilan/Wasser in 2-Butanon 3/10 g 2-[Dimethylamino-(4-fluorphenyl)methyl]-1-(3-trifluormethylbenzyl)cyclohexanol, Hydrochlorid (69,1% der Theorie) erhielt, das sich beim Erhitzen ab 210°C zersetzt.

**Beispiel 48**

2-(Dimethylaminophenylmethyl)bicyclohexyl-1-ol, Hydrochlorid

3,0 g (13,0 mmol) des nach Beispiel 1 hergestellten 2-(Dimethylaminophenylmethyl)cyclohexanons wurden in 10 ml Tetrahydrofuran gelost, bei Eisbadkuhlung zu 7,8 ml (15,6 mmol) Cyclohexylmagnesiumchlorid (2 M Lösung in Tetrahydrofuran) getropft und 15 Stunden bei RT gerührt. Zur Aufarbeitung wurden 20 ml gesattigte Ammoniumchloridlösung bei Eisbadkühlung zugegeben und bei RT dreimal mit je 20 ml Ether extrahiert. Die vereinigten organischen Extrakte wurden über Natriumsulfat getrocknet, filtriert und am Rotationsverdampfer (500 bis 10 mbar) eingeengt. Es wurden 4,03 g Rohbase (98,5% der Theorie) erhalten, aus der nach Beispiel 1 (3. Stufe) mit Chlortrimethylsilan/Wasser in 2-Butanon 2,22 g 2-(Dimethylaminophenylmethyl)bicyclohexyl-1-ol, Hydrochlorid (48,5% der Theorie) mit einem Schmelzpunkt von 222 - 223°C erhalten wurden.

**Beispiel 49**

2-(Dimethylaminophenylmethyl)-1-(4-methoxybenzyl)cyclohexanol, Hydrochiorid

0,38 g (15,6 mmol) Magnesiumspane wurden in 15 ml Tetra-hydrofuran p.a. gerührt. Es wurden 2,44 g (15,6 mmol) 4-Methoxybenzylchlorid, gelöst in 15 ml Tetrahydrofuran, so zugetropft, daß das Reaktionsgemisch leicht siedete. Nach beendeter Zugabe wurde 1,5 Stunden bei 65°C nachgerührt. 3,0 g (13,0 mmol) des nach Beispiel 1 hergestellten 2-(Dimethylaminophenylmethyl)cyclohexanons wurden in 15 ml Tetrahydrofuran gelöst, bei Eisbadkuhlung zum Grignard-Ansatz getropft und 15 Stunden bei RT geruhrt. Zur Aufarbeitung wurden 20 ml gesättigte Ammoniumchloridlösung bei Eisbadkuhlung zugegeben und bei RT dreimal mit je 20 ml Ether extrahiert. Die vereinigten organischen Extrakte wurden über Natriumsulfat getrocknet, filtriert und am Rotationsverdampfer (500 bis 10 mbar) eingeengt. Es wurden 4,26 g Rohbase (93,0% der Theorie) erhalten, aus der. man nach Beispiel 1 (3. Stufe) mit Chlortrimethylsilan/Wasser in 2-Butanon 2, 87 g 2-(Dimethylaminophenylmethyl)-1-(4-methoxybenzyl)cyclohexanol, Hydrochlorid (56,8% der Theorie) erhielt, das sich beim Erhitzen ab 130°C zersetzt.

**Beispiel 50**

1-(2,4-Difluorbenzyl)-2-(dimethylaminophenylmethyl)cyclohexanol, Hydrochlorid

0,29 g (11,9 mmol) Magnesiumspäne wurden in 5 ml Ether p.a. gerührt. Es wurden 2,47 g (11,9 mmol) 2,4-Difluorbenzylbromid, gelöst in 10 ml Ether, so zugetropft, daß das Reaktionsgemisch leicht siedete. Nach beendeter Zugabe wurde eine Stunde bei RT nachgeruhrt. 2,30 g (9,9 mmol) des nach Beispiel 1 hergestellten 2-(Dimethylaminophenylmethyl)cyclohexanons wurden in 10 ml Ether gelöst, bei Eisbadkühlung zum Grignard-Ansatz getropft und 15 Stunden bei RT gerührt. Zur Aufarbeitung wurden 10 ml gesättigte Ammoniumchloridlösung bei Eisbadkühlung zugegeben und bei RT dreimal mit je 15 ml Ether extrahiert. Die vereinigten organischen Extrakte wurden uber Natriumsulfat getrocknet, filtriert und am Rotationsverdampfer (500 bis 10 mbar) eingeengt. Es wurden 1,78 g Rohbase (49,9 % der Theorie) erhalten. Die wässrige Lösung wurde noch dreimal mit je 15 ml Ether und je 15 ml Dichlormethan extrahiert. Die vereinigten organischen Extrakte wurden über Natriumsulfat getrocknet, filtriert und am Rotationsverdampfer (500 bis 10 mbar) eingeengt. Es wurden 1, 31 g Rohbase (36, 7% der Theorie) erhalten. Aus beiden Basen wurde nach Beispiel 1 (3. Stufe) mit Chlortrimethylsilan/Wasser in 2-Butanon das Hydrochlorid gefallt. Man erhielt aus der ersten Base 0,95 g 1-(2,4-Difluorbenzyl)-2-(dimetylaminophenylmethyl)cyclohexanol, Hydrochlorid (20,2% der Theorie), aus der zweiten Base 1,27 g 1-(2,4-Difluorbenzyl)-2-(dimethylaminophenylmethyl)cyclohexanol, Hydrochlorid ( 32,4 % der Theorie) mit einem Schmelzpunkt von 178 °C.

**Beispiel 51**

1-(4-tert.-Butylbenzyl)-2-[(2-chlorphenyl)dimethylaminomethyl]cyclohexanol, Hydrochlorid

0,22 g (9,0 mmol) Magnesiumspäne wurden in 5 ml Ether p.a. gerührt. Es wurden 1,65 g (9,0 mmol) 4-tert. -Butylbenzylchlorid, gelöst in 5 ml Ether, so zugetropft, daß das Reaktionsgemisch leicht siedete. Nach beendeter Zugabe wurde eine Stunde bei RT nachgerührt. 2,0 g (7,5 mmol) des nach Beispiel 38 hergestellten 2-[(2-Chlorphenyl) dimethylaminomethyl]cyclohexanons wurden in 10 ml Ether gelöst, bei Eisbadkühlung zum Grignard-Ansatz getropft und 15 Stunden bei RT gerührt. Zur Aufarbeitung wurden 10 ml gesättigte Ammoniumchloridlösung bei Eisbadkuhlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 2,50 g Rohbase (80,1% der Theorie) erhalten, aus der nach Beispiel 1 (3. Stufe) mit Chlortrimethylsilan/Wasser in 2-Butanon 1,03 g 1-(4-tert.-Butylbenzyl)-2-[(3-chlorphenyl)dimethylaminomethyl]cyclohexanol, Hydrochlorid (30,5% der Theorie) mit einem Schmelzpunkt über 225°C erhalten wurden.

**Beispiel 52**

2- Dimethylamino- (3-phenoxyphenyl)methyl]-1-phenethylcyclohexanol, Hydrochlorid

2,0 g (6,2 mmol) des nach Beispiel 34 hergestellten 2-[Dimethylamino-(3-phenoxyphenyl)methyl]cyclohexanons wurden in 9 ml Tetrahydrofuran gelöst, bei Eisbadkuhlung zu 7,4 ml (9,0 mmol) Phenethylmagnesiumchlorid (1 M Lösung in Tetrahydrofuran) getropft und 15 Stunden bei RT gerührt. Zur Aufarbeitung wurden 10 ml gesättigte Ammoniumchloridlösung bei Eisbadkuhlung zugegeben und bei RT dreimal mit je 10 ml Ether extrahiert. Die vereinigten organischen Extrakte wurden über Natriumsulfat getrocknet, filtriert und am Rotationsverdampfer (500 bis 10 mbar) eingeengt. Es wurden 2,55 g Rohbase (96,0% der Theorie) erhalten, aus der nach Beispiel 1 (3. Stufe) mit Chlortrimethylsilan/Wasser in 2-Butanon ein Hydrochlorid gefällt wurde. Hieraus wurde mit 20 ml Wasser und 5 ml Ammoniaklösung (25 Vol.%) die Base freigesetzt, dreimal mit je 10 ml Ether extrahiert, die vereinigten organischen Extrakte uber Natriumsulfat getrocknet, filtriert und am Rotationsverdampfer (500 bis 10 mbar) eingeengt. Es wurden 1,51 g Rohbase (56,8% der Theorie) erhalten, aus der man nach Beispiel 1 (3. Stufe) mit Chlortrimethylsilan/Wasser in 2-Butanon 1,31 g 2-[Dimethylamino-(3-phenoxyphenyl)methyl]-1-phenethylcyclohexanol, Hydrochlorid (45,2 % der Theorie) erhielt, das sich beim Erhitzen ab 120°C zersetzt.

**Beispiel 53**

2- [Dimethylamino-(3-phenoxyphenyl)methyl]-1-(3-trifluormethylbenzyl)cyclohexanol, Hydrochlorid

0,18 g (7,4 4 mmol) Magnesiumspäne wurden in 5 ml Ether p.a. gerührt. Es wurden 1,44 g (7,4 4 mmol) 3-Chlormethylbenzotrifluorid, gelöst in 5 ml Ether, so zugetropft, daß das Reaktionsgemisch leicht siedete. Nach beendeter Zugabe wurde eine Stunde bei RT nachgerührt. 2,0 g (6,2 mmol) des nach Beispiel 34 hergestellten 2-[Dimethyiamino-(3-phenoxyphenyl)methyl]cyclohexanon wurden in 15 ml Ether gelöst, bei Eisbadkühlung zum Grignard-Ansatz getropft und 15 Stunden bei RT gerührt. Zur Aufarbeitung wurden 10 ml gesättigte Ammoniumchloridlösung bei Eisbadkuhlung zugegeben und bei RT dreimal mit je 10 ml Ether extrahiert. Die vereinigten organischen Extrakte wurden uber Natriumsulfat getrocknet, filtriert und am Rotationsverdampfer (500 bis 10 mbar) eingeengt. Es wurden 2,85 g Rohbase (95,3% der Theorie) erhalten, aus der nach Beispiel 1 (3. Stufe) mit Chlortrimethylsilan/Wasser in 2-Butanon ein Hydrochlorid gefällt wurde. Hieraus wurde mit 20 ml Wasser und 5 ml Ammoniaklösung (25 Vol.%) die Base freigesetzt, dreimal mit je 10 ml Ether extrahiert, die vereinigten organischen Extrakte über Natriumsulfat getrocknet, filtriert und am Rotationsverdampfer (500 bis 10 mbar) eingeengt. Es wurden 0,94 g Rohbase (31,4 % der Theorie) erhalten, aus der nach Beispiel 1 (3. Stufe) mit Chlortrimethylsilan/Wasser in 2-Butanon 0,35 g 2-[Dimethylamino-(3-phenoxyphenyl)methyl]-1- (trifluormethylbenzyl)cyclohexanol, Hydrochlorid (10,8 % der Theorie) erhalten wurden. Zur Reinigung wurde das Hydrochlorid mit 120 ml Cyclohexan bei 50°C geruhrt, im Eisbad abgekuhlt, dekantiert und der Rückstand getrocknet. Auf diese Weise wurden 0,27 g Hydrochlorid (8,4% der Theorie) erhalten.

**Beispiel 54**

1-(2,5-Difluorbenzyl)-2-(dimethylaminophenylmethyl)cyclohexanol, Hydrochlorid

0,29 g (11,9 mmol) Magnesiumspäne wurden in 5 ml Ether p.a. gerührt. Es wurden 2,47 g (11,9 mmol) 2,5-Difluorbenzylbromid, gelöst in 10 ml Ether, so zugetropft, daß das Reaktionsgemisch leicht siedete. Nach beendeter Zugabe wurde eine Stunde bei RT nachgeruhrt. 2,30 g (9,9 mmol) des nach Beispiel 1 hergestellten 2-(Dimethylaminophenylmethyl) cyclohexanons wurden in 10 ml Ether gelost, bei Eisbadkuhlung zum Grignard-Ansatz getropft und 15 Stunden bei RT gerührt. Zur Aufarbeitung wurden 15 ml gesättigte Ammoniumchloridlösung bei Eisbadkuhlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 3,49 g Rohbase (97,8. %der Theorie) erhalten, aus der nach Beispiel 1 (3. Stufe) mit Chlortrimethylsilan/Wasser in 2-Butanon ein Hydrochlorid gefallt wurde. Hieraus wurde mit 30 ml Wasser und 10 ml Ammoniaklösung (25 Vol.%) die Base freigesetzt, dreimal mit je 20 ml Ether extrahiert, die vereinigten organischen Extrakte über Natriumsulfat getrocknet, filtriert und am Rotationsverdampfer (500 bis 10 mbar) eingeengt. Es wurden 2,75 g Rohbase (77,0% der Theorie) erhalten, aus der nach Beispiel 1 (3. Stufe) mit Chlortrimethylsilan/Wasser in 2-Butanon 2,21 g 1- (2,5-Difluorbenzyl)-2-(dimethylaminophenylmethyl) cyclohexanol, Hydrochlorid (56, 4% der Theorie) mit einem Schmelzpunkt von 219 - 221°C erhalten wurden.

**Beispiel 55**

1-(3,4-Difluorbenzyl)-2-(dimethylaminophenylmethyl)cyclohexanol, Hydrochlorid

0,29 g (11,9 mmol) Magnesiumspäne wurden in 5 ml Ether p.a. gerührt. Es wurden 2,47 g (11,9 mmol) 3,4-Difluorbenzylbromid, gelöst in 10 ml Ether, so zugetropft, daß das Reaktionsgemisch leicht siedete. Nach beendeter Zugabe wurde eine Stunde bei RT nachgerührt. 2,30 g (9,9 mmol) des nach Beispiel 1 hergestellten 2- (Dimethylaminophenylmethyl) cyclohexanons wurden in 10 ml Ether gelöst, bei Eisbadkühlung zum Grignard-Ansatz getropft und 15 Stunden bei RT gerührt. Zur Aufarbeitung wurden 15 ml gesättigte Ammoniumchloridlösung bei Eisbadkuhlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 3,58 g Rohbase (100% der Theorie) erhalten, aus der nach Beispiel 1 (3. Stufe) mit Chlortrimethylsilan/Wasser in 2-Butanon ein Hydrochlorid gefällt wurde. Hieraus wurde mit 30 ml Wasser und 10 ml Ammoniaklösung (25 Vol.%) die Base freigesetzt, dreimal mit je 20 ml Ether extrahiert, die vereinigten organischen Extrakte über Natriumsulfat getrocknet, filtriert und am Rotationsverdampfer (500 bis 10 mbar) eingeengt. Es wurden 2,31 g Rohbase (64,7% der Theorie) erhalten, aus der nach Beispiel 1 (3. Stufe) mit Chlortrimethylsilan/Wasser in 2-Butanon 2,0 g 1-(3,4-Difluorbenzyl)-2-(dimethylaminophenylmethyl)cyclohexanol, Hydrochlorid (51,0 % der Theorie) mit einem Schmelzpunkt von 185 - 188°C erhalten wurden.

**Beispiel 56**

1-(2-Chlor-6-fluorbenzyl)-2-(dimethylaminophenylmethyl)-cyclohexanol, Hydrochlorid

0,38 g (15,6 mmol) Magnesiumspäne wurden in 10 ml Ether p.a. gerührt. Es wurden 2,79 g (15,6 mmol) 2-Chlor-6-fluorbenzylchlorid, gelost in 10 ml Ether, so zugebropft, daß das Reaktionsgemisch leicht siedete. Nach beendeter Zugabe wurde eine Stunde bei RT nachgerührt. 3,00 g (13,0 mmol) des nach Beispiel 1 hergestellten 2-(Dimethylaminophenylmethyl)cyclohexanons wurden in 15 ml Ether gelöst, bei Eisbadkühlung zum Grignard-Ansatz getropft und 15 Stunden bei RT gerührt. Zur Aufarbeitung wurden 20 ml gesättigte Ammoniumchloridlösung bei Eisbadkühlung zugegeben und bei RT dreimal mit je 30 ml Ether extrahiert. Die vereinigten organischen Extrakte wurden über Natriumsulfat getrocknet, filtriert und am Rotationsverdampfer (500 bis 10 mbar) eingeengt. Es wurden 4,92 g Rohbase (101 % der Theorie) erhalten, aus der man nach Beispiel 1 (3. Stufe) mit Chlortrimethylsilan/Wasser in 2-Butanon 3,28 g 1-(2-Chlor-6-fluorbenzyl)-2-(dimethylaminophenylmethyl)cyclohexanol, Hydrochlorid (61,2% der Theorie) erhielt, das sich beim Erhitzen ab 225°C zersetzt.

**Beispiel 57**

1-(2,3-Difluorbenzyl)-2-(dimethylaminophenylmethyl)cyclohexanol, Hydrochlorid

0,41 g (16,8 mmol) Magnesiumspäne wurden in 10 ml Ether p.a. gerührt. Es wurden 3,47 g (16,8 mmol)

2,3-Difluorbenzylbromid, gelöst in 10 ml Ether, so zugetropft, daß das Reaktionsgemisch leicht siedete. Nach beendeter Zugabe wurde eine Stunde bei RT nachgerührt. 3,23 g (14,0 mmol) des nach Beispiel 1 hergestellten 2-(Dimethylaminophenylmethy) cyclohexanons wurden in 15 ml Ether gelöst, bei Eisbadkühlung zum Grignard-Ansatz getropft und 15 Stunden bei RT gerührt. Zur Aufarbeitung wurden 20 ml gesättigte Ammoniumchloridlösung bei Eisbadkühlung zugegeben und bei RT dreimal mit je 20 ml Ether extrahiert. Die vereinigten organischen Extrakte wurden über Natriumsulfat getrocknet, filtriert und am Rotationsverdampfer (500 bis 10 mbar) eingeengt. Es wurden 5,13 g Rohbase (102% der Theorie) erhalten, aus der man nach Beispiel 1 (3. Stufe) mit Chlortrimethylsilan/Wasser in 2-Butanon 1,67 g 1-(2,3-Di-fluorbenzyl)-2-(dimethylaminophenylmethyl)cyclohexanol, Hydrochlorid (30,0% der Theorie) erhielt, das sich beim Erhitzen ab 140°C zersetzt.

**Beispiel 58**

1-Benzyl-2-[(4-chlorphenyl)dimethylaminometyl]cyclohexanol, Hydrochlorid

1. Stufe

2-[(4-Chlorphenyl)dimethylamino methyl]cyclohexamon

Zu 407 ml (407 mmol) Natriumiodidlösung (1 M in Acetonitril), mit einem Eisbad auf 0°C gekühlt, wurden unter Rühren 15,1 g (185 mmol) frisch getrocknetes Dimethylamin-Hydrochlorid gegeben, 52 ml (370 mmol) Triethylamin und 52 ml (407 mmol) Chlortrimethylsilan zugetropft und eine Stunde bei RT nachgerührt. Bei Eiskuhlung wurden 26,0 g (185 mmol) 4-Chlorbenzaldehyd zugegeben und noch eine Stunde bei RT weitergerührt. Es wurde erneut mit einem Eisbad auf 0°C gekühlt, 12,0 ml (185 mmol) 1-(Pyrrolidino)-1-cyclohexen zugegeben und zwei Stunden bei RT weitergerührt. Zur Aufarbeitung wurde der Ansatz bei Eiskühlung mit 280 ml halkonzentrierter Salzsaure versetzt, 10 Minuten gerührt, zweimal mit je 280 ml Ether gewaschen und mit 700 ml verdünnter Ammoniaklösung (5 Vol.%) alkalisch gestellt (pH ca. 9). Es wurde dreimal mit je 280 ml Ether extrahiert, die vereinigten organischen Extrakte über Natriumsulfat getrocknet, filtriert und am Rotationsverdampfer (500 bis 10 mbar) ohne Warmezufuhr eingeengt. Es wurden 16,2 g Rohbase (33,0% der Theorie) erhalten, aus der nach Beispiel 1 (2. Stufe) mit Chlortrimethylsilan/Wasser in 2-Butanon 14,8 g des Hydrochlorids von 2-[(4-Chlorphenyl)dimethylaminomethyl] cyclohexanon (57, 2% der Theorie) erhalten wurden.

2. Stufe

1-Benzyl-2-[(4-chlorphenyl)dimethylaminomethyl]cyclohexanol, Hydrochlorid

Aus 2,5 g (8,27 mmol) des nach Stufe 1 erhaltenen Hydrochlorids von 2-[(4-Chlorphenyl)dimethylaminomethyl] cyclohexanon wurde mit 30 ml Wasser und 5 ml Ammoniaklösung (25 Vol.%) die Base freigesetzt, dreimal mit je 30 ml Ether extrahiert, die vereinigten organischen Extrakte über Natriumsulfat getrocknet, filtriert und am Rotationsverdampfer ohne Wärmezufuhr (500 bis 10 mbar) eingeengt. 1,80 g (6,75 mmol) dieser Base wurden in 10 ml Tetrahydrofuran gelöst, bei Eisbadkuhlung zu 4,05 ml (8,13 mmol) Benzylmagnesiumchlorid (2 M Losung in Tetrahydrofuran) getropft und 15 Stunden bei RT gerührt. Es wurden 2,30 g Rohbase (94,9% der Theorie) erhalten, aus der man nach Beispiel 1 (3. Stufe) mit Chlortrimethylsilan/Wasser in 2-Butanon 1,24 g 1-Benzyl-2 [(4-chlorphenyl)dimethylaminomethyl]cyclohexanol, Hydrochlorid (46,7% der Theorie) erhielt, das sich beim Erhitzen ab 130°C zersetzt.

**Beispiel 59**

1-Dimethylamino-3-ethyl-2-methyl-1, 5-diphenylpentan-3-ol, Hydrochlorid

1. Stufe

1-(1-Ethylpropenyl)pyrrolidin

Zu 40 g (0,464 mol) 3-Pentanon, gelöst in 1600 ml n-Pentan, wurden 99 g (1,39 mol) Pyrrolidin, gelost in 460 ml n-Pentan, getropft und die Lösung mit einem Eisbad auf 0°C gekühlt. Innerhalb einer Stunde wurden 48,4 4 g (0,255 mol) Titantetrachlorid, gelöst in 480 ml n-Pentan, bei 0 - 10°C zugetropft, zwei Stunden bei RT nachgerührt und die Suspension filtriert. Das Filtrat wurde am Rotationsverdampfer (500 bis 10 mbar) eingeengt und auf diese Weise 44,3 g 1-(1-Ethylpropenyl)pyrrolidin (68,6% der Theorie) erhalten.

2. Stufe

1-Dimethylamino-2-methyl-1-phenylpentan-3-on

Zu 700 ml (700 mmol) Natriumiodidlösung (1 M in Acetonitril), mit einem Eisbad auf 0°C gekühlt, wurden unter Rühren 25,9 g (318 mmol) frisch getrocknetes Dimethylamin-Hydrochlorid gegeben, 89 ml (636 mmol) Triethylamin und 89 ml (700 mmol) Chlortrimethylsilan zugetropft und eine Stunde bei RT nachgeruhrt. Bei Eiskuhlung wurden 33,8 g (318 mmol) Benzaldehyd zugegeben und noch eine Stunde bei RT weitergerührt. Es wurde erneut mit einem Eisbad auf 0°C gekühlt, 44,3 g (318 mmol) 1-(1-Ethylpropenyl)pyrrolidin aus Stufe 1 zugegeben und zwei Stunden bei RT weitergerührt. Zur Aufarbeitung wurde der Ansatz bei Eiskuhlung mit 480 ml halkonzentrierter Salzsäure versetzt, 10 Minuten gerührt, zweimal mit je 480 ml Ether gewaschen und mit 1200 ml verdünnter Ammoniaklösung (5 Vol.%) alkalisch gestellt (pH ca. 9). Es wurde dreimal mit je 480 ml Ether extrahiert, die vereinigten organischen Extrakte über Natriumsulfat getrocknet, filtriert und am Rotationsverdampfer (500 bis 10 mbar) ohne Wärmezufuhr eingeengt. Es wurden 51,3 g Rohbase (73,6 % der Theorie) erhalten, aus der nach Beispiel 1 (2. Stufe) mit Chlortrimethylsilan/Wasser in 2-Butanon 26,5 g des Hydrochlorids von 1-Dimethylamino-2-methyl-1-phenylpentan-3-on(32,6% der Theorie) erhalten wurden.

3. Stufe

1-Dimethylamino-3-ethyl-2-methyl-1,5-diphenylpentan-3-ol, Hydrochlorid

Aus 2,5 g (9,77 mmol) des nach Stufe 2 erhaltenen Hydrochlorids von 1-Dimethylamino-2-methyl-1-phenylpentan-3-on wurde mit 30 ml Wasser und 5 ml Ammoniaklösung (25 Vol.%) die Base freigesetzt, dreimal mit je 30 ml Ether extrahiert, die vereinigten organischen Extrakte über Natriumsulfat getrocknet, filtriert und am Rotationsverdampfer ohne Warmezufuhr (500 bis 10 mbar) eingeengt. 1,90 g (8,7 mmol) dieser Base wurden in 13 ml Tetrahydrofuran gelöst, bei Eisbadkühlung zu 10,4 ml (10,4 mmol) Phenethylmagnesiumchlorid (1 M Lösung in Tetrahydrofuran) getropft und 15 Stunden bei RT gerührt. Zur Aufarbeitung wurden 15 ml gesättigte Ammoniumchloridlösung bei Eisbadkuhlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 1,96 g Rohbase (69,6% der Theorie) erhalten, aus der nach Beispiel 1 (3. Stufe) mit Chlortrimethylsilan/Wasser in 2-Butanon ein Hydrochlorid gefallt wurde. Hieraus wurde mit 20 ml Wasser und 5 ml Ammoniaklösung (25 Vol.%) die Base freigesetzt, dreimal mit je 20 ml Ether extrahiert, die vereinigten organischen Extrakte über Natriumsulfat getrocknet, filtriert und am Rotationsverdampfer (500 bis 10 mbar) eingeengt. Es wurden 0,72 g Rohbase erhalten, die auf eine 3,5 x 25 cm Saule, gefüllt mit Kieselgel, gegeben wurde. Die Elution mit Essigsäureethylester/n-Hexan 1:4 ergab 0,41 g Base, aus der nach Beispiel 1 (3. Stufe) mit Chlortrimethylsilan/Wasser in 2-Butanon 0, 19 g 1-Dimethylamino-3-ethyl-2-methyl-1,5-diphenylpentan-3-ol, Hydrochlorid (6,0% der Theorie) mit einem Schmelzpunkt von 63 - 66°C erhalten wurden.

**Beispiel 60**

1-(2-Chlorbenzyl)-2-[(2-chlorphenyl)dimethylaminomethyl]-Cyclohexanol, Hydrochlorid

0,22 g (9,0 mmol) Magnesiumspäne wurden in 5 ml Ether p.a. gerührt. Es wurden 1,45 g (9,0 mmol) 2-Chlorbenzylchlorid, gelöst in 5 ml Ether, so zugetropft, daß das Reaktionsgemisch leicht siedete. Nach beendeter Zugabe wurde eine Stunde bei RT nachgerührt. 2,00 g (7,5 mmol) des nach Beispiel 38 hergestellten 2- [(2-Chlorphenyl) dimethylaminomethyl]cyclohexanons wurden in 10 ml Ether gelöst, bei Eisbadkühlung zum Grignard-Ansatz getropft und 15 Stunden bei RT gerührt. Zur Aufarbeitung wurden 10 ml gesättigte Ammoniumchloridlösung bei Eisbadkühlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 2,57 g Rohbase (87,1% der Theorie) erhalten, aus der nach Beispiel 1 (3. Stufe) mit Chlortrimethylsilan/Wasser in 2-Butanon ein Hydrochlorid gefällt wurde. Hieraus wurde mit 20 ml Wasser und 5 ml Ammoniaklösung (25 Vol.%) die Base freigesetzt, dreimal mit je 20 ml Ether extrahiert, die vereinigten organischen Extrakte über Natriumsulfat getrocknet, filtriert und am Rotationsverdampfer (500 bis 10 mbar) eingeengt. Es wurden 0,74 g Rohbase erhalten, die auf eine 3 x 15 cm Säule, gefüllt mit Kieselgel, gegeben wurde. Die Elution mit Essigsäureethylester/n-Hexan 1:4 ergab 0,60 g Base, aus der nach Beispiel 1 (3. Stufe) mit Chlortrimethylsilan/Wasser in 2-Butanon 0,42 g 1-(2-Chlorbenzyl-2-[(2-chlorbenzyl)-dimethylaminomethyl]cyclohexanol, Hydrochlorid (12,9% der Theorie) mit einem Schmelzpunkt von 146 - 147°C erhalten wurden.

**Beispiel 61**

1-Benzyl-2-[(4-bromphenyl)dimethylaminomethyl]cyclohexanol, Hydrochlorid

1. Stufe

2-[(4-Bromphenyl)dimethylaminomethyl]cyclohexanon

Zu 71 ml (71 mmol) Natriumiodidlosung (1 M in Acetonitril), mit einem Eisbad auf 0°C gekühlt, wurden unter Rühren 2,64 g (32,4 mmol) frisch getrocknetes Dimethylamin-Hydrochlorid gegeben, 9,0 ml (65 mmol) Triethylamin und 9,0 ml (71 mmol) Chlortrimethylsilan zugetropft und eine Stunde bei RT nachgerührt. Bei Eiskühlung wurden 6,0 g (32,4 mmol) 4-Brombenzaldehyd zugegeben und noch eine Stunde bei RT weitergeruhrt. Es wurde erneut mit einem Eisbad auf 0°C gekühlt, 5,2 ml (2,45 mmol) 1-(Pyrrolidino)-1-cyclohexen zugegeben und zwei Stunden bei RT weitergerührt. Zur Aufarbeitung wurde der Ansatz bei Eiskuhlung mit 50 ml halkonzentrierter Salzsaure versetzt, 10 Minuten gerührt, zweimal mit je 50 ml Ether gewaschen und mit 120 ml verdünnter Ammoniaklösung (5 Vol. %)alkalisch gestellt (pH ca. 9). Es wurde dreimal mit je 50 ml Ether extrahiert, die vereinigten organischen Extrakte über Natriumsulfat getrocknet, filtriert und am Rotationsverdampfer (500 bis 10 mbar) ohne Wärmezufuhr eingeengt. Es wurden 7,37 g Rohbase (73,3% der Theorie) erhalten, aus der nach Beispiel 1 (2. Stufe) mit Chlortrimethylsilan/Wasser in 2-Butanon 6,48 g des Hydrochlorids von 2-[(4-Bromphenyl)dimethylaminomethyl]cyclohexanon (57,6% der Theorie) erhalten wurden.

2. Stufe

1-Benzyl-2-[(4-bromphenyl)dimethylaminomethyl]cyclohexanol, Hydrochlorid

Aus 2,0 g (5,77 mmol) des nach Stufe 1 erhaltenen Hydrochlorids von 2-[(4-Bromphenyl)dimethylaminomethyl]cyclohexanon wurde mit 20 ml Wasser und 5 ml Ammoniaklösung (25 Vol.%) die Base freigesetzt, dreimal mit je 20 ml Ether extrahiert, die vereinigten organischen Extrakte über Natriumsulfat getrocknet, filtriert und am Rotationsverdampfer ohne Wärmezufuhr (500 bis 10 mbar) eingeengt. 1,70 g (5,5 mmol) dieser Base wurden in 8,5 ml Tetrahydrofuran gelöst, bei Eisbadkühlung zu 3,3 ml (6,6 mmol) Benzylmagnesiumchlorid (2 M Lösung in Tetrahydrofuran) getropft und 15 Stunden bei RT gerührt. Zur Aufarbeitung wurden 10 ml gesättigte Ammoniumchloridlösung bei Eisbadkühlung zugegeben und bei RT dreimal mit je 10 ml Ether extrahiert. Die vereinigten organischen Extrakte wurden über Natriumsulfat getrocknet, filtriert und am Rotationsverdampfer (500 bis 10 mbar) eingeengt. Es wurden 1,91 g Rohbase (86,6% der Theorie) erhalten, aus der man nach Beispiel 1 (3. Stufe) mit Chlortrimethylsilan/Wasser in 2-Butanon 0,86 g 1-Benzyl-2-[(4-bromphenyl)dimethylaminomethyl] cyclohexanol, Hydrochlorid (35,9% der Theorie) erhielt, das sich beim Erhitzen ab 151°C zersetzt.

**Beispiel 62**

2-[(4-Chlorphenyl)dimethylaminomethyl]-1-(4-trifluormethylphenyl)cyclohexanol, Hydrochlorid

0,22 g (9,0 mmol) Magnesiumspäne wurden in 5 ml Ether p.a. gerührt. Es wurden 2,03 g (9,0 mmol) 4-Brombenzotrifluorid, gelöst in 5 ml Ether, so zugetropft, daß das Reaktionsgemisch leicht siedete. Nach beendeter Zugabe wurde eine Stunde bei R.T nachgerührt. 2,00 g (7,5 mmol) des nach Beispiel 58 hergestellten 2-[(4-Chlorphenyl)dimethylaminomethyl]cyclohexanon wurden in 10 ml Ether gelost, bei Eisbadkühlung zum Grignard-Ansatz getropft und 15 Stunden bei RT gerührt. Zur Aufarbeitung wurden 10 ml gesättigte Ammoniumchloridlösung bei Eisbadkuhlung zugegeben und bei RT dreimal mit je 15 ml Ether extrahiert. Die vereinigten organischen Extrakte wurden uber Nüatriumsulfat getrocknet, filtriert und am Rotationsverdampfer (500 bis 10 mbar) eingeengt. Es wurden 2,65 g Rohbase (85,4 % der Theorie) erhalten, aus der nach Beispiel 1 (3. Stufe) mit Chlortrimethylsilan/Wasser in 2-Butanon ein Hydrochlorid gefallt wurde. Hieraus wurde mit 20 ml Wasser und 5 ml Ammoniaklösung (25 Vol. %) die Base freigesetzt, dreimal mit je 20 ml Ether extrahiert, die vereinigten organischen Extrakte uber Natriumsulfat getrocknet, filtriert und am Rotationsverdampfer (500 bis 10 mbar) eingeengt. Es wurden 0,50 g Rohbase (16,2% der Theorie) erhalten, aus der nach Beispiel 1 (3. Stufe) mit Chlortrimethylsilan/Wasser in 2-Butanon 0,20 g 2-[(4-Chlorphenyl)dimethylaminomethyl-1-(4-trifluormethylphenyl)cyclohexanol, Hydrochlorid (6,0% der Theorie) mit einem Schmelzpunkt uber 240°C erhalten wurden.

**Beispiel 63**

2-[(4-Chlorphenyl)dimethylaminomethyl]-1-(4-trifluor methylbenzyl)cyclohexanol, Hydrochlorid

0,22 g (9,0 mmol) Magnesiumspäne wurden in 5 ml Ether p.a. gerührt. Es wurden 1,76 g (9,0 mmol) 3-Chlormethylbenzotrifluorid, gelost in 5 ml Ether, so zugetropft, daß das Reaktionsgemisch leicht siedete. Nach beendeter Zugabe wurde eine Stunde bei RT nachgerührt. 2,00 g (7,5 mmol) des nach Beispiel 58 hergestellten 2-[(4-Chlorphenyl) dimethylaminomethyl]cyclohexanon wurden in 10 ml Ether gelöst, bei Eisbadkühlung zum Grignard-Ansatz getropft und 15 Stunden bei RT gerührt. Zur Aufarbeitung wurden 10 ml gesättigte Ammoniumchloridlösung bei Eisbadkühlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 1,46 g Rohbase (45,6% der Theorie) erhalten, aus der nach Beispiel 1 (3. Stufe) mit Chlortrimethylsilan/Wasser in 2-Butanon ein Hydrochlorid gefällt wurde. Hieraus wurde mit 15 ml Wasser und 5 ml Ammoniaklösung (25 Vol.%) die Base freigesetzt, dreimal mit je 15 ml Ether extrahiert, die vereinigten organischen Extrakte über Natriumsulfat getrocknet, filtriert und am Rotationsverdampfer (500 bis 10 mbar) eingeengt. Es wurden 0,33 g Rohbase (10,3% der Theorie) erhalten, aus der man nach Beispiel 1 (3. Stufe) mit Chlortrimethylsilan/Wasser in 2-Butanon 0,12 g 2-[(4-Chlorphenyl])dimethylaminomethyl]-1-(3-trifluormethylbenzyl)cyclohexanol, Hydrochlorid (3,6% der Theorie) erhielt, das sich beim Erhitzen ab 115°C zersetzt.

**Beispiel 64**

1-(4-tert.-Butylbenzyl)-2-[dimethylamino-(3-phenoxyphenyl)methyl]cyclohexanol, Hydrochlorid

0,15 g (6,3 mmol) Magnesiumspäne wurden in 5 ml Ether p.a. gerührt. Es wurden 1,15 g (6,3 mmol) 4-tert. -Butylbenzylchlorid, gelöst in 5 ml Ether, so zugetropft, daß das Reaktionsgemisch leicht siedete. Nach beendeter Zugabe wurde eine Stunde bei RT nachgeruhrt. 1,70 g (5,3 mmol) des nach Beispiel 34 hergestellten 2-[Dimethylamino-(3-phenoxyphenyl)methyl] cyclohexanons wurden in 5 ml Ether gelöst, bei Eisbadkühlung zum Grignard-Ansatz getropft und 15 Stunden bei RT geruhrt. Zur Aufarbeitung wurden 10 ml gesättigte Ammoniumchloridlösung bei Eisbadkühlung zugegeben und bei RT dreimal mit je 10 ml Ether extrahiert. Die vereinigten organischen Extrakte wurden über Natriumsulfat getrocknet, filtriert und am Rotationsverdamfer (500 bis 10 mbar) eingeengt. Es wurden 2,01 g Rohbase (68,6% der Theorie) erhalten, aus der nach Beispiel 1 (3. Stufe) mit Chlortrimethylsilan/Wasser in 2-Butanon ein Hydrochlorid gefällt wurde. Hieraus wurde mit 20 ml Wasser und 5 ml Ammoniaklosung (25 Vol.%) die Base freigesetzt, dreimal mit je 15 ml Ether extrahiert, die vereinigten organischen Extrakte über Natriumsulfat getrocknet, filtriert und am Rotationsverdampfer (500 bis 10 mbar) eingeengt. Es wurden 0, 70 g Rohbase erhalten, die auf eine 3 x 15 cm Säule, gefüllt mit Kieselgel, gegeben wurde. Die Elution mit Essigsäureethylester/n-Hexan 1:4 ergab 0,40 g Base, aus der nach Beispiel 1 (3. Stufe) mit Chlortrimethylsilan/Wasser in 2-Butanon 0,22 g 1-(4-tert.-Butylbenzyl)-2-[dimethylamino-(3-phenoxyphonyl)methyl] cyclohexanol, Hydrochlorid (8,2% der Theorie) mit einem Schmelzpunkt von 1.92 - 195°C erhalten wurden, das sich beim Erhitzen ab 92°C zersetzt.

**Beispiel 65**

4-{Dimethylamino-[2-hydroxy-2(4-tri fluormethylphenyl)-cyclohexyl]methyl}benzonitril, Hydrochlorid

1. Stufe

4-[Dimethylamino-(2-oxocyclohexyl)methyl]benzonitril

Zu 75 ml (75 mmol) Natriumiodidlösung (1 M in Acetonitril), mit einem Eisbad auf 0°C gekühlt, wurden unter Rühren 2,8 g (34,3 mmol ) frisch getrocknetes Dimethylamin-Hydrochlorid gegeben, 9,6 ml (68,6 mnol) Triethylamin und 9,5 ml (75,5 mmol) Chlortrimethylsilan zugetropft und eine Stunde bei RT nachgerührt. Bei Eiskühlung wurden 4,50 g (34,3 mmol) 4-Cyanobenzaldehyd zugegeben und noch eine Stunde bei RT weitergeruhrt. Es wurde erneut mit einem Eisbad auf 0°C gekuhlt, 5,5 ml (34,3 mmol) 1-(Pyrrolidino)-1-cyclohexen zugegeben und zwei Stunden bei RT weitergeruhrt. Zur Aufarbeitung wurde der Ansatz bei Eiskühlung mit 50 ml halkonzentrierter Salzsäure versetzt, 10 Minuten gerührt, zweimal mit je 50 ml Ether gewaschen und mit 130 ml verdünnter Ammoniaklösung (5 Vol.%) älkalish gestellt (pH ca. 9) . Es wurde dreimal mit je 50 ml Ether extrahiert, die vereinigten organischen Extrakte über Natriumsulfat getrocknet, filtriert und am Rotationsverdampfer (500 bis 10 mbar) ohne Wärmezufuhr eingeengt. Es wurden 8,2 g Rohbase (93% der Theorie) erhalten, aus der nach Beispiel 1 (2. Stufe) mit Chlortrimethylsilan/Wasser in 2-Butanon 6,75 g des Hydrochlorids von 4-[Dimethylamino-(2-oxocyclohexyl)methyl] benzonitril (67,2% der Theorie) erhalten wurden.

2. Stufe

4-{Dimethylamino-[2-hydroxy-2-(4-trifluormethylphenyl)-cyclohexyl]methyl}benzonitril, Hydrochlorid

0,34 g (14, 0 mmol) Magnesiumspäne wurden in 10 ml Ether p.a. geruhrt . Es wurden 3,16 g (14,0 mmol) 4-Brombenzotrifluorid, gelost in 10 ml Ether, so zugetropft, daß das Reaktionsgemisch leicht siedete. Nach beendeter Zugabe wurde eine Stunde bei RT nachgerührt. Aus 3,5 g (12,0 mmol) des nach Stufe 1 erhaltenen Hydrochlorids von 4-[Dimetylamino-(2-oxocyclohexyl)methyl]benzonitril wurde mit 30 ml Wasser und 5 ml Ammoniaklösung (25 Vol.% ) die Base freigesetzt, dreimal mit je 30 ml Ether extrahiert, die vereinigten organischen Extrakte über Natriumsulfat getrocknet, filtriert und am Rotationsverdampfer ohne Wärmezufuhr (500 bis 10 mbar) eingeengt. 3,0 g (11,7 mmol) dieser Base wurden in 10 ml Ether gelöst, bei Eisbadkühlung zum Grignard-Ansatz getropft und 15 Stunden bei RT gerührt. Zur Aufarbeitung wurden 20 ml gesättigte Ammoniumchloridlösung bei Eisbadkülung zugegeben und bei RT dreimal mit je 20 ml Ether extrahiert. Die vereinigten organischen Extrakte wurden über Natriumsulfat getrocknet, filtriert und am Rotationsverdampfer (500 bis 10 mbar) eingeengt. Es wurden 3,52 g Rohbase (74,7% der Theorie) erhalten, aus der nach Beispiel 1 (3. Stufe) mit Chlortrimethylsilan/Wasser in 2-Butanon 0,17 g 4-{Dimethylamino-[2-hydroxy-2-(4-trifluormethylphenyl)cyclohexyl]methyl}benzonitril, Hydrochlorid (3,3% der Theorie) mit einem Schmelzpunkt über 250°C erhalten wurden.

**Beispiel 66**

2-(Dimethylamino-o-tolylmethyl)-1-phenylcyclohexanol, Hydrochlorid

1. Stufe

2-(Dimethylamino-o-tolylmethyl)cyclohexanon

Zu 183 ml (183 mmol) Natriumiodidlösung (1 M in Acetonitril), mit einem Eisbad auf 0°C gekühlt, wurden unter Rühren 6,79 g (83,2 mmol) frisch getrocknetes Dimethyl-amin-Hydrochlorid gegeben, 23 ml (166 mmol) Triethylamin und 23 ml (183 mmol) Chlortrimethylsilan zugetropft und eine Stunde bei RT nachgerührt. Bei Eiskühlung wurden 10,0 g (83,2 mmol) 2-Tolualdehyd zugegeben und noch eine Stunde bei RT weitergeruhrt. Es wurde erneut mit einem Eisbad auf 0°C gekühlt, 13,4 ml (83,2 mmol) 1-(Pyrrolidino)-1-cyclohexen zugegeben und zwei Stunden bei RT weitergerührt. Zur Aufarbeitung wurde der Ansatz bei Eiskuhluiig mit 125 ml halkonzentrierter Salzsaure versetzt, 10 Minuten gerührt, zweimal mit je 125 ml Ether gewaschen und mit 310 ml verdünnter Ammoniaklösung (5 Vol.%) alkalisch gestellt (pH ca. 9) . Es wurde dreimal mit je 125 ml Ether extrahiert, die vereinigten organischen Extrakte uber Natriumsulfat getrocknet, filtriert und am Rotationsverdampfer (500 bis 10 mbar) ohne Wärmezufuhr eingeengt. Es wurden 11,8 g Rohbase (57,8 % der Theorie) erhalten, aus der nach Beispiel 1 (2. Stufe) mit Chlortrimethylsilan/Wasser in 2-Butanon 10,4 g des Hydrochlorids von 2-(Dimethylamino-o-tolylmethyl)cyclohexanon (44, 4% der Theorie) erhalten wurden.

2. Stufe

2-(Dimethylamino-o-tolylmethyl)-1-phenylcyclohexanol, Hydrochlorid

Aus 3,0 g (10,6 mmol) des nach Stufe 1 erhaltenen Hydrochlorids von 2-(Dimethylamino-o-tolylmethyl)cyclohexanon wurde mit 30 ml Wasser und 5 ml Ammoniaklösung (25 Vol.%) die Base freigesetzt, dreimal mit je 30 ml Ether extrahiert, die vereinigten organischen Extrakte über Natriumsulfat getrocknet, filtriert und am Rotationsverdampfer ohne Wärmezufuhr (500 bis 10 mbar) eingeengt. 2,50 g (10,2 mmol) dieser Base wurden in 15 ml Tetrahydrofuran gelöst, bei Eisbadkühlung zu 6,1 ml (12,2 mmol) Phenylmagnesiumchlorid (2 M Lösung in Tetrahydrofuran) getropft und 15 Stunden bei RT gerührt. Zur Aufarbeitung wurden 15 ml gesättigte Ammoniumchloridlösung bei Eisbadkühlung zugegeben und bei RT dreimal mit je 20 ml Ether extrahiert. Die vereinigten organischen Extrakte wurden uber Natriumsulfat getrocknet, filtriert und am Rotationsverdampfer (500 bis 10 mbar) eingeengt. Es wurden 3, 12 g Rohbase (94,5% der Theorie) erhalten, aus der man nach Beispiel 1 (3. Stufe) mit Chlortrimethylsilan/Wasser in 2-Butanon 1,97 g 2-(Dimethylamino-o-tolylmethyl)-1-phenylcyclohexanol, Hydrochlorid (53,7% der Theorie) erhielt, das sich beim Erhitzen ab 137°C zersetzt.

**Beispiel 67**

1-Benzyl- 2-(dimethylamino-o-tolylmethyl)cyclohexanol, Hydrodilorid

2,0 g (7,5 mmol) des nach Beispiel 66 hergestellten 2-(Dimethylamino-o-tolylmethyl)cyclohexanons wurden in 15 ml Tetrahydrofuran gelöst, bei Eisbadkühlung zu 6,1 ml (12, 2 mmol) Benzylmagnesiumchlorid (2 M Lösung in Tetrahydrofuran) getropft und 15 Stunden bei RT gerührt. Zur Aufarbeitung wurden 158 ml gesättigte Ammoniumchloridlösung bei Eisbadkühlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 3,39 g Rohbase (98,5 % der Theorie) erhalten, aus der nach Beispiel 1 (3. Stufe) mit Chlortrimethylsilan/Wasser in 2-Butanon 0,83 g 1-Benzyl-2-(dimethylamino-o-tolylmethylcyclohexanol, Hydrochlorid (21,8 % der Theorie) mit einem Schmelzpunkt von 180 - 183°C erholten wurden.

**Beispiel 68**

2-(Dimethylaminophenylmethyl)-1-(3-phenylpropyl)cyclohexanol, Hydrochlorid

0,32 g (13,0 mmol) Magnesiumspäne wurden in 10 ml Ether p.a. gerührt. Es wurden 2, 58 g (13,0 mmol) 1-Brom-3-phenylpropan, gelöst in 10 ml Ether, so zugetropft, daß das Reaktionsgemisch leicht siedete. Nach beendeter Zugabe wurde eine Stunde bei RT nachgerührt. 2,50 g (10,8 mmol) des nach Beispiel 1 hergestellten 2-(Dimethylaminophenylmethyl)cyclohexanons wurden in 10 ml Ether gelost, bei Eisbadkühlung zum Grignard-Ansatz getropft und 15 Stunden bei RT gerührt. Zur Aufarbeitung wurden 15 ml gesättigte Ammoniumchloridlösung bei Eisbadkühlung zugegeben und bei RT dreimal mit je 15 ml Ether extrahiert. Die vereinigten organischen Extrakte wurden uber Natriumsulfat getrocknet, filtriert und am Rotationsverdampfer (500 bis 10 mbar) eingeengt. Es wurden 3,73 g Rohbase (98,2 %der Theorie) erhalten, aus der man nach Beispiel 1 (3. Stufe) mit Chlortrimethylsilan/Wasser in 2-Butanon 2,92 g 2-(Dimethylaminophenylmethyl)-1-(3-phenylpropyl)cycloexanol, Hydrochlorid (67,7 % der Theorie) erhielt, das sich beim Erhitzen ab 90°C zersetzt.

**Beispiel 69**

2-[(2-Chlorphenyl)dimethylaminomethyl]-1-[2-(4-fluorphenyl)ethyl]cyclohexanol, Hydrochlorid

0,22 g (9,0 mmol) Magnesiumspäne wurden in 5 ml Ether p.a. gerührt. Es wurden 1,83 g (9,0 mmol) 1-(2-Bromethyl)-4-fluorbenzol, gelöst in 5 ml Ether, so zugetropft, daß das Reaktionsgemisch leicht siedete. Nach beendeter Zugabe wurde eine Stunde bei RT nachgerührt. 2,00 g (7,5 mmol) des nach Beispiel 38 hergestellten 2-[(2-Chlorphenyl) dimethylaminomethyl]cyclohexanons wurden in 10 ml Ether gelöst, bei Eisbadkühlung zum Grignard-Ansatz getropft und 15 Stunden bei RT gerührt. Zur Aufarbeitung wurden 10 ml gesättigte Ammoniumchloridlosung bei Eisbadkuhlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 2,85 g Rohbase (97,2% der Theorie) erhalten, aus der man nach Beispiel 1 (3. Stufe) mit Chlortrimethylsilan/Wasser in 2-Butanon 1,74 g 2-[(2-chlorphenyl)dimethylaminomethyl]-1-[2-(4-fluorphenyl)ethyl]cyclohexanol, Hydrochlorid (54,1% der Theorie) erhielt, das sich beim Erhitzen ab 170°C zersetzt.

**Beispiel 70**

2-[Dimethylaminothiophen-2-ylmethyl ]-1-(3-trifluormethylbenzyl)cyclohexanol, Hydrochlorid

1. Stufe

2-[Dimethylaminothiophen-2-ylmethyl]cyclohexanon

Zu 118 ml (118 mmol) Natriumiodidlösung (1 M in Acetonitril), mit einem Eisbad auf 0°C gekühlt, wurden unter Rühren 4,36 g (53,5 mmol) frisch getrocknetes Dimethylamin-Hydrochlorid gegeben, 15 ml (107 mmol) Triethylamin und 15 ml (118 mmol) Chlortrimethylsilan zugetropft und eine Stunde bei RT nachgerührt. Bei Eiskühlung wurden 6,0 g (53,5 mmol) Thiophen-2-carboxaldehyd zugegeben und noch eine Stunde bei RT weitergeruhrt. Es wurde erneut mit einem Eisbad auf 0°C gekühlt, 8,6 ml (53,5 mmol) 1-(Pyrrolidino)-1-cyclohexen zugegeben und zwei Stunden bei RT weitergeruhrt. Zur Aufarbeitung wurde der Ansatz bei Eiskuhlung mit 80 ml halkonzenbrierter Salzsäure versetzt, 10 Minuten gerührt, zweimal mit je 80 ml Ether gewaschen und mit 200 ml verdünnter Ammoniaklösung (5 Vol. %)

alkalisch gestellt (pH ca. 9). Es wurde dreimal mit je 80 ml Ether extrahiert, die vereinigten organischen Extrakte uber Natriumsulfat getrocknet, filtriert und am Rotationsverdampfer (500 bis 10 mbar) ohne Wärmezufuhr eingeengt. Es wurden 8,09 g Rohbase von 2-(Dimethylaminothiophen-2-ylmethyl)cyclohexanon (63,7% der Theorie) erhalten.

2. Stufe

2-(Dimethylaminothiophen-2-ylmethyl)-1-(3-trifluormethylbenzyl)cyclohexanol, Hydrochlorid

0,31 g (12,6 mmol) Magnesiumspäne wurden in 10 ml Ether p.a. gerührt. Es wurden 2,46 g (12,6 mmol) 3-Chlormethylbenzotrifluorid, gelost in 10 ml Ether, so zugetropft, daß das Reaktionsgemisch leicht siedete. Nach beendeter Zugabe wurde eine Stunde bei RT nachgerührt. 2,50 g (10,5 mmol) des nach Stufe 1 hergestellten 2-(Dimethylaminothiopen-2-ylmethyl)cyclohexanons wurden in 10 ml Ether gelost, bei Eisbadkyhlung zum Grignard-Ansatz getropft und 15 Stunden bei RT gerührt. Zur Aufarbeitung wurden 15 ml gesättigte Ammoniumchloridlösung bei Eisbadkuhlung zugegeben und bei RT dreimal mit je 20 ml Ether extrahiert. Die vereinigten organischen Extrakte wurden über Natriumsulfat getrocknet, filtriert und am Rotationsverdampfer (500 bis 10 mbar) eingeengt. Es wurden 3,33 g Rohbase (79,6% der Theorie) erhalten, aus der nach Beispiel 1 (3. Stufe) mit Chlortrimethylsilan/Wasser in 2-Butanon ein Hydrochlorid gefällt wurde. Hieraus wurde mit 20 ml Wasser und 5 ml Ammoniaklösung (25 Vol.%) die Base freigesetzt, dreimal mit je 20 ml Ether extrahiert:, die vereinigten organischen Extrakte über Natriumsulfat getrocknet, filtriert und am Rotationsverdampfer (500 bis 10 mbar) eingeengt. Es wurden 0,63 g Rohbase (15,0% der Theorie) erhalten, aus der man nach Beispiel 1 (3. Stufe) mit Chlortrimethylsilan/Wasser in 2-Butanon 0,39 g 2-[Dimethylaminothiophen-2-ylmethyl]-1(3-trifluormethylbenzyl)cyclohexanol, Hydrochlorid (8,5% der Theorie) erhielt, das sich ab 98°C zersetzt.

**Beispiel 71**

Methyl-4-[2-(dimethylaminophenylmethyl)-1-hydroxycyclohexyl]benzoat, Hydrochlorid

Zu 2,72 g (10,4 mmol) Methyl-4-iodbenzoat, gelost in 20 ml Ether und mit einem Trockeneis/Isopropanol-Bad auf -40°C gekühlt, wurden 5,6 ml (10,4 mmol) Isopropylmagnesiumchlorid (2 M Lösung in Ether) getropft und eine Stunde nachgerührt. 2,0 g (8,65 inmol) des nach Beispiel 1 hergestellten 2-(Dimethylaminophenylmethyl)cyclohexanons wurden in 10 ml Ether gelöst, bei -40°C zum Grignard-Ansatz getropft und 15 Stunden bei RT gerührt. Zur Aufarbeitung wurden 20 ml gesättigte Ammoniumchloridlösung bei Eisbadkühlung zugegeben und bei RT dreimal mit je 20 ml Ether extrahiert. Die vereinigten organischen Extrakte wurden uber Natriumsulfat getrocknet, filtriert und am Rotationsverdampfer (500 bis 10 mbar) eingeengt. Es wurden 3,19 g Rohbase (100% der Theorie) erhalten, aus der man nach Beispiel 1 (3. Stufe) mit Chlortrimethylsilan/Wasser in 2-Butanon 3,49 g Methyl-4-[2-(dimethylaminophenylmethyl)-1-hydroxycyclohexyl]benzoat, Hydrochlorid (57,3 % der Theorie) erhielt, das sich ab 140°C zersetzt.

**Beispiel 72**

1-Benzyl-2-(dimethylaminophenylmethyl)-4-phenylcyclohexanol, Hydrochlorid

1. Stufe

1-(4-Phenylcyclohex-1-enyl)pyrrolidin

Zu 30,0 g (0,172 mol) 4-Phenylcyclohexanon, gelöst in 860 ml n-Hexan, wurden 36,8 g (0,517 mol) Pyrrolidin, gelöst in 170 ml n-Hexan, getropft und die Lösung mit einem Eisbad auf 0°C gekühlt. Innerhalb einer Stunde wurden 18,0 g (0,095 mol) Titantetrachlorid, gelöst in 140 ml. n-Hexan, bei 0 - 10°C zugetropft, zwei Stunden bei RT nachgerührt und die Suspension filtriert. Das Filtrat wurde am Rotationsverdampfer (500 bis 10 mbar) eingeengt und das zurückbleibende Öl zur Reinigung bei weniger als 1 mbar destilliert; die Hauptfraktion siedete bei 135°C. Es wurden 22,2 g Rohprodukt erhalten, die wegen unvollständigen Umsatzes erneut in der Kälte mit 27,2 g (0,379 mol) Pyrrolidin, gelöst in 125 ml n-Hexan, und 13,1 g (0, 069 Mol) Titantetrachlorid, gelöst in 140 ml n-Hexan, versetzt und anschließend für zwei Stunden zum Ruckfluß erhitzt wurden. Auf diese Weise wurden 20,2 g 1-(4-Phenylcyclohex-1-enyl)pyrrolidin (51,7% der Theorie) erhalten.

2. Stufe

2-(Dimethylaminophenylmethyl)-4-phenylcyclohexanon

Zu 58 ml (58 mmol) Natriumiodidlösung (1 M in Acetonitril), mit einem Eisbad auf 0°C gekuhlt, wurden unter Rühren 2,15 g (26,4 mmol) frisch getrocknetes Dimethylamin-Hydrochlorid gegeben, 7, 4 ml (52,8 mmol) Triethylamin und 7,3 ml (58,0 mmol) Chlortrimethylsilan zugetropft und eine Stunde bei RT nachgerührt. Bei Eiskühlung wurden 2,80 g (26,4 mmol) Benzaldehyd zugegeben und noch eine Stunde bei RT weitergerührt. Es wurde erneut mit einem Eisbad auf 0°C gekühlt, 6,00 g (26,4 mmol) 1- (4-Phenylcyclohex-1-enyl)pyrrolidin aus Stufe 1 zugegeben und zwei Stunden bei RT weitergerührt. Zur Aufarbeitung wurde der Ansatz bei Eiskühlung mit 40 ml halkonzentrierter Salzsäure versetzt, 10 Minuten gerührt, zweimal mit je 40 ml Ether gewaschen und mit 100 ml verdünnter Ammoniaklösung (5 Vol.%) alkalisch gestellt (pH ca. 9). Es wurde dreimal mit je 40 ml Ether extrahiert, die vereinigten organischen Extrakte über Natriumsulfat getrocknet, filtriert und am Rotationsverdampfer (500 bis 10 mbar) ohne Wärmezufuhr eingeengt. Es wurden 6,77 g Rohbase (83,5% der Theorie) erhalten, aus der nach Beispiel 1 (2. Stufe) mit Chlortrimethylsilan/ Wasser in 2-Butanon 5,95 g des Hydrochlorids von 2-(Dimethylaminophenylmethyl)-4-phenylcyclohexanon (65,5% der Theorie) erhalten wurden.

3.Stufe

1-Benzyl-2-(dimethylaminophenylmethyl)-4-phenylcyclohexanol, Hydrochlorid

Aus 2,5 g (7,27 mmol) des nach Stufe 2 erhaltenen Hydrochlorids von 2-(Dimethylaminophenylmethyl)-4-phenylcyclohexanons wurde mit 30 ml Wasser und 5 ml Ammoniaklösung (25 Vol. %) die Base freigesetzt, dreimal mit je 30 ml Ether extrahiert, die vereinigten organischen Extrakte über Natriumsulfat getrocknet, filtriert und am Rotationsverdampfer ohne Wärmezufuhr (500 bis 10 mbar) eingeengt. 2,00 g (6,51 mmol) dieser Base wurden in 10 ml Tetrahydrofuran gelost, bei Eisbadkühlung zu 3,9 ml (7,8 mmol) Benzylmagnesiumchlorid (2 M Lösung in Tetrahydrofuran) getropft und 15 Stunden bei RT gerührt, Zur Aufarbeitung wurden 10 ml gesättigte Ammoniumchloridlösung bei Eisbadkuhlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 2,12 g Rohbase (84,6 % der Theorie) erhalten, aus der nach Beispiei 1 (3. Stufe) mit Chlortrimethylsilan/Wasser in 2-Butanon 1,67 g 1-Benzyl-2-(dimethylaminophenylmethyl)-4-phenylcyclohexanol, Hydrochlorid (60,7% der Theorie) mit einem Schmelzpunkt über 240°C erhalten wurden.

**Beispiel 73**

1-(4-Bromphenyl)-2-(dimethylaminophenylmethyl)cyclohexanol, Hydrochlorid

2,78 g (9, 8 mmol) 1-Brom-4-iodbenzol wurden in 10 ml Ether gelöst, im Eisbad (Methanol/Eis) auf -10°C gekuhlt und 5,45 ml (10,1 mmol) Isopropy]magnesiumchlorid (2 M Lösung in Tetrahydrofuran) zugetropft. Nach einer Stunde Rühren wurden bei 0°C 2,5 g (10,8 mmol) des nach Beispiel 1 hergestellten 2-(Dimethylaminophenylmethyl) cyclohexanons, gelöst in 30 ml Ether, zugetropft und 15 Stunden bei RT geruhrt. Zur Aufarbeitung wurden 40 ml gesattigte Ammoniumchloridlösung bei Eisbadkühlung zugegeben und bei RT dreimal mit je 40 ml Ether extrahiert. Die vereinigten organischen Extrakte wurden über Natriumsulfat getrocknet, filtriert und am Rotationsverdampfer (500 bis 10 mbar) eingeengt. Es wurden 4,13 g Rohbase (98,4 % der Theorie) erhalten, aus der nach Beispiel 1 (3. Stufe) mit Chlortrimethylsilan/Wasser in 2-Butanon ein Hydrochlorid gefällt wurde. Hieraus wurde mit 25 ml Wasser und 5 ml Ammoniaklösung (25 Vol.%) die Base freigesetzt, dreimal mit je 25 ml Ether extrahiert, die vereinigten organischen Extrakte über Natriumsulfat getrocknet, filtriert und am Rotationsverdampfer (500 bis 10 mbar) eingeengt. Es wurden 2,07 g Rohbase (54,4% der Theorie) erhalten, aus der man nach Beispiel 1 (3. Stufe) mit Chlortrimethysilan/Wasser in 2-Butanon 2,01 g 1-(4-Bromphenyl )-2-(dimethylaminophenylmethyl)cyclohexanol, Hydrochlorid (43,8% der Theorie) erhielt, das sich ab 165°C zersetzt.

**Beispiel 74**

2-(Dimethylaminophenylmethyl)-1-naphthalin-1-cylcyclohexanol, Hydrochlorid

0,824 g (3,24 mmol) 1-Iodnaphthalin wurden in 2 ml Ether gelöst, auf -10°C gekühlt und 1,62 ml (3,24 mmol) Isopropylmagnesiumchlorid (2 M Lösung in Tetrahydrofuran) zugetropft. Nach einer Stunde Rühren wurden bei 0°C 0,50 g (2,16 mmol) des nach Beispiel 1 hergestellten 2-(Dimethylaminophenylmethyl)cyclohexanons, gelöst in 2 ml

Ether, zugetropft und 15 Stunden bei RT gerührt. Zur Aufarbeitung wurden 2 mi gesättigte Ammoniumchloridlösung bei Eisbadkühlung zugegeben und bei RT dreimal mit je 5 ml Ether extrahiert. Die vereinigten organischen Extrakte wurden über Natriumsulfat getrocknet, filtriert und am Rotationsverdampfer (500 bis 10 mbar) eingeengt. Es wurden 1,00 g Rohbase (129% der Theorie) erhalten, aus der nach Beispiel 1 (3. Stufe) mit Chlortrimethylsilan/Wasser in 2-Butanon 0,23 g 2-(Dimethylaminophenylmethyl)-1-naphthalin-1-ylcyclohexanol, Hydrochlorid (17,9% der Theorie) mit einem Schmelzpunkt über 250°C erhalten wurden.

**Beispiel 75**

2-(Dimethylaminophenylmethyl)-1-(2-methylsulfanylphenyl)-cyclohexanol, Hydrochlorid

0,811 g (3,24 mmol) 2-Methylmercaptoiodbenzol wurden in 2 ml Ether gelöst, auf -10°C gekühlt und 1,62 ml (3,24 mmol) Isopropylmagnesiumchlorid (2 M Lösung in Tetrahydrofuran) zugetropft. Nach einer Stunde Ruhren wurden bei °C 0,50 g (2,16 mmol) des nach Beispiel 1 hergestellten 2-(Dimethylaminophenylmethyl)cyclohexanons, gelöst in 2 ml Ether, zugetropft und 15 Stunden bei RT gerührt. Zur Aufarbeitung wurden 2 ml gesattigte Ammoniumchloridlösung bei Eisbadkuhlung zugegeben und bei RT dreimal mit je 5 ml Ether extrahiert. Die vereinigten organischen Extrakte wurden über Natriumsulfat getrocknet, filtriert und am Rotationsverdampfer (500 bis 10 mbar) eingeengt. Es wurden 0,84 g Rohbase (109% der Theorie) erhalten, aus der nach Beispiel 1 (3. Stufe) mit Chlortrimethylsilan/Wasser in 2-Butanon 0,483 g 2-(Dimethylaminophenylmethyl)-1-(2-methylsulfanylphenyl)cyclohexanol, Hydrochlorid (38,0% der Theorie) erhalten wurden, das sich ab 230°C zersetzt.

**Beispiel 76**

1-Benzyl-2-(dimethylaminonaphthalin-2-ylmethyl)cyclohexanol, Hydrochlorid

1. Stufe

2-(Dimethylaminonaphthalin-2-ylmethyl)cyclohexanon

Zu 520 ml (520 mmol) Natriumiodidlösung (1 M in Acetonitril), mit einem Eisbad auf 0°C gekühlt, wurden unter Rühren 19,3 g (237 mmol) frisch getrocknetes Dimethylamin-Hydrochlorid gegeben, 66 ml (474 mmol) Triethylamin und 66 ml (521 mmol) Chlortrimethylsilan zugetropft und eine Stunde bei RT nachgerührt. Bei Eiskühlung wurden 37,0 g (237 mmol) 2-Naphthaldehyd zugegeben und noch eine Stunde bei RT weitergerührt. Es wurde erneut mit einem Eisbad auf 0°C gekühlt, 38 ml (237 mmol) 1-(Pyrrolidino)-1-cyclohexen zugegeben und zwei Stunden bei RT weitergeruhrt. Zur Aufarbeitung wurde der Ansatz bei Eiskühlung mit 350 ml halbkonzentrierter Salzsäure versetzt, 10 Minuten geruhrt, zweimal mit je 350 ml Ether gewaschen und mit t 890 ml verdünnter Ammoniaklösung (5 Vol.%) alkalisch gestellt (pH ca. 9). Es wurde dreimal mit je 350 ml Ether extrahiert, die vereinigten organischen Extrakte über Natriumsulfat getrocknet, filtriert und am Rotationsverdampfer (500 bis 10 mbar) ohne Wärmezufuhr eingeengt. Es wurden 54,7 g Rohbase (82,1% der Theorie) erhalten, aus der nach Beispiel 1 (2. Stufe) mit Chlortrimethylsilan/Wasser in 2-Butanon 50,8 g des Hydrochlorids von 2-(Dimethylaminophthalin-2-ylmethyl)cyclohexanon (67,5% der Theorie) erhalten wurden.

2. Stufe

1-Benzyl-2-(dimethylaminonaphthalin-2-ylmethyl)cyclohexanol, Hydrochlorid

Aus 3,0 g (9,44 mmol) des nach Stufe 1 erhaltenen Hydrochlorids von 2-(Dimethylaminonaphthalin-2-ylmethyl)cyclohexanon wurde mit 30 ml Wasser und 5 ml Ammoniaklösung (25 Vol.%) die Base freigesetzt, dreimal mit je 30 ml Ether extrahiert, die vereinigten organischen Extrakte uber Natriumsulfat getrocknet, filtriert und am Rotationsverdampfer ohne Wärmezufuhr (500 bis 10 mbar) eingeengt. 2,5 g (8, 9 mmol) dieser Base wurden in 15 ml Tetrahydrofuran gelöst, bei Eisbadkühlung zu 5,3 ml (10,7 mmol) Benzylmagnesiumchlorid (2 M Lösung in Tetrahydrofuran) getropft und 15 Stunden bei RT gerührt. Zur Aufarbeitung wurden 20 ml gesättigte Ammoniumchloridlösung bei Eisbadkühlung zugegeben und bei RT dreimal mit je 20 ml Ether extrahiert. Die vereinigten organischen Extrakte wurden über Natriumsulfat getrocknet, filtriert und am Rotationsverdampfer (500 bis 10 mbar) eingeengt. Es wurden 3,17 g Rohbase (99,2 % der Theorie) erhalten, aus der man nach Beispiel 1 (3. Stufe) mit Chlortrimethylsiian/Wasser in 2-Butanon 2, 4 g 1-Benzyl-2-(dimethylaminonaphthalin-2-ylmethyl)cyclohexanol, Hydrochlorid (68,1%, der Theorie) erhielt, das sich ab 180°C zersetzt.

**Beispiel 77**

1-Benzyl-2-(dimethylaminopentafluorphenylmethyl)cyclohexanol, Hydrochlorid

1. Stufe

2-(Dimethylaminopentafluorphenylmethyl)cyclohexanon

Zu 280 ml (280 mmol) Natriumiodidlösung (1 M in Acetonitril) , mit einem Eisbad auf 0°C gekuhlt, wurden unter Ruhren 10,4 g (128 mmol) frisch getrocknetes Dimethylamin-Hydrochlorid gegeben, 35,5 ml (255 mmol) Triethylamin und 35, 5 ml (280 mmol) Chlortrimethylsilan zugetropft und eine Stunde bei RT nachgerührt. Bei Eiskühlung wurden 25,0 g (128 mmol) Pentafluorbenzaldehyd zugegeben und noch eine Stunde bei RT weitergerührt. Es wurde erneut mit eineim Eisbad auf 0°C gekühlt, 20,5 ml (128 mmol) 1-(Pyrrolidino)-1-cyclohexen zugegeben und zwei Stunden bei RT weitergerührt. Zur Aufarbeitung wurde der Ansatz bei Eiskühlung mit 1.90 ml halkonzentrierter Salzsäure versetzt, 10 Minuten gerührt, zweimal mit je 190 ml Ether gewaschen und mit 480 ml verdünnter Ammoniaklösung (5 Vol.%) alkalisch gestellt (pH ca. 9) . Es wurde dreimal mit je 190 ml Ether extrahiert, die vereinigten organischen Extrakte uber Natriumsulfat getrocknet, filtriert und am Rotationsverdampfer (500 bis 10 mbar) ohne Wärmezufuhr eingeengt. Es wurden 30,2 g Rohbase (73,7% der Theorie) erhalten, aus der nach Beispiel 1 (2. Stufe) mit Chlortrimethylsilan/Wasser in 2-Butanon 14,7 g des Hydrochlorids von 2-(Dimethylaminopentafluorphenylmethyl)cyclohexanon (32,3 % der Theorie) erhalten wurden.

2. Stufe

1-Benzyl-2-(dimetlylaminopentafluorphenylmethyl)cyclohexanol, Hydrochlorid

Aus 3,0 g (8,39 mmol) des nach Stufe 1 erhaltenen Hydrochlorids von 2-(Dimethylaminopentafluorphenylmethyl)cyclohexanon wurde mit 30 ml Wasser und 5 ml Ammoniaklösung (25 Vol.%) die Base freigesetzt, dreimal mit je 30 ml Ether extrahiert, die vereinigten organischen Extrakte uber Natriumsulfat getrocknet, filtriert und am Rotationsverdampfer ohne Wärmezufuhr (500 bis 10 mbar) eingeengt. 2,5 g (7,8 mmol) dieser Base wurden in 12 ml Tetrahydrofuran gelöst, bei Eisbadkühlung zu 4,7 ml (9,3 mmol) Benzylmagnesiumchlorid (2 M Lösung in Tetrahydrofuran) getropft und 15 Stunden bei RT gerührt. Zur Aufarbeitung wurden 10 ml gesättigte Ammoniumchoridlösung bei Eisbadkühlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Es wurden 2,46 g Rohbase (76,4% der Theorie) erhalten, aus der man nach Beispiel 1 (3. Stufe) mit Chlortrimethylsilan/Wasser in 2-Butanon 0,68 g 1-Benzyl-2-(dimethylaminopentafluorphenylmethyl)cyclohexanol, Hydrochlorid (19, 4% der Theorie) erhielt, das sich ab 100°C zersetzt.

**Beispiel 78**

1-Benzyl-2-(phenylpiperidin-1-ylmethyl)cyclohexanol, Hydrochlorid

1. Stufe

2-(Phenylpiperidin-1-ylmethyl)cyclohexanon

10 g (47,7 mmol) 1-Benzylidenpiperidiniumchlorid wurden in 20 ml Dichlormethan gelöst und im Kältebad (Isopropanol/Trockeneis) auf -70°C gekühlt. Es wurden 7,21 g (47,7 mmol) 1-(Pyrrolidino)-1-cyclohexen zugegeben und 15 Stunden bei RT weitergerührt. Zur Aufarbeitung wurde der Ansatz bei Eiskühlung mit 70 ml halbkonzentrierter Salzsäure versetzt, 10 Minuten gerührt, zweimal mit je 70 ml Ether gewaschen und mit 180 ml verdünnter Ammoniaklösung (5 Vol.%) alkalisch gestellt (pH ca. 9). Es wurde dreimal mit je 70 ml Ether extrahiert, die vereinigten organischen Extrakte über Natriumsulfat getrocknet, filtriert und am Rotationsverdampfer (500 bis 10 mbar) ohne Wärmezufuhr eingeengt. Es wurden 8,73 g Rohbase (67,5% der Theorie) erhalten, aus der nach Beispiel 1 (2. Stufe) mit Chlortrimethylsilan/Wasser in 2-Butanon 6,3 g des Hydrochlorids von 2-(Phenylpiperidin-1-ylmethyl)cyclohexanon (42,9% der Theorie) erhalten wurden.

2. Stufe

1-Benzyl-2-(phenylpiperidin-1-ylmethyl)cyclohexanol, Hydrochlorid

Aus 2,5 g (8,12 mmol) des nach Stufe 1 erhaltenen Hydrochlorids von 2-(Phenylpiperidin-1-ylmethyl)cyclohexanon wurde mit 25 ml Wasser und 5 ml Ammoniaklosung (25 Vol.%) die Base freigesetzt, dreimal mit je 30 ml. Ether extrahiert, die vereinigten organischen Extrakte über Natriumsulfat getrocknet, filtriert und am Rotationsverdampfer ohne Wärmezufuhr (500 bis 10 mbar) eingeengt. 2,00g (7,4 4 mmol) dieser Base wurden in 10 ml Tetrahydrofuran qelöst, bei Eisbadkühlung zu 4,4 ml (8,8 mmol) Benzylmagnesiumchlorid (2 M Losung in Tetrahydrofuran) getropft und 15 Stunden bei RT gerührt. Zur Aufarbeitung wurden 10 ml gesättigte Ammoniumchloridlösung bei Eisbadkühlung zugegeben und bei RT dreimal mit je 15 ml Ether extrahiert. Die vereinigten organischen Extrakte wurden uber Natriumsulfat getrocknet, filtriert und am Rotationsverdampfer (500 bis 10 mbar) eingeengt. Es wurden 2,29 g Rohbase (85,4% der Theorie) erhalten, aus der man nach Beispiel 1 (3. Stufe) mit Chlortrimethylsilan/Wasser in 2-Butanon 1,05 g 1-Benzyl-2-(phenylpiperidin-1-ylmethyl)cyclohexanol, Hydrochlorid (35,7% der Theorie) erhielt, das sich ab 218°C zersetzt.

**Beispiel 79**

2-(Dimethylaminophenylamethyl)-1-(4-trifluormethylphenyl)-cyclohexanol, Hydrochlorid

0,76 g (31,0 mmol) Magnesiumspäne wurden in 15 ml Ether p.a. gerührt. Man tropfte 4,46 ml (31,0 mmol) 4-Brombenzotrifluorid, gelöst in 15 ml Ether, so zu, daß das Reaktionsgemisch leicht siedete. Nach beendeter Zugabe wurde eine Stunde bei RT nachgerührt. 6,0 g (26,0 mmol) des nach Beispiel 1 hergestellten 2-(Dimethylaminophenylmethyl)-cyclohexanons wurden in 15 ml Ether gelöst, bei Eisbadkühlung zum Grignard-Ansatz getropft und 15 Stunden bei RT gerührt. Zur Aufarbeitung wurden 30 ml gesättigte Aminoniumchloridlösung bei Eisbadkuhlung zugegeben und bei RT dreimal mit je 50 ml Ether extrahiert. Die vereinigten organischen Extrakte wurden über Natriumsulfat getrocknet, filtriert und am Rotationsverdampfer (500 bis 10 mbar) eingeengt. Es wurden 8,49 g Rohbase (86,7% der Theorie) erhalten, aus der man nach Beispiel 1 (3. Stufe) mit Chlortrimethylsilan/Wasser in 2-Butanon 6,62 g 2-(Dimethylaminophenylmethyl)-1-(4-trifluormethylphenyl)cyclohexanol, Hydrochlorid (61,7% der Theorie) erhielt, das sich ab 170°C zersetzt.

**Beispiel 80**

3-(4-tert.-Butylbenzyl)-1-dimethylamino-2-methyl-1-phenylpentan-3-ol, Hydrochlorid

0,33 g (13,7 mmol) Magnesiumspäne wurden in 10 ml Ether p.a. gerührt. Man tropfte 2,50 g (13,7 mmol) 4-tert.-Butylbenzylchlorid, gelöst in 10 ml Ether, so zu, daß das Reaktionsgemisch leicht siedete. Nach beendeter Zugabe wurde eine Stunde bei RT nachgerührt. 2,60 g (11,2 mmol) des nach Beispiel 59 hergestellten 1-Dimethylamino-2-methyl-1-phenylpentan-3-ons wurden in 10 ml Ether gelost, bei Eisbadkühlung zum Grignard-Ansatz getropft und 15 Stunden bei RT gerührt. Zur Aufarbeitung wurden 20 ml gesättigte Ammoniumchloridlösung bei Eisbadkühlung zugegeben und bei RT dreimal mit je 20 ml Ether extrahiert. Die vereinigten organischen Extrakte wurden über Natriumsulfat getrocknet, filtriert und am Rotationsverdampfer (500 bis 10 mbar) eingeengt. Es wurden 3,93 g Rohbase (93,8% der Theorie) erhalten, die auf eine 3,5 x 30 ein Säule, gefullt mit Kieselgel, gegeben wurde, Die Elution mit Essigsäureethylester/Hexan 1:4 ergab 0,59 g Base, aus der nach Beispiel 1 (3. Stufe) mit Chlortrimethylsilan/Wasser in 2-Butanon 0,26 g 3-(4-tert .-Butylbenzyl)-1-dimethylamino-2-methyl-1-phenylpentan-3-ol, Hydrochlorid (5, 6% der Theorie) erhalten wurden, das sich ab 91°C zersetzt.

**Beispiel 81**

2-(Dimethylamino-o- tolylmethyl)-1-phenethylcyclohexanol, Hydrochlorid

2,50 g (9,3 mmol) des nach Beispiel 66 hergestellten 2-(Dimethylamino-*o*-tolylmethyl)cyclohexanons wurden in 10 ml Tetrahydrofuran gelöst, bei Eisbadkuhlung zu 12,2 ml (12, 2 mmol) Phenethylmagnesiumchlorid (1 M Lösung in Tetrahydrofuran) getropft und 15 Stunden bei RT gerührt. Zur Aufarbeitung wurden 15 ml gesättigte Ammoniumchloridlösung bei Eisbadkühlung zugegeben und bei RT dreimal mit je 15 ml Ether extrahiert. Die vereinigten organischen Extrakte wurden über Natriumsulfat getrocknet, filtriert und am Rotationsverdampfer (500 bis 10 mbar) eingeengt. Man erhielt 3,32 g Rohbase (92,7% der Theorie), aus der nach Beispiel 1 (3. Stufe) mit Chlortrimethylsian/Wasser in 2-Butanon 2, 09 g 2-(Dimethylamino-o-tolylmethyl)-1-phenethylcyclohexanol, Hydrochlorid (52, 9% der Theorie) mit

einem Schmelzpunkt von 187°C erhalten wurden.

**Beispiel 82**

1-(4-*tert*.-Butylbenzyl)-2-[dimethylaminothiophen-2-yl-methyl]cyclohexanol, Hydrochlorid

0,31 g (12,6 mmol) Magnesiumspäne wurden in 10 ml Ether p.a. geruhrt. Es wurden 2,30 g (12,6 mmol) 4-tert. -Butylbenzylbromid, gelöst in 10 ml Ether, so zugetropft, daß das Reaktionsgemisch leicht siedete. Nach beendeter Zugabe wurde eine Stunde bei RT nachgerührt. 2,50 g (10,5 mmol) des nach Beispiel 70 hergestellten 2-(Dimethylaminothiophen-2-ylmethyl)cyclohexanons wurden in 10 ml Ether gelöst, bei Eisbadkühlung zum Grignard-Ansatz getropft und 15 Stunden bei RT gerührt. Zur Aufarbeitung wurden 15 ml gesättigte Ammoniumchloridlösung bei Eisbadkühlung zugegeben und bei RT dreimal mit je 20 ml Ether extrahiert. Die vereinigten organischen Extrakte wurden über Natriumsulfat getrocknet, filtriert und am Rotationsverdampfer (500 bis 10 mbar) eingeengt. Es wurden 3,69 g Rohbase (90,7% der Theorie) erhalten, aus der man nach Beispiel 1 (3. Stufe) mit Chlortrimethylsilan/Wasser in 2-Butanon 1,16 g 1-(4-*tert*.-Butylbenzyl)-2-[dimethylaminothiophen-2-ylmethyl]-cyclohexanol, Hydrochlorid (26,2% der Theorie) erhielt, das sich ab 210°C zersetzt.

## Patentansprüche

1. Substituierte 3-Amino-3-arylpropan-1-ole der allgemeinen Formel I,

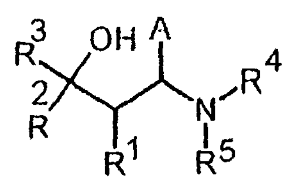

I

worin

$R^1$, $R^2$, jeweils unabhängig voneinander, $C_{1-6}$-Alkyl oder $R^1$ und $R^2$ zusammen einen $(CH_2)_{2-6}$-Ring bilden, der auch phenylsubstituiert sein kann,

$R^3$ $C_{3-6}$-Alkyl, $C_{3-6}$-Cycloalkyl, Aryl mit gegebenenfalls Heteroatomen im Ringsystem und den Substituenten $R^6$ bis $R^8$ am Arylring, oder ein substitiertes $C_{1-3}$-Alkylphenyl der Formel XII,

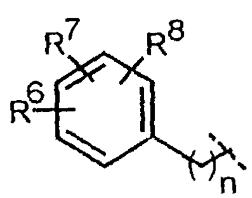

n = 1, 2 oder 3

XII,

$R^4$, $R^5$, jeweils unabhängig voneinander, $C_{1-6}$-Alkyl, $C_{3-6}$-Cycloalkyl, Phenyl, Benzyl, Phenethyl oder $R^4$ und $R^5$ zusammen einen $(CH_2)_{3-6}$- oder $CH_2CH_2OCH_2CH_2$-Ring bilden,

$R^6$ bis $R^8$, jeweils unabhängig voneinander, H, F, Cl , Br, $CHF_2$, $CF_3$, OH, $OCF_3$, $OR^{14}$, $NR^{15}R^{16}$, $SR^{14}$, Phenyl, $SO_2CH_3$, $SO_2CF_3$, $C_{1-6}$-Alkyl, CN, $COOR^{14}$, $CONR^{15}R^{16}$ oder $R^6$ und $R^7$ zusammen einen $OCH_2O$, $OCH_2CH_2O$, CH=CHO, $CH=C(CH_3)O$ oder $(CH_2)_4$-Ring bilden, worin

$R^{14}$ $C_{1-6}$-Alkyl, Phenyl, Benzyl, Phenethyl und

$R^{15}$, $R^{16}$, jeweils unabhängig voneinander, H, $C_{1-6}$- Alkyl, Phenyl, Benzyl, Phenethyl, und

A ein Arylrest, der gegebenenfalls Heteroatome im Ringsystem aufweist und/oder substituiert sein kann,

bedeuten,
und deren Diastereomere oder Enantiomere in Form ihrer Basen oder Salze physiologisch verträglicher Sauren, wobei 1-Benzyl-2-(dimethylaminophenylmethyl)-cyclohexanol, dessen Diastereomere und dessen Enantiomere in Form ihrer Basen und dessen Umsetzungsprodukt mit Methyljodid ausgenommen sind.

**2.** Verbindungen gemäß Anspruch 1 **dadurch gekennzeichnet, daß** $R^1$ und $R^2$ zusammen einen $(CH_2)_{2-6}$-Ring bilden, der phenylsubstituiert sein kann, $R^3$ bis $R^5$ und A die Bedeutung gemäß Anspruch 1 besitzen.

**3.** Verbindungen gemäß Anspruch 1 **dadurch gekennzeichnet, daß** $R^1$ und $R^2$ zusammen einen $(CH_2)_4$-Ring bilden, der phenylsubstituiert sein kann, $R^3$ bis $R^5$ und A die Bedeutung gemäß Anspruch 1 besitzen.

**4.** Verbindungen gemäß Anspruch 1 **dadurch gekennzeichnet, daß** $R^3$ ein substitiertes $C_{1-3}$-Alkylphenyl der Formel XII darstellt, $R^1$, $R^2$, $R^4$ bis $R^5$ und A die Bedeutung gemäß Anspruch 1 besitzen.

**5.** Verbindungen gemäß Anspruch 1 **dadurch gekennzeichnet, daß** $R^3$ einen Arylrest mit gegebenenfalls Heteroatomen im Ringsystem und den Substituenten $R^6$ bis $R^8$ am Arylring darstellt, $R^1$, $R^2$, $R^4$ bis $R^5$ und A die Bedeutung gemäß Anspruch 1 besitzen.

**6.** Verbindungen gemäß Anspruch 1 **dadurch gekennzeichnet, daß** A einen Rest aus der Gruppe von substituiertem Phenyl der Formel XI

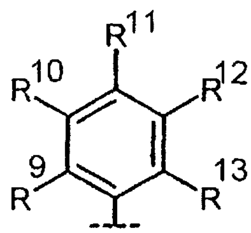

XI,

worin

$R^9$ bis $R^{13}$, jeweils unabhängig voneinander, H, F, Cl, Br, I, $CF_3$, OH, $OR^{14}$, $OCF_3$, $SR^{14}$, $SO_2CH_3$, $SO_2CF_3$, $C_{1-6}$-Alkyl, Phenyl, CN, $COOR^{14}$, $NO_2$ oder $R^9$ und $R^{10}$ oder $R^{10}$ und $R^{11}$ zusammen einen $OCH_2O$- oder $OCH_2CH_2O$-Ring bilden,

$R^{14}$ $C_{1-6}$-Alkyl, Phenyl, Benzyl, Phenethyl

oder A einen unsubstituierten oder substituierten Thiophen-Rest oder einen unsubstituierten oder substituierten Furan-Rest bedeutet und $R^1$ bis $R^5$ die Bedeutung gemäß Anspruch 1 besitzen.

**7.** Verbindungen gemäß Anspruch 1 **dadurch gekennzeichnet, daß** $R^1$ und $R^2$ zusammen einen $(CH_2)_{2-6}$-Ring bilden, der phenylsubstituiert sein kann, $R^3$ ein substitiertes $C_{1-3}$-Alkylphenyl der Formel XII darstellt, $R^4$ bis $R^5$ und A die Bedeutung gemäß Anspruch 1 besitzen.

**8.** Verbindungen gemäß Anspruch 1 **dadurch gekennzeichnet, daß** $R^1$ und $R^2$ zusammen einen $(CH_2)_{2-6}$-Ring bilden, der phenylsubstituiert sein kann, $R^3$ einen Arylrest mit gegebenenfalls Heteroatomen im Ringsystem und den Substituenten $R^6$ bis $R^8$ am Arylring darstellt, $R^4$ bis $R^5$ und A die Bedeutung gemaß Anspruch 1 besitzen.

**9.** Verbindungen gemäß Anspruch 1 **dadurch gekennzeichnet, daß** $R^1$ und $R^2$ zusammen einen $(CH_2)_4$-Ring bilden,

der phenylsubstituiert sein kann, A einen Rest aus der Gruppe von substituiertem Phenyl der Formel XI oder einen unsubstituierten oder substituierten Thiophen-Rest oder einen unsubstituierten oder substituierten Furan-Rest, $R^3$ ein substitiertes $C_{1-3}$-Alkylphenyl der Formel XII darstellt, $R^4$ bis $R^5$ die Bedeutung gemäß Anspruch 1 besitzen.

**10.** Verbindungen gemäß Anspruch 1 **dadurch gekennzeichnet, daß** $R^1$ und $R^2$ zusammen einen $(CH_2)_4$-Ring bilden, der phenylsubstituiert sein kann, A einen Rest aus der Gruppe von substituiertem Phenyl der Formel XI oder einen unsubstituierten oder substituierten Thiophen-Rest oder einen unsubstituierten oder substituierten Furan-Rest, $R^3$ einen Arylrest mit gegebenenfalls Heteroatomen im Ringsystem und den Substituenten $R^6$ bis $R^8$ am Arylring darstellt, $R^4$ bis $R^5$ die Bedeutung gemäß Anspruch 1 besitzen.

**11.** Verbindungen gemäß Anspruch 1 **dadurch gekennzeichnet, daß** $R^1$ und $R^2$ zusammen einen $(CH_2)_4$-Ring bilden, A einen Rest aus der Gruppe von substituiertem Phenyl der Formel XI oder einen unsubstituierten oder substituierten Thiophen-Rest, $R^3$ ein substitiertes $C_{1-3}$-Alkylphenyl der Formel XII darstellt, $R^4$ bis $R^5$ die Bedeutung gemäß der Definition des Anspruchs 1 besitzen.

**12.** Verbindungen gemäß Anspruch 1 **dadurch gekennzeichnet, daß** $R^1$ und $R^2$ zusammen einen $(CH_2)_4$ -Ring biden, A einen Rest aus der Gruppe von substituiertem Phenyl der Formel XI oder einen unsubstituierten oder substituierten Thiophen-Rest, $R^3$ einen Arylrest mit gegebenenfalls Heteroatomen im Ringsystem und den Substituenten $R^6$ bis $R^8$ am Arylring darstellt, $R^4$ bis $R^5$ die Bedeutung gemäß der Definition des Anspruchs 1 besitzen.

**13.** Verbindungen gemäß Anspruch 1 **dadurch gekennzeichnet, daß** $R^1$ und $R^2$ zusammen einen $(CH_2)_4$-Ring bilden, A einen unsubstituierten oder substituierten Thiophen-Rest, $R^3$ ein substitiertes $C_{1-3}$-Alkylphenyl der Formel XII darstellt, $R^4$ bis $R^5$ die Bedeutung gemäß der Definition des Anspruchs 1 besitzen.

**14.** Verbindungen gemäß Anspruch 1 **dadurch gekennzeichnet, daß** $R^1$ und $R^2$ zusammen einen $(CH_2)_4$-Ring bilden, A einen unsubstituierten oder substituierten Thiophen-Rest, $R^3$ einen Arylrest mit gegebenenfalls Heteroatomen im Ringsystem und den Substituenten $R^6$ bis $R^8$ am Arylring darstellt, $R^4$ bis $R^5$ die Bedeutung gemäß der Definition des Anspruchs 1 besitzen.

**15.** Verbindungen gemäß Anspruch 1 **dadurch gekennzeichnet, daß** $R^1$ und $R^2$ zusammen einen $(CH_2)_4$-Ring bilden, A einen unsubstituierten oder substituierten Furan-Rest, $R^3$ ein substitiertes $C_{1-3}$-Alkylphenyl der Formel XII darstellt, $R^4$ bis $R^5$ die Bedeutung gemäß der Definition des Anspruchs 1 besitzen.

**16.** Verbindungen gemäß Anspruch 1 **dadurch gekennzeichnet, daß** $R^1$ und $R^2$ zusammen einen $(CH_2)_4$-Ring bilden, A einen unsubstituierten oder substituierten Furan-Rest, $R^3$ einen Arylrest mit gegebenenfalls Heteroatomen im Ringsystem und den Substituenten $R^6$ bis $R^8$ am Arylring darstellt, $R^4$ bis $R^5$ die Bedeutung gemäß der Definition des Anspruchs 1 besitzen.

**17.** Verbindungen gemäß Anspruch 1 ;

2-(Dimethylaminophenylmethyl-1-(3-methoxyphenyl)cyclohexanol und das entsprechende Hydrochlorid

2-(Dimethylaminophenylmethyl)-1-(3-fluorphenyl)cyclohexanol und das entsprechende Hydrochlorid

2-(Dimethylaminophenylmethyl)-1-phenylcyclohexanol und das entsprechende Hydrochlorid

3-[2-(Dimethylaminoplenylmethyl)-1-hydroxycyclohexyl]-phenol und das entsprechende Hydrochlorid

2-(Dimethylaminophenylmethyl)-1- (4-methoxyphenyl)cyclohexanol und das entsprechende Hydrochlorid

1-(4-Chlorphenyl)-2-(dimethylaminophenylmethyl)cyclohexanol und das entsprechende Hydrochlorid

2-(Dimethylaminophenylmethyl)-1-(4-fluorphenyl)cyclohexanol und das entsprechende Hydrochlorid

2-(Dimethylaminophenylmethyl)-1-p-tolylcyclohexanol und das entsprechende Hydrochlorid

1-(3-Chlorphenyl)-2-dimethylamino-(3-methoxyphenyl)-methyl]cyclohexanol und das entsprechende Hydrochlorid

1-(4-Dimethylaminophenyl-2-[dimethylaminophenylmethyl]cyclolexanol und das entsprechende Hydrochlorid

1-Benzo[1,3] dioxol-4-yl-2-(dimethylaminophenylmethyl)-cyclohexanol und das entsprechende Hydrochlorid

1-(3,4-Dimethoxyphenyl)-2-(dimethylaminophenylmethyl)-cyclohexanol und das entsprechende Hydrochlorid

2-(Dimethylaminophenylmethyl-1-(3-methoxybenzyl)cyclohexanol und das entsprechende Hydrochlorid

1-Benzyl-2-(dimethylaminophenylmethyl)cyclohexanol, Hydrochlorid

2-(Dimethylaminophenylmethyl)-1-(4-fluor-3-trifluormethylphenyl)cyclohexanol und das entsprechende Hydrochlorid

2-(Dimethylaminophenylmethyl)-1-(4-trifluormethoxybenzyl)cyclohexanol und das entsprechende Hydrochlorid

2-(Dimethylaminophenylmethyl)-1-furan-3-ylcyclohexanol und das entsprechende Hydrochlorid

1-Butyl-2-(dimethylaminophenylmethyl)cyclohexanol und das entsprechende Hydrochlorid

1-(3,4-Dichlorphenyl)-2-(dimethylaminophenylmethyl)-cyclohexanol und das entsprechende Hydrochlorid

(+)-1-(3, 4-Dichlorphenyl)-2-[dimethylaminophenylmethyl)cyclohexanol und das entsprechende Hydrochlorid

(-)-1-(3,4-Dichlorphenyl)-2-(dimethylaminophenylmethyl)cyclohexanol und das entsprechende Hydrochlorid

4-[2-(Dimethylaminophenylmethyl)-1-hydroxycyclohexyl]-phenol und das entsprechende Hydrochlorid

2-(Dimethylaminophenymethyl)-1-naphthalin-2-ylcyclohexanol und das entsprechende Hydrochlorid

2-[Dimethylamino-(4-trifluormethylphenyl)methyl]-1-(3-methoxybenzyl)cyclohexanol und das entsprechende Hydrochlorid

1-(4-Chlorbenzyl )-2-(dimethylaminophenylmethyl)-1-cyclohexanol und das entsprechende Hydrochlorid

2-(Dimethylaminophenylmethyl)-1-(2-fluorbenzyl)cyclohexanol und das entsprechende Hydrochlorid

2-(Dimethylaminophenylmethyl)-1-(4-fluorbenzyl)cyclohexanol und das entsprechende Hydrochlorid

1-(2,5-Dimethoxyphenyl)-2-(dimethylaminophenylmethyl)-cyclohexanol und das entsprechende Hydrochlorid

1-(2-Chlor-4-fluorbenzyl)-2-(dimethylaminophenylmethyl)cyclohexanol und das entsprechende Hydrochlorid

1-(4-tert.-Butylbenzyl)-2-(dimethylaminophenylmethyl)-cyclohexanol und das entsprechende Hydrochlorid

2-(Dimethylaminophenylmethyl)-1-(3-fluorbenzyl)cyclohexanol und das entsprechende Hydrochlorid

1-(2-Chlorbenzyl)-2-(dimethylaminophenylmethyl)cyclohexanol und das entsprechende Hydrochlorid

1-Benzo[1,3] dioxol-5-yl-2-[dimethylamino-(3-methoxyphenyl) methyl] cyclohexanol und das entsprechende Hydrochlorid

1-(3-Chlorbenzyl)-2-(dimethylaminophenylmethyl)cyclohexanol und das entsprechende Hydrochlorid

1-(2,4-Dichlorbenzyl)-2-(dimethylaminophenylmethyl-cyclohexanol und das entsprechende Hydrochlorid

1-Benzyl-2-[dimethylaminophenyl-(3-phenoxyphenyl)-methyl]cyclohexanol und das entsprechende Hydrochlorid

1-Benzyl-2-[dimethylaminophenyl-(3-methoxyphenyl)-methyl]cyclohexanol und das entsprechende Hydrochlorid

2-(Dimethylaminophenylmethyl)-1-(3-trifluormethylbenzyl)cyclohexanol und das entsprechende Hydrochlorid

2-[Dimethylamino-(3-methoxyphenyl)methyl]-1-(3-methoxybenzyl)cyclohexanol und das entsprechende Hydrochlorid

2-[(2-Chlorphenyl)dimethylaminomethyl]-1-naphthalin-2-ylcyclohexanol und das entsprechende Hydrochlorid

1-Benzyl-2-[(3,4-dichlorphenyl)dimethylaminomethyl]-cyclohexanol und das entsprechende Hydrochlorid

2-[(3,4-Dichlorphenyl)dimethylaminomethyl]-1-phenethylcyclohexanol und das entsprechende Hydrochlorid

1-Benzyl-2-[dimethylamino-(4-fluorphenyl)methyl]cyclohexanol und das entsprechende Hydrochlorid

2-[(3-Chlorphenyl)dimethylaminomethyl]-1-phenylcyclohexanol und das entsprechende Hydrochlorid

1-(2,4-Dichlorphenyl)-2-(3-dimethylaminophenylmethyl)-1-cyclohexanol und das entsprechende Hydrochlorid

1-Benzyl-2-[(3-Chlorphenyl)dimethylaminomethyl]cyclohexanol und das entsprechende Hydrochlorid

1-Benzyl-2-[(2-chlorphenyl)dimethylaminomethyl]cyclohexanol und das entsprechende Hydrochlorid

1-(4-tert.-Butylbenzyl)-2-[(3,4-dichlorphenyl) dimethylaminomethyl] cyclohexanol und das entsprechende Hydrochlorid

2-[Dimethylamino-(4-fluorphenyl)methyl ]-1-(3-trifluor-methylbenzyl)cyclohexanol und das entsprechende Hydrochlorid

2-(Dimethylaminophenylmethyl)bicyclohexyl-1-ol und das entsprechende Hydrochlorid

2-(Dimethylaminophenylmethyl)-1-(4-methoxybenzyl)cyclohexanol und das entsprechende Hydrochlorid

1-(2,4-Difluorbenzyl)-2-(dimethylaminophenylmethyl)-cyclohexanol und das entsprechende Hydrochlorid

1-(4-tert.-Butylbenzyl)-2-[(3-chlorphenyl)dimethylaminomethyl]cyclohexanol und das entsprechende Hydrochlorid

2-[Dimethylamino-(3-phenoxyphenylmethyl]-1-phenethylcyclohexanol und das entsprechende Hydrochlorid

2-(Dimethylamino-(3-phenoxyphenyl)methyl]-1-(3-trifluormethylbenzyl)cyclohexanol und das entstprechende Hydrochlorid

1-(2,5-Difluorbenzyl)-2-(dimethylaminophenylmethyl)-cyclohexanol und das entsprechende Hydrochlorid

1-(3,4-Difluorbenzyl)-2-(dimethylaminophenylmethyl)-cyclohexanol und das entsprechende hydrochlorid

1-(2-Chlor-6-fluorbenzyl)-2-(dimethylaminophenylmethyl)cyclohexanol und das entsprechende Hydrochlorid

1-(2,3-Difluorbenzyl)-2-(dimethylaminophenylmethyl)-cyclohexanol und das entsprechende Hydrochlorid

1-Benzyl-2-[(4-chlorphenyl)dimethylaminomethyl]cyclohexanol und das entsprechende Hydrochlorid

1-Dimethylamino-3-ethyl-2-methyl-1,5-diphenylpentan-3-ol und das entsprechende Hydrochlorid

1-(2-Chlorbenzyl)-2-[(2-chlorphenyl)-dimethylaminomethyl]cyclohexanol und das entsprechende Hydrochlorid

1-Benzyl-2-[(4-bromphenyl)dimethylaminomethyl]cyclohexanol und das entsprechende Hydrochlorid

2-[(4-Chlorphenyl)dimethylaminomethyl]-1-(4-trifluormethylphenyl)cyclohexanol und das entsprechende Hydrochlorid

2-[(4-Chlorphenyl)dimethylaminomethyl]-1-(3-trifluormethylbenzyl)cyclohexanol und das entsprechende Hydrochlorid

1-(4-tert.-Butylbenzyl)-2-[dimethylamino-(3-phenoxyphenyl)methyl]cyclohexanol und das entsprechende Hydrochlorid

4-{Dimethylamino-[2-hydroxy-2-(4-trifluormethylphenyl)-cyclohexyl]methyl}benzonitril und das entsprechende Hydrochlorid

2-[Dimethylamino-o-tolylmethyl]-1-phenylcyclohexanol und das entsprechende Hydrochlorid

1-Benzyl-2-(dimethylamino-o-tolylmethyl)cyclohexanol und das entsprechende Hydrochlorid

2-(Dimethylaminophenylmethyl)-1-(3-phenylpropyl)cyclohexanol und das entsprechende Hydrochlorid

2-[(2-chlorphenyl)dimethylaminomethyl]-1-[2-(4-fluorphenyl)ethyl]cyclohexanol und das entsprechende Hydrochlorid

2-[Dimethylaminothiophen-2-ylmethyl]-1-(3-trifluormethylbenzyl)cyclohexanol und das entsprechende Hydrochlorid

Methyl-4-[2-(dimethylaminophenylmethyl)-1-hydroxycyclohexyl]benzoat und das entsprechende Hydrochlorid

1-Benzyl-2-(dimethylaminophenylmethyl)-4-phenylcyclohexanol und das entsprechende Hydrochlorid

1-(4-Bromphenyl)-2-(dimethylaminophenylmethyl)cyclohexanol und das entsprechende Hydrochlorid

2-(Dimethylaminophenylmethyl)-1-naphthalin-1-ylcyclohexanol und das entsprechende Hydrochlorid

2-(Dimethylaminophenylmethyl)-1-(2-methylsulfanylphenyl)cyclohexanol und das entsprechende Hydrochlorid

1-Benzyl-2-(dimethylaminonaphthalin-2-ylmethyl)cyclohexanol und das entsprechende Hydrochlorid

1-Benzyl-2-(dimethylaminopentafluorphenylmethyl)cyclohexanol und das entsprechende Hydrochlorid

1-Benzyl-2-(phenylpiperidin-1-ylmethyl)cyclohexanol und das entsprechende Hydrochlorid

2-(Dimethylaminophenyl)-1-4-trifluormethylphenyl)cyclohexanol und das entsprechende Hydrochlorid

3-(4-tert.-Butylbenzyl)-1-dimethylamino-2-methyl-1-phenylpentan-3-ol und das entsprechende Hydrochlorid

2-(Dimethylamino-o-tolylmethyl)-1-phenethylcyclohexanol und das entsprechende Hydrochlorid

1-4-*tert.*-Butylbenzyl)-2- [dimethylaminothiophen-2-ylmethyl]cyclohexanol und das entsprechende Hydrochlorid

**18.** Arzneimittel enthaltend als Wirkstoff wenigstens eine Verbindung gemäß den Ansprüchen 1 bis 17 einschließlich der gemaß Anspruch 1 ausgenommenen Verbindungen.

**19.** Arzneimittel enthaltend als Wirkstoffe ein Gemisch der Enantiomeren einer Verbindung gemaß den Anspruchen 1 bis 17 einschließlich der gemäß Anspruch 1 ausgenommenen Verbindungen, wobei die beiden Enantiomeren

nicht in äquimolaren Mengen vorliegen, und gegebenenfalls weitere Wirkstoffe.

**20.** Arzneimittel nach Anspruch 19, **dadurch gekennzeichnet, daß** eines der Enantiomere einen relativen Anteil zwischen 5 und 45 Massenprozent am Enantiomerengemisch hat.

**21.** Verfahren zur Herstellung einer Verbindung gemäß den Anspuchen 1 bis 17 **dadurch gekennzeichnet, daß** eine Mannich-Base der allgemeinen Formel II

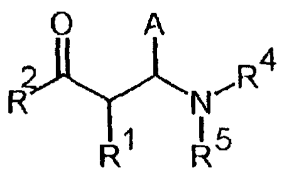

II

worin $R^1$ bis $R^5$ und A die Bedeutung gemaß der allgemeinen Formel I besitzen, mit einer metallorganischen Verbindungen $R^3Y$, worin Y MgCl, MgBr, MgI oder Li und $R^3$ gemäß Formel I bedeutet, zu einem Alkohol der allgemeinen Formel I umgesetzt wird

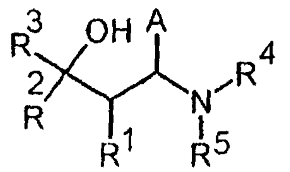

I

worin $R^1$ bis $R^5$ und A die Bedeutung gemäß Formel I haben.

**22.** Verwendung wenigstens einer Verbindung nach den Ansprüchen 1 bis 17 einschließlich der gemäß Anspruch 1 ausgenommenen Verbindungen zur Herstellung eines Arzneimittels zur Schmerzbekämpfung.

**23.** Verwendung nach Anspruch 22 zur Herstellung eines Arzneimittels zur Bekämpfung neuropathischer Schmerzen.

**24.** Verwendung nach Anspruch 22 zur Herstellung eines Arzneimittels zur Bekämpfung chronischer Schmerzen.

**25.** Verwendung wenigstens einer Verbindung nach den Ansprüchen 1 bis 17 einschließlich der gemäß Anspruch 1 ausgenommenen Verbindungen zur Herstellung eines Arzneimittels mit lokalanästetischer Wirkung.

**26.** Verwendung wenigstens einer Verbindung nach den Anspruchen 1 bis 17 einschließlich der gemäß Anspruch 1 ausgenommenen Verbindungen zur Herstellung eines Arzneimittels mit antiarrhythmischer Wirkung.

**27.** Verwendung wenigstens einer Verbindung nach den Ansprüchen 1 bis 17 einschließlich der gemäß Anspruch 1 ausgenommenen Verbindungen zur Herstellung eines Arzneimittels mit antiemetischer Wirkung.

**28.** Verwendung wenigstens einer Verbindung nach den Anspruchen 1 bis 17 einschließlich der gemäß Anspruch 1 ausgenommenen Verbindungen zur Herstellung eines Arzneimittels mit nootropischer (neurotropischer) Wirkung.

**29.** Verwendung wenigstens einer Verbindung nach den Ansprüchen 1 bis 17 einschließlich der gemäß Anspruch 1 ausgenommenen Verbindungen zur Herstellung eines Arzneimittels zur Behandlung von cardiovaskularen Erkrankungen.

**30.** Verwendung wenigstens einer Verbindung nach den Anspruchen 1 bis 17 einschließlich der gemäß Anspruch 1 ausgenommenen Verbindungen zur Herstellung eines Arzneimittels zur Behandlung von Harninkontinenz.

**31.** Verwendung wenigstens einer Verbindung nach den Ansprüchen 1 bis 17 einschließlich der gemäß Anspruch 1 ausgenommenen Verbindungen zur Herstellung eines Arzneimittels zur Behandlung von Diarrhöe.

**32.** Verwendung wenigstens einer Verbindung nach den Ansprachen 1 bis 17 einschließlich der gemäß Anspruch 1 ausgenommenen Verbindungen zur Herstellung eines Arzneimittels zur Behandlung von Pruritus.

**33.** Verwendung wenigstens einer Verbindung nach den Ansprüchen 1 bis 17 einschließlich der gemäß Anspruch 1 ausgenommenen Verbindungen zur Herstellung eines Arzneimittels zur Behandlung von Alkoholabhängigkeit.

**34.** Verwendung wenigstens einer Verbindung nach den Ansprüchen 1 bis 17 einschließlich der gemäß Anspruch 1 ausgenommenen Verbindungen zur Herstellung eines Arzneimittels zur Behandlung von Drogenabhangigkeit.

**35.** Verwendung wenigstens einer Verbindung nach den Ansprüchen 1 bis 17 einschließlich der gemäß Anspruch 1 ausgenommenen Verbindungen zur Herstellung eines Arzneimittels zur Behandlung von Medikamentenabhängigkeit.

**36.** Verwendung wenigstens einer Verbindung nach den Ansprüchen 1 bis 17 einschließlich der gemäß Anspruch 1 ausgenommenen Verbindungen zur Herstellung eines Arzneimittels zur Behandlung von Entzundungen.

**37.** Verwendung wenigstens einer Verbindung nach den Ansprüchen 1 bis 17 einschließlich der gemäß Anspruch 1 ausgenommenen Verbindungen zur Herstellung eines Arzneimittels zur Behandlung von Depressionen.

**38.** Verwendung wenigstens einer Verbindung nach den Ansprüchen 1 bis 17 einschließlich der gemäß Anspruch 1 ausgenommenen Verbindungen zur Herstellung eines Arzneimittels zur Libidosteigerung.

**39.** Verwendung wenigstens einer Verbindung nach den Ansprüchen 1 bis 17 einschließlich der gemäß Anspruch 1 ausgenommenen Verbindungen zur Herstellung eines Arzneimittels zur Vigilanzsteigerung.

## Claims

**1.** Substituted 3-amino-3-arylpropan-1-ols of the general formula I,

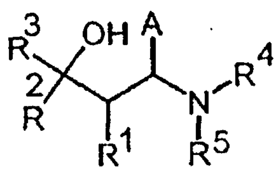

I

wherein

$R^1$, $R^2$ denote, in each case independently of one another, $C_{1-6}$ alkyl or $R^1$ and $R^2$ together form a $(OH_2)_{2-6}$ ring that may also be substituted by phenyl,

$R^3$ denotes $C_{3-6}$ alkyl, $C_{3-6}$ cycloalkyl, aryl with optionally heteroatoms in the ring system and the substituents $R^6$ to $R^8$ on the aryl ring, or a substituted $C_{1-3}$ alkylphenyl of the formula XII,

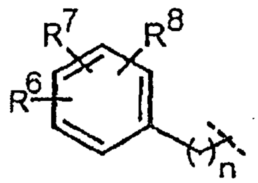

n = 1, 2 or 3

XII,

$R^4$, $R^5$ denote, in each case independently of one another, $C_{1-6}$ alkyl, $C_{3-6}$ cycloalkyl, phenyl, benzyl, phenethyl or $R^4$ and $R^5$ together form a $(CH_2)_{3-6}$ ring or $CH_2CH_2OCH_2CH_2$ ring,

$R^6$ to $R^8$ denote, in each case independently of one another, H, F, Cl, Br, $CHF_2$, $CF_3$, OH, $OCF_3$, $OR^{14}$, $NR^{15}R^{16}$, $SR^{14}$, phenyl, $SO_2CH_3$, $SO_2CF_3$, $C_{1-6}$ alkyl, CN, $COOR^{14}$, $CONR^{15}R^{16}$ or $R^6$ and $R^7$ together form a $OCH_2O$, $OCH_2CH_2O$, CH=CHO, $CH=C(CH_3)O$ or $(CH_2)_4$ ring, wherein

$R^{14}$ denotes $C_{1-6}$ alkyl, phenyl, benzyl, phenethyl, and

$R^{15}$, $R^{16}$ denote, in each case independently of one another, H, $C_{1-6}$ alkyl, phenyl, benzyl, phenethyl, and

A denotes an aryl radical that optionally contains heteroatoms in the ring system, and/or may be substituted,

and their diastereomers or enantiomers in the form of their bases or salts of physiologically compatible acids, wherein 1-benzyl-2-(dimethylaminophenylmethyl)cyclohexanol, its diastereomers and its enantiomers in the form of its bases and its reaction product with methyl iodide are excluded.

2. Compounds according to claim 1, **characterised in that** $R^1$ and $R^2$ together form a $(CH_2)_{2-6}$ ring that may be substituted by phenyl, and $R^3$ to $R^5$ and A have the meanings according to claim 1.

3. Compounds according to claim 1, **characterised in that** $R^1$ and $R^2$ together form a $(CH_2)_4$ ring that may be substituted by phenyl, and $R^3$ to $R^5$ and A have the meanings according to claim 1.

4. Compounds according to claim 1, **characterised in that** $R^3$ denotes a substituted $C_{1-3}$-alkylphenyl of the formula XII, and $R^1$, $R^2$, $R^4$ to $R^5$ and A have the meanings according to claim 1.

5. Compounds according to claim 1, **characterised in that** $R^3$ denotes an aryl radical with optionally heteroatoms in the ring system and the substituents $R^6$ to $R^8$ on the aryl ring, and $R^1$, $R^2$, $R^4$ to $R^5$ and A have the meanings according to claim 1.

6. Compounds according to claim 1, **characterised in that** A denotes a radical from the group of substituted phenyl of the formula XI

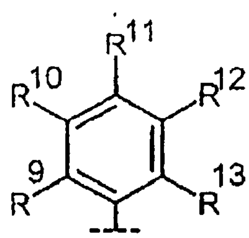

XI,

wherein

$R^9$ to $R^{13}$ denote, in each case independently of one another, H, F, Cl, Br, I, $CF_3$, OH, $OR^{14}$, $OCF_3$ $SR^{14}$, $SO_2CH_3$, $SO_2CF_3$, $C_{1-6}$-alkyl, phenyl, CN, $COOR^{14}$, $NO_2$ or $R^9$ and $R^{10}$ or $R^{10}$ and $R^{11}$ together form a $OCH_2O$ or $OCH_2CH_2O$ ring,

$R^{14}$ denotes $C_{1-6}$ alkyl, phenyl, benzyl, phenethyl, or

A denotes an unsubstituted or substituted thiophene radical or an unsubstituted or substituted furan radical, and $R^1$ to $R^5$ have the meanings according to claim 1.

**7.** Compounds according to claim 1, **characterised in that** $R^1$ and $R^2$ together form a $(CH_2)_{2-6}$ ring that may be substituted by phenyl, $R^3$ denotes a substituted $C_{1-3}$ alkylphenyl of the formula XII, and $R^4$ to $R^5$ and A have the meanings according to claim 1.

**8.** Compounds according to claim 1, **characterised in that** $R^1$ and $R^2$ together form a $(CH_2)_{2-6}$ ring that may be substituted by phenyl, $R^3$ denotes an aryl radical with optionally heteroatoms in the ring system and the substituents $R^6$ to $R^8$ on the aryl ring, and $R^4$ to $R^5$ and A have the meanings according to claim 1.

**9.** Compounds according to claim 1, **characterised in that** $R^1$ and $R^2$ together form a $(CH_2)_4$ ring that may be substituted by phenyl, A denotes a radical from the group of substituted phenyl of the formula XI or an unsubstituted or substituted thiophene radical or an unsubstituted or substituted furan radical, $R^3$ denotes a substituted $C_{1-3}$ alkylphenyl of the formula XII, and $R^4$ to $R^5$ have the meanings according to claim 1.

**10.** Compounds according to claim 1, **characterised in that** $R^1$ and $R^2$ together form a $(CH_2)_4$ ring that may be substituted by phenyl, A denotes a radical from the group of substituted phenyl of the formula XI or an unsubstituted or substituted thiophene radical or an unsubstituted or substituted furan radical, $R^3$ denotes an aryl radical with optionally heteroatoms in the ring system and the substituents $R^6$ to $R^8$ on the aryl ring, and $R^4$ to $R^5$ have the meanings according to claim 1.

**11.** Compounds according to claim 1, **characterised in that** $R^1$ and $R^2$ together form a $(CH_2)_4$ ring, A denotes a radical from the group of substituted phenyl of the formula XI or an unsubstituted or substituted thiophene radical, $R^3$ denotes a substituted $C_{1-3}$ alkylphenyl of the formula XII, and $R^4$ to $R^5$ have the meanings according to the definition of claim 1.

**12.** Compounds according to claim 1, **characterised in that** $R^1$ and $R^2$ together form a $(CH_2)_4$ ring, A denotes a radical from the group of substituted phenyl of the formula XI or an unsubstituted or substituted thiophene radical, $R^3$ denotes an aryl radical with optionally heteroatoms in the ring system and the substituents $R^6$ to $R^8$ on the aryl ring, and $R^4$ to $R^5$ have the meanings according to the definitions of claim 1.

**13.** Compounds according to claim 1, **characterised in that** $R^1$ and $R^2$ together form a $(CH_2)_4$ ring, A denotes an unsubstituted or substituted thiophene radical, $R^3$ denotes a substituted $C_{1-3}$alkylphenyl of the formula XII, and $R^4$ to $R^5$ have the meanings according to the definitions of claim 1.

**14.** Compounds according to claim 1, **characterised in that** $R^1$ and $R^2$ together form a $(CH_2)_4$ ring, A denotes an unsubstituted or substituted thiophene radical, $R^3$ denotes an aryl radical with optionally heteroatoms in the ring system and the substituents $R^6$ to $R^8$ on the aryl ring, and $R^4$ to $R^5$ have the meanings according to the definitions of claim 1.

**15.** Compounds according to claim 1, **characterised in that** $R^1$ and $R^2$ together form a $(CH_2)_4$ ring, A denotes an unsubstituted or substituted furan radical, $R^3$ denotes a substituted $C_{1-3}$ alkylphenyl of the formula XII, and $R^4$ to $R^5$ have the meanings according to the definitions of claim 1.

**16.** Compounds according to claim 1, **characterised in that** $R^1$ and $R^2$ together form a $(CH_2)_4$ ring, A denotes an unsubstituted or substituted furan radical, $R^3$ denotes an aryl radical with optionally heteroatoms in the ring system and the substituents $R^6$ to $R^8$ on the aryl ring, and $R^4$ to $R^5$ have the meanings according to the definitions of claim 1.

**17.** Compounds according to claim 1:

2-(dimethylaminophenylmethyl)-1-(3-methoxyphenyl) cyclohexanol and the corresponding hydrochloride

2-(dimethylaminophenylmethyl)-1-(3-fluorophenyl) cyclohexanol and the corresponding hydrochloride

2-(dimethylaminophenylmethyl)-1-phenylcyclohexanol and the corresponding hydrochloride

3-[2-(dimethylaminophenylmethyl)-1-hydroxycyclohexyl] phenol and the corresponding hydrochloride

2-(dimethylaminophenylmethyl)-1-(4-methoxyphenyl) cyclohexanol and the corresponding hydrochloride

1-(4-chlorophenyl)-2-(dimethylaminophenylmethyl) cyclohexanol and the corresponding hydrochloride

2-(dimethylaminophenylmethyl)-1-(4-fluorophenyl) cyclohexanol and the corresponding hydrochloride

2-(dimethylaminophenylmethyl)-1-p-tolylcyclohexanol and the corresponding hydrochloride

1-(3-chlorophenyl)-2-[dimethylamino-(3-methoxyphenyl)-methyl]cyclohexanol and the corresponding hydrochloride

1-(4-dimethylaminophenyl)-2-(dimethylaminophenylmethyl] cyclohexanol and the corresponding hydrochloride

1-benzo[1,3]dioxol-4-yl-2-(dimethylaminophenylmethyl)-cyclohexanol and the corresponding hydrochloride

1-(3,4-dimethoxyphenyl)-2-(dimethylaminophenylmethyl)-cyclohexanol and the corresponding hydrochloride

2-(dimethylaminophenylmethyl)-1-(3-methoxybenzyl)-cyclohexanol and the corresponding hydrochloride

1-benzyl-2-(dimethylaminophenylmethyl)cyclohexanol and the corresponding hydrochloride

2-(dimethylaminophenylmethyl)-1-(4-fluoro-3-trifluoromethylphenyl)cyclohexanol and the corresponding hydrochloride

2-(dimethylaminophenylmethyl)-1-(4-trifluoromethoxybenzyl)cyclohexanol and the corresponding hydrochloride

2-(dimethylaminophenylmethyl)-1-furan-3-ylcyclohexanol and the corresponding hydrochloride

1-butyl-2-(dimethylaminophenylmethyl)cyclohexanol and the corresponding hydrochloride

1-(3,4-dichlorophenyl)-2-(dimethylaminophenylmethyl)-cyclohexanol and the corresponding hydrochloride

(+)-1-(3,4-dichlorophenyl)-2-(dimethylaminophenyl-methyl)cyclohexanol and the corresponding hydrochloride

(-) -1-(3,4-dichlorophenyl)-2-(dimethylaminophenyl-methyl)cyclohexanol and the corresponding hydrochloride

4-[2-(dimethylaminophenylmethyl)-1-hydroxycyclohexyl]-phenol and the corresponding hydrochloride

2-(dimethylaminophenylmethyl)-1-naphthalen-2-ylcyclohexanol] and the corresponding hydrochloride

2-[dimethylamino-(4-trifluoromethylphenyl)methyl]-1-(3-methoxybenzyl)cyclohexanol and the corresponding hydrochloride

1-(4-chlorobenzyl)-2-(dimethylaminophenylmethyl)-1-cyclohexanol and the corresponding hydrochloride

2-(dimethylaminophenylmethyl)-1-(2-fluorobenzyl) cyclohexanol and the corresponding hydrochloride

2-(dimethylaminophenylmethyl)-1-(4-fluorobenzyl) cyclohexanol and the corresponding hydrochloride

1-(2,5-dimethoxyphenyl)-2-(dimethylaminophenylmethyl)-cyclohexanol and the corresponding hydrochloride

1-(2-chloro-4-fluorobenzyl)-2-(dimethylaminophenyl-methyl)cyclohexanol and the corresponding hydrochloride

1-(4-tert.-butylbenzyl)-2-(dimethylaminophenylmethyl)-cyclohexanol and the corresponding hydrochloride

2-(dimethylaminophenylmethyl)-1-(3-fluorobenzyl) cyclohexanol and the corresponding hydrochloride

1-(2-chlorobenzyl)-2-(dimethylaminophenylmethyl) cyclohexanol and the corresponding hydrochloride

1-benzo[1,3]dioxol-5-yl-2-[dimethylamino(3-methoxyphenyl)methyl]cyclohexanol and the corresponding hydrochloride

1-(3-chlorobenzyl)-2-(dimethylaminophenylmethyl) cyclohexanol and the corresponding hydrochloride

1-(2,4-dichlorobenzyl)-2-(dimethylaminophenylmethyl)-cyclohexanol and the corresponding hydrochloride

1-benzyl-2-[dimethylaminophenyl-(3-phenoxyphenyl)-methyl]cyclohexanol and the corresponding hydrochloride

1-benzyl-2-[dimethylaminophenyl-(3-methoxyphenyl)-methyl]cyclohexanol and the corresponding hydrochloride

2-(dimethylaminophenylmethyl)-1-(3-trifluoromethylbenzyl)cyclohexanol and the corresponding hydrochloride

2-(dimethylamino-(3-methoxyphenyl)methyl]-1-(3-methoxybenzyl)cyclohexanol and the corresponding hydrochloride

2-[(2-chlorophenyl) dimethylaminomethyl]-1-naphthalen-2-ylcyclohexanol and the corresponding hydrochloride

1-benzyl-2-[(3,4-dichlorophenyl)dimethylaminomethyl]-cyclohexanol and the corresponding hydrochloride

2-[(3,4-dichlorophenyl)dimethylaminomethyl]-1-phenethyl-cyclohexanol and the corresponding hydrochloride

1-benzyl-2-[dimethylamino-(4-fluorophenyl)methyl] cyclohexanol and the corresponding hydrochloride

2-[(3-chlorophenyl)dimethylaminomethyl]-1-phenyl-cyclohexanol and the corresponding hydrochloride

1-(2,4-dichlorophenyl)-2-(3-dimethylaminomethyl)-1-cyclohexanol and the corresponding hydrochloride

1-benzyl-2-[(3-chlorophenyl)dimethylaminomethyl] cyclohexanol and the corresponding hydrochloride

1-benzyl-2-[(2-chlorophenyl)dimethylaminomethyl] cyclohexanol and the corresponding hydrochloride

1-(4-tert.-butylbenzyl)-2-[(3,4-dichlorophenyl) dimethylaminomethyl)cyclohexanol and the corresponding hydrochloride

2-[dimethylamino-(4-fluorophenyl)methyl]-1-(3-trifluoromethylbenzyl)cyclohexanol and the corresponding hydrochloride

2-(dimethylaminophenylmethyl)bicyclohexyl-1-ol and the corresponding hydrochloride

2-(dimethylaminophenylmethyl)-1-(4-methoxybenzyl)-cyclohexanol and the corresponding hydrochloride

1-(2,4-difluorobenzyl)-2-(dimethylaminophenylmethyl)-cyclohexanol and the corresponding hydrochloride

1-(4-tert.-butylbenzyl)-2-[(3-chlorophenyl)dimethyl-aminomethyl]cyclohexanol and the corresponding hydrochloride

2-[dimethylamino-(3-phenoxyphenyl)methyl]-1-phenethyl-cyclohexanol and the corresponding hydrochloride

2-[dimethylamino-(3-phenoxyphenyl)methyl]-1-(3-trifluoromethylbenzyl)cyclohexanol and the corresponding hydrochloride

1-(2,5-difluorobenzyl)-2-(dimethylaminophenylmethyl) cyclohexanol and the corresponding hydrochloride

1-(3,4-difluorobenzyl)-2-(dimethylaminophenylmethyl) cyclohexanol and the corresponding hydrochloride

1-(2-chloro-6-fluorobenzyl)-2-(dimethylaminophenylmethyl)cyclohexanol and the corresponding hydrochloride

1-(2,3-difluorobenzyl)-2-(dimethylaminophenylmethyl) cyclohexanol and the corresponding hydrochloride

1-benzyl-2-[(4-chlorophenyl)dimethylaminomethyl] cyclohexanol and the corresponding hydrochloride

1-dimethylamino-3-ethyl-2-methyl-1,5-diphenylpentane-3-ol and the corresponding hydrochloride

1-(2-chlorobenzyl)-2-[(2-chlorophenyl)-dimethylaminomethyl]cyclohexanol and the corresponding hydrochloride

1-benzyl-2-[(4-bromophenyl)dimethylaminomethyl] cyclohexanol and the corresponding hydrochloride

2-[(4-chlorophenyl)dimethylaminomethyl]-1-(4-trifluoro-methylphenyl)cyclohexanol and the corresponding hydrochloride

2-[(4-chlorophenyl)dimethylaminomethyl]-1-(3-trifluoro-methylbenzyl)cyclohexanol and the corresponding hydrochloride

1-(4-tert.-butylbenzyl)-2-[dimethylamino-(3-phenoxyphenyl)methyl]cyclohexanol and the corresponding hydrochloride

4-{dimethylamino-[2-hydroxy-2-(4-trifluoromethylphenyl)cyclohexyl]methyl}benzonitrile and the corresponding hydrochloride

2-(dimethylamino-o-tolylmethyl)-1-phenylcyclohexanol and the corresponding hydrochloride

1-benzyl-2-(dimethylamino-o-tolylmethyl)cyclohexanol and the corresponding hydrochloride

2-(dimethylaminophenylmethyl)-1-(3-phenylpropyl) cyclohexanol and the corresponding hydrochloride

2-[(2-chlorophenyl)dimethylaminomethyl]-1-[2-(4-fluoro-phenyl)ethyl]cyclohexanol and the corresponding hydrochloride

2-[dimethylaminothiophen-2-ylmethyl]-1-(3-trifluoromethylbenzyl)cyclohexanol and the corresponding hydrochloride

Methyl-4-[2-(dimethylaminophenylmethyl)-1-hydroxycyclo-hexyl]benzoate and the corresponding hydrochloride

1-benzyl-2-(dimethylaminophenylmethyl)-4-phenylcyclohexanol and the corresponding hydrochloride

1-(4-bromophenyl)-2-(dimethylaminophenylmethyl)cyclohexanol and the corresponding hydrochloride

2-(dimethylaminophenylmethyl)-1-naphthalen-1-ylcyclohexanol and the corresponding hydrochloride

2-(dimethylaminophenylmethyl)-1-(2-methylsulfanylphenyl) cyclohexanol and the corresponding hydrochloride

1-benzyl-2-(dimethylaminonaphthalen-2-ylmethyl) cyclohexanol and the corresponding hydrochloride

1-benzyl-2-(dimethylaminonapentafluorophenylmethyl) cyclohexanol and the corresponding hydrochloride

1-benzyl-2-(phenylpiperidin-1-ylmethyl)cyclohexanol and the corresponding hydrochloride

2-(dimethylaminophenylmethyl)-1-(4-trifluoromethylphenyl)cyclohexanol and the corresponding hydrochloride

3 -(4-tert.-butylbenzyl)-1-dimethylamino-2-methyl-1-phenylpentan-3-ol and the corresponding hydrochloride

2-(dimethylamino-o-tolylmethyl)-1-phenethylcyclohexanol and the corresponding hydrochloride

1-(4-tert .-butylbenzyl)-2-[dimethylaminothiophen-2-ylmethyl]cyclohexanol and the corresponding hydrochloride

**18.** Medicament containing as active agent at least one compound according to claims 1 to 17 including the excluded compounds according to claim 1.

**19.** Medicament containing as active agent a mixture of the enantiomers of a compound according to claims 1 to 17 including the excluded compounds according to claim 1, the two enantiomers not being present in equimolar amounts, and optionally further active agents.

**20.** Medicament according to claim 19, **characterised in that** one of the enantiomers is present in an amount of between 5 and 45 wt.% relative to the enantiomer mixture.

**21.** Process for preparing a compound according to claims 1 to 17, **characterised in that** a Mannich base of the general formula II

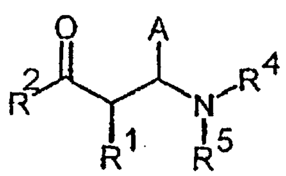

II

wherein $R^1$ to $R^5$ and A have the meanings according to the general formula I, is reacted with an organometallic compound $R^3Y$, wherein Y denotes MgCl, MgBr, MgI or Li and $R^3$ is according to formula I, to form an alcohol of the general formula I

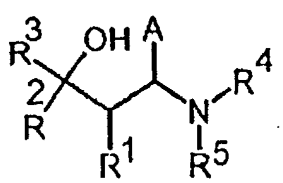

I

wherein $R^1$ to $R^5$ and A have the meanings according to formula I.

22. Use of at least one compound according to claims 1 to 17 including the compounds excluded according to claim 1 for producing a medicament to relieve pain.

23. Use according to claim 22 for producing a medicament to relieve neuropathic pain.

24. Use according to claim 22 for producing a medicament to relieve chronic pain.

25. Use of at least one compound according to claims 1 to 17 including the compounds excluded according to claim 1 for producing a medicament having a local anaesthetic effect.

26. Use of at least one compound according to claims 1 to 17 including the compounds excluded according to claim 1 for producing a medicament having an anti-arrythmic action.

27. Use of at least one compound according to claims 1 to 17 including the compounds excluded according to claim 1 for producing a medicament having an anti-emetic action.

28. Use of at least one compound according to claims 1 to 17 including the compounds excluded according to claim 1 1 for producing a medicament having a nootropic (neurotropic) action.

29. Use of at least one compound according to claims 1 to 17 including the compounds excluded according to claim 1 for producing a medicament for treating cardiovascular conditions.

30. Use of at least one compound according to claims 1 to 17 including the compounds excluded according to claim 1 for producing a medicament for treating urinary incontinence.

31. Use of at least one compound according to claims 1 to 17 including the compounds excluded according to claim 1 for producing a medicament for treating diarrhoea.

32. Use of at least one compound according to claims 1 to 17 including the compounds excluded according to claim 1 for producing a medicament for treating pruritus.

33. Use of at least one compound according to claims 1 to 17 including the compounds excluded according to claim 1 for producing a medicament for treating alcohol dependence.

34. Use of at least one compound according to claims 1 to 17 including the compounds excluded according to claim 1 for producing a medicament for treating narcotics dependence.

35. Use of at least one compound according to claims 1 to 17 including the compounds excluded according to claim 1 for producing a medicament for treating drug dependence.

36. Use of at least one compound according to claims 1 to 17 including the compounds excluded according to claim 1 for producing a medicament for treating inflammations.

37. Use of at least one compound according to claims 1 to 17 including the compounds excluded according to claim 1 for producing a medicament for treating depression.

38. Use of at least one compound according to claims 1 to 17 including the compounds excluded according to claim 1 for producing a medicament for improving libido.

39. Use of at least one compound according to claims 1 to 17 including the compounds excluded according to claim 1 for producing a medicament for improving alertness and attentiveness.

## Revendications

1. 3-amino-3-arylpropane-1-ols substitués de formule générale I

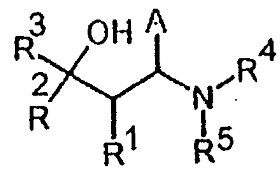

I

dans laquelle

$R^1$, $R^2$ représentent chacun, indépendamment l'un de l'autre, un reste alkyle en $C_1$ à $C_6$, ou bien $R^1$ et $R^2$ forment ensemble un noyau ayant 2 à 6 chaînons $CH_2$, qui peut aussi porter un substituant phényle,

$R^3$ est un reste alkyle en $C_3$ à $C_6$, cycloalkyle en $C_3$ à $C_6$, aryle ayant éventuellement des hétéroatomes dans le noyau et portant éventuellement sur le noyau aryle les substituants $R^6$ à $R^8$, ou bien un reste (alkyle en $C_1$ à $C_3$)-phényle substitué de formule XII

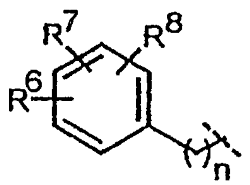

n = 1, 2 ou 3

XII,

$R^4$, $R^5$ représentent chacun, indépendammment l'un de l'autre, un reste alkyle en $C_1$ à $C_6$, cycloalkyle en $C_3$ à $C_6$, phényle, benzyle, phénéthyle ou bien $R^4$ et $R^5$ forment ensemble un noyau ayant 3 à 6 chaînons $CH_2$ ou un enchaînement $CH_2CH_2OCH_2CH_2$,

$R^6$ à $R^8$ représentent chacun, indépendamment l'un de l'autre, l'hydrogène, le fluor, le chlore, le brome, un reste $CHF_2$, $CF_3$, OH, $OCF_3$, $OR^{14}$, $NR^{15}R^{16}$, $SR^{14}$, phényle, $SO_2CH_3$, $SO_2CF_3$, alkyle en $C_1$ à $C_6$, CN, $COOR^{14}$, $CONR^{15}R^{16}$, ou bien $R^6$ et $R^7$ forment ensemble un noyau à enchaînement $OCH_2O$, $OCH_2CH_2O$, CH=CHO, $CH=C(CH_3)O$ ou $(CH_2)_4$, où

$R^{14}$ est un reste alkyle en $C_1$ à $C_6$, phényle, benzyle, phénéthyle et

$R^{15}$, $R^{16}$ représentent ensemble, indépendamment l'un de l'autre, l'hydrogène, un reste alkyle en $C_1$ à $C_6$, phényle, benzyle, phénéthyle, et

A est un reste alkyle qui contient éventuellement des hétéroatomes dans le noyau et/ou qui peut être substitué,

et leurs diastéréo-isomères ou leurs énantiomères sous forme de leurs bases ou leurs sels d'acides acceptables du point de vue physiologique, le 1-benzyl-2-(diméthylaminophénylméthyl)-cyclohexanol, ses diastéréo-isomères et ses énantiomères sous forme de leurs bases et son produit de réaction avec l'iodure de méthyle étant exclus.

2. Composés suivant la revendication 1, **caractérisés en ce que** $R^1$ et $R^2$ forment ensemble un noyau à enchaînement de deux à six chaînons $CH_2$ qui peut porter un substituant phényle, $R^3$ à $R^5$ et A ont la définition indiquée dans la revendication 1.

3. Composés suivant la revendication 1, **caractérisés en ce que** $R^1$ et $R^2$ forment ensemble un noyau à enchaînement de quatre chaînons $CH_2$ qui peut porter un substituant phényle, $R^3$ à $R^5$ et A ont la définition indiquée dans la revendication 1.

4. Composés suivant la revendication 1, **caractérisés en ce que** $R^3$ est un groupe (alkyle en $C_1$ à $C_3$)-phényle substitué de formule XII, $R^1$, $R^2$, $R^4$, $R^5$ et A ont la définition indiquée dans la revendication 1.

5. Composés suivant la revendication 1, **caractérisés en ce que** $R^3$ est un reste aryle ayant éventuellement des hétéroatomes dans le noyau et portant éventuellement sur le noyau aryle les substituants $R^6$ à $R^8$, $R^1$, $R^2$ $R^4$, et

$R^5$ et A ont les définitions indiquées dans la revendication 1.

**6.** Composés suivant la revendication 1, **caractérisés en ce que** A est un reste du groupe d'un reste phényle substitué de formule XI

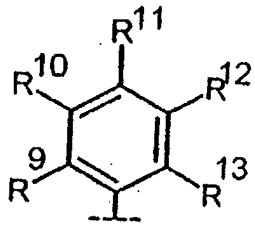

XI,

dans laquelle

$R^9$ à $R^{13}$ représentent chacun, indépendamment l'un de l'autre, l'hydrogène, le fluor, le chlore, le brome, l'iode, un reste $CF_3$, OH, $OR^{14}$, $OCF_3$, $SR^{14}$, $SO_2CH_3$, $SO_2CF_3$, alkyle en $C_1$ à $C_6$, phényle, CN, $COOR^{14}$, $NO_2$, ou bien $R^9$ et $R^{10}$ ou $R^{10}$ et $R^{11}$ forment ensemble un noyau à enchaînement $OCH_2O$ ou $OCH_2CH_2O$,

$R^{14}$ est un reste alkyle en $C_1$ à $C_6$, phényle, benzyle, phénéthyle

ou bien A est un reste thiophène non substitué ou substitué ou un reste furanne non substitué ou substitué et $R^1$ à $R^5$ ont la signification indiquée dans la revendication 1.

**7.** Composés suivant la revendication 1, **caractérisés en ce que** $R^1$ et $R^2$ forment ensemble un noyau à enchaînement de deux à six chaînons $CH_2$ qui peut porter un substituant phényle, $R^3$ est un reste (alkyle en $C_1$ à $C_3$)-phényle substitué de formule XII, $R^4$, $R^5$ et A ont la définition indiquée dans la revendication 1.

**8.** Composés suivant la revendication 1, **caractérisés en ce que** $R^1$ et $R^2$ forment un noyau à enchaînement de deux à six chaînons $CH_2$ qui peut porter un substituant phényle, $R^3$ est un reste aryle contenant éventuellement des hétéroatomes dans le noyau et portant éventuellement sur le noyau aryle les substituants $R^6$ à $R^8$, $R^4$, $R^5$ et A ont la définition indiquée dans la revendication 1.

**9.** Composés suivant la revendication 1, **caractérisés en ce que** $R^1$ et $R^2$ forment ensemble un noyau à enchaînement de quatre chaînons $CH_2$ qui peut porter un substituant phényle, A est un reste du groupe d'un reste phényle substitué de formule XI ou un reste thiophène non substitué ou substitué ou un reste furanne non substitué ou substitué, $R^3$ est un reste (alkyle en $C_1$ à $C_3$)-phényle substitué de formule XII et $R^4$ et $R^5$ ont la définition indiquée dans la revendication 1.

**10.** Composés suivant la revendication 1, **caractérisés en ce que** $R^1$ et $R^2$ forment ensemble un noyau à enchaînement de quatre chaînons $CH_2$ qui peut porter un substituant phényle, A est un reste du groupe d'un reste phényle substitué de formule XI ou un reste thiophène non substitué ou substitué ou un reste furanne non substitué ou substitué, $R^3$ est un reste aryle contenant éventuellement des hétéroatomes dans le noyau et portant éventuellement sur le noyau aryle les substituants $R^6$ à $R^8$, et $R^4$ et $R^5$ ont la définition indiquée dans la revendication 1.

**11.** Composés suivant la revendication 1, **caractérisés en ce que** $R^1$ et $R^2$ forment ensemble un noyau à enchaînement de quatre chaînons $CH_2$, A est un reste du groupe d'un reste phényle substitué de formule XI ou un reste thiophène non substitué ou substitué, $R^3$ est un reste (alkyle en $C_1$ à $C_3$)-phényle substitué de formule XII, $R^4$ et $R^5$ ont la signification suivant la définition de la revendication 1.

**12.** Composés suivant la revendication 1, **caractérisés en ce que** $R^1$ et $R^2$ forment ensemble un noyau à enchaînement de quatre chaînons $CH_2$, A représente un reste du groupe d'un reste phényle substitué de formule XI ou un reste thiophène non substitué ou substitué, $R^3$ est un reste aryle contenant éventuellement des hétéroatomes dans le noyau et portant éventuellement sur le noyau aryle les substituants $R^6$ à $R^8$, $R^4$ et $R^5$ ont la signification

suivant la définition de la revendication 1.

**13.** Composés suivant la revendication 1, **caractérisés en ce que** $R^1$ et $R^2$ forment ensemble un noyau à enchaînement de quatre chaînons $CH_2$, A est un reste thiophène non substitué ou substitué, $R^3$ est un reste (alkyle en $C_1$ à $C_3$)-phényle substitué de formule XII, $R^4$ et $R^5$ ont la signification selon la définition de la revendication 1.

**14.** Composés suivant la revendication 1, **caractérisés en ce que** $R^1$ et $R^2$ forment ensemble un noyau à enchaînement de quatre chaînons $CH_2$, A est un reste thiophène non substitué ou substitué, $R^3$ est un reste aryle contenant éventuellement des hétéroatomes dans le noyau et portant éventuellement sur le noyau aryle les substituants $R^6$ à $R^8$, $R^4$ et $R^5$ ont la signification selon la définition de la revendication 1.

**15.** Composés suivant la revendication 1, **caractérisés en ce que** $R^1$ et $R^2$ forment ensemble un noyau à enchaînement de quatre chaînons $CH_2$, A est un reste furanne non substitué ou substitué, $R^3$ est un reste (alkyle en $C_1$ à $C_3$)-phényle substitué de formule XII, $R^4$ et $R^5$ ont la signification selon la définition de la revendication 1.

**16.** Composés suivant la revendication 1, **caractérisés en ce que** $R^1$ et $R^2$ forment ensemble un noyau à enchaînement de quatre chaînons $CH_2$, A est un reste furanne non substitué ou substitué, $R^3$ est un reste aryle contenant éventuellement des hétéroatomes dans le noyau et portant éventuellement sur le noyau aryle les substituants $R^6$ à $R^8$, $R^4$ et $R^5$ ont la signification suivant la définition de la revendication 1.

**17.** Composés suivant la revendication 1, comprenant les composés suivants :

2-(diméthylaminophénylméthyl)-1-(3-méthoxyphényl)-cyclohexanol et le chlorhydrate correspondant
2-(diméthylaminophénylméthyl)-1-(3-fluorophényl)-cyclohexanol et le chlorhydrate correspondant
2-(diméthylaminophénylméthyl)-1-phénylcyclohexanol et le chlorhydrate correspondant
3-[2-(diméthylaminophénylméthyl)-1-hydroxycyclohexyl]-phénol et le chlorhydrate correspondant
2-(diméthylaminophénylméthyl)-1-(4-méthoxyphényl)-cyclohexanol et le chlorhydrate correspondant
1-(4-chlorophényl)-2-(diméthylaminophénylméthyl)-cyclohexanol et le chlorhydrate correspondant
2-(diméthylaminophénylméthyl)-1-(4-fluorophényl)-cyclohexanol et le chlorhydrate correspondant
2-(diméthylaminophénylméthyl)-1-p-tolylcyclohexanol et le chlorhydrate correspondant
1-(3-chlorophényl)-2-[diméthylamino-(3-méthoxyphényl)-méthyl]cyclohexanol et le chlorhydrate correspondant
1-(4-diméthylaminophényl)-2-[diméthylaminophénylméthyl]-cyclohexanol et le chlorhydrate correspondant
1-benzo[1,3] dioxole-4-yl-2-(diméthylaminophénylméthyl)-cyclohexanol et le chlorhydrate correspondant
1-(3,4-diméthoxyphényl)-2-(diméthylaminophénylméthyl)-cyclohexanol et le chlorhydrate correspondant
2-(diméthylaminophénylméthyl)-1-(3-méthoxybenzyl)-cyclohexanol et le chlorhydrate correspondant
chlorhydrate de 1-benzyl-2-(diméthylaminophénylméthyl)-cyclohexanol
2-(diméthylaminophénylméthyl)-1-(4-fluoro-3-trifluorométhylphényl)-cyclohexanol et le chlorhydrate correspondant
2-(diméthylaminophénylméthyl)-1-(4-trifluorométhoxybenzyl)-cyclohexanol et le chlorhydrate correspondant
2-(diméthylaminophénylméthyl)-1-furanne-3-ylcyclohexanol et le chlorhydrate correspondant
1-butyl-2-(diméthylaminophénylméthyl)-cyclohexanol et le chlorhydrate correspondant
1-(3,4-dichlorophényl)-2-(diméthylaminophénylméthyl)-cyclohexanol et le chlorhydrate correspondant
(+)-1-(3,4-dichlorophényl)-2-(diméthylaminophénylméthyl)-cyclohexanol et le chlorhydrate correspondant
(-)-1-(3,4-dichlorophényl)-2-(diméthylaminophénylméthyl)-cyclohexanol et le chlorhydrate correspondant
4-[2-(diméthylaminophénylméthyl)-1-hydroxycyclohexyl]-phénol et le chlorhydrate correspondant
2-(diméthylaminophénylméthyl)-1-naphtalène-2-ylcyclohexanol et le chlorhydrate correspondant
2-[diméthylamino-(4-trifluorométhylphényl)-méthyl]-1-(3-méthoxybenzyl)-cyclohexanol et le chlorhydrate correspondant
1-(4-chlorobenzyl)-2-(diméthylaminophénylméthyl)-1-cyclohexanol et le chlorhydrate correspondant
2-(diméthylaminophénylméthyl)-1-(2-fluorobenzyl)-cyclohexanol et le chlorhydrate correspondant
2-(diméthylaminophénylméthyl)-1-(4-fluorobenzyl)-cyclohexanol et le chlorhydrate correspondant
1-(2,5-diméthoxyphényl)-2-(diméthylaminophénylméthyl)-cyclohexanol et le chlorhydrate correspondant
1-(2-chloro-4-fluorobenzyl)-2-(diméthylaminophénylméthyl)-cyclohexanol et le chlorhydrate correspondant
1-(4-tertiobutylbenzyl)-2-(diméthylaminophénylméthyl)-cyclohexanol et le chlorhydrate correspondant
2-(diméthylaminophénylméthyl)-1-(3-fluorobenzyl)-cyclohexanol et le chlorhydrate correspondant
1-(2-chlorobenzyl)-2-(diméthylaminophénylméthyl)-cyclohexanol et le chlorhydrate correspondant
1-benzo[1,3]dioxole-5-yl-2-[diméthylamino-(3-méthoxyphényl)-méthyl]-cyclohexanol et le chlorhydrate correspondant
1-(3-chlorobenzyl)-2-(diméthylaminophénylméthyl)-cyclohexanol et le chlorhydrate correspondant
1-(2,4-dichlorobenzyl)-2-(diméthylaminophénylméthyl)-cyclohexanol et le chlorhydrate correspondant
1-benzyl-2-[diméthylaminophényl-(3-phénoxyphényl)-méthyl]-cyclohexanol et le chlorhydrate correspondant
1-benzyl-2-[diméthylaminophényl-(3-méthoxyphényl)-méthyl]-cyclohexanol et le chlorhydrate correspondant
2-(diméthylaminophénylméthyl)-1-(3-trifluorométhylbenzyl)-cyclohexanol et le chlorhydrate correspondant
2-[diméthylamino-(3-méthoxyphényl)-méthyl]-1-(3-méthoxybenzyl)-cyclohexanol et le chlorhydrate correspondant
2-[(2-chlorophényl)-diméthylaminométhyl]-1-naphtalène-2-ylcyclohexanol et le chlorhydrate correspondant
1-benzyl-2-[(3,4-dichlorophényl)-diméthylaminométhyl]-cyclohexanol et le chlorhydrate correspondant
2-[(3,4-dichlorophényl)-diméthylaminométhyl]-1-phénéthylcyclohexanol et le chlorhydrate correspondant
1-benzyl-2-[diméthylamino-(4-fluorophényl)-méthyl]-cyclohexanol et le chlorhydrate correspondant
2-[(3-chlorophényl)-diméthylaminométhyl]-1-phénylcyclohexanol et le chlorhydrate correspondant
1-(2,4-dichlorophényl)-2-(3-diméthylaminophénylméthyl)-1-cyclo-hexanol et le chlorhydrate correspondant
1-benzyl-2-[(3-chlorophényl)-diméthylaminométhyl]-cyclohexanol et le chlorhydrate correspondant
1-benzyl-2-[(2-chlorophényl)-diméthylaminométhyl]-cyclohexanol et le chlorhydrate correspondant
1-(4-tertiobutylbenzyl)-2-[(3,4-dichlorophényl)-diméthylaminométhyl]-cyclohexanol et le chlorhydrate correspondant
2-[diméthylamino-(4-fluorophényl)-méthyl]-1-(3-trifluorométhylbenzyl)-cyclohexanol et le chlorhydrate correspondant
2-(diméthylaminophénylméthyl)-bicyclohexyl-1-ol et le chlorhydrate correspondant
2-(diméthylaminophénylméthyl)-1-(4-méthoxybenzyl)-cyclohexanol et le chlorhydrate correspondant
1-(2,4-difluorobenzyl)-2-(diméthylaminophénylméthyl)-cyclohexanol et le chlorhydrate correspondant
1-(4-tertiobutylbenzyl)-2-[(3-chlorophényl)-diméthylaminométhyl]-cyclohexanol et le chlorhydrate correspondant
2-[diméthylamino-(3-phénoxyphényl)-méthyl]-1-phénéthylcyclohexanol et le chlorhydrate correspondant
2-[diméthylamino-(3-phénoxyphényl)-méthyl]-1-(3-trifluorométhylbenzyl)-cyclohexanol et le chlorhydrate correspondant
1-(2,5-difluorobenzyl)-2-(diméthylaminophénylméthyl)-cyclohexanol et le chlorhydrate correspondant
1-(3,4-difluorobenzyl)-2-(diméthylaminophénylméthyl)-cyclohexanol et le chlorhydrate correspondant
1-(2-chloro-6-fluorobenzyl)-2-(diméthylaminophénylméthyl)-cyclohexanol et le chlorhydrate correspondant
1-(2,3-difluorobenzyl)-2-(diméthylaminophénylméthyl)-cyclohexanol et le chlorhydrate correspondant
1-benzyl-2-[(4-chlorophényl)-diméthylaminométhyl]-cyclohexanol et le chlorhydrate correspondant
1-diméthylamino-3-éthyl-2-méthyl-1,5-diphénylpentane-3-ol et le chlorhydrate correspondant
1-(2-chlorobenzyl)-2-[(2-chlorophényl)-diméthylaminométhyl]-cyclohexanol et le chlorhydrate correspondant
1-benzyl-2-[(4-bromophényl)-diméthylaminométhyl]-cyclohexanol et le chlorhydrate correspondant
2-[(4-chlorophényl)-diméthylaminométhyl]-1-(4-trifluorométhylphényl)-cyclohexanol et le chlorhydrate correspondant
2-[(4-chlorophényl)-diméthylaminométhyl]-1-(3-trifluorométhylbenzyl)-cyclohexanol et le chlorhydrate correspondant
1-(4-tertiobutylbenzyl)-2-[diméthylamino-(3-phénoxyphényl)-méthyl]-cyclohexanol et le chlorhydrate correspondant
4-{diméthylamino-[2-hydroxy-2-(4-trifluorométhylphényl)-cyclohexyl]-méthyl}-benzonitrile et le chlorhydrate correspondant
2-(diméthylamino-o-tolylméthyl)-1-phénylcyclohexanol et le chlorhydrate correspondant
1-benzyl-2-(diméthylamino-o-tolylméthyl)-cyclohexanol et le chlorhydrate correspondant
2-(diméthylaminophénylméthyl)-1-(3-phénylpropyl)-cyclohexanol et le chlorhydrate correspondant
2-[(2-chlorophényl)-diméthylaminométhyl]-1-[2-(4-fluorophényl)-éthyl]-cyclohexanol et le chlorhydrate correspondant
2-[diméthylaminothiophène-2-ylméthyl]-1-(3-trifluorométhylbenzyl)-cyclohexanol et le chlorhydrate correspondant
4-[2-(diméthylaminophénylméthyl)-1-hydroxycyclohexyl]-benzoate de méthyle et le chlorhydrate correspondant
1-benzyl-2-(diméthylaminophénylméthyl)-4-phénylcyclohexanol et le chlorhydrate correspondant
1-(4-bromophényl)-2-(diméthylaminophénylméthyl)-cyclohexanol et le chlorhydrate correspondant
2-(diméthylaminophénylméthyl)-1-naphtalène-1-ylcyclohexanol et le chlorhydrate correspondant
2-(diméthylaminophénylméthyl)-1-(2-méthylsulfanylphényl)-cyclohexanol et le chlorhydrate correspondant
1-benzyl-2-(diméthylaminonaphtalène-2-ylméthyl)-cyclohexanol et le chlorhydrate correspondant

1-benzyl-2-(diméthylaminopentafluorophénylméthyl)-cyclohexanol et le chlorhydrate correspondant
1-benzyl-2-(phénylpipéridine-1-ylméthyl)-cyclohexanol et le chlorhydrate correspondant
2-(diméthylaminophénylméthyl)-1-(4-trifluorométhylphényl)-cyclohexanol et le chlorhydrate correspondant
3-(4-tertiobutylbenzyl)-1-diméthylamino-2-méthyl-1-phénylpentane-3-ol et le chlorhydrate correspondant
2-(diméthylamino-o-tolylméthyl)-1-phénéthylcyclohexanol et le chlorhydrate correspondant
1-(4-tertiobutylbenzyl)-2-[diméthylaminothiophène-2-yl-méthyl]-cyclohexanol et le chlorhydrate correspondant.

**18.** Médicament contenant comme substance active au moins un composé suivant les revendication 1 à 17, y compris les composés exceptés selon la revendication 1.

**19.** Médicament contenant comme substances actives un mélange des énantiomères d'un composé suivant les revendications 1 à 17, y compris des composés exceptés selon la revendication 1, les deux énantiomères n'étant pas présents en quantités équimolaires, et le cas échéant d'autres substances actives.

**20.** Médicament suivant la revendication 19, **caractérisé en ce que** l'un des énantiomères est en une proportion relative du mélange d'énantiomères entre 5 et 45 % en masse.

**21.** Procédé de production d'un composé suivant l'une des revendications 1 à 17, **caractérisé en ce qu'**on fait réagir une base de Mannich de formule générale II

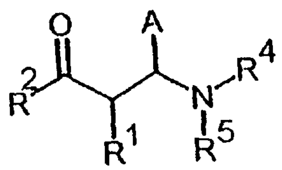

II

dans laquelle $R^1$ à $R^5$ et A ont la définition donnée pour la formule générale I, avec des composés organométalliques $R^3Y$ où Y représente MgCl, MgBr, MgI ou Li et $R^3$ a la définition donnée pour la formule I, pour former un alcool de formule générale I

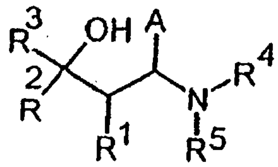

I

dans laquelle $R^1$ à $R^5$ et A ont la définition suivant la formule I.

**22.** Utilisation d'au moins un composé suivant les revendications 1 à à 17, y compris des composés exceptés selon la revendication 1 pour la préparation d'un médicament destiné à combattre la douleur.

**23.** Utilisation suivant la revendication 22, pour la préparation d'un médicament destiné à combattre des douleurs névropathiques.

**24.** Utilisation suivant la revendication 22, pour la préparation d'un médicament destiné à combattre des douleurs chroniques.

**25.** Utilisation d'au moins un composé suivant les revendications 1 à 17, y compris des composés exceptés selon la revendication 1 pour la préparation d'un médicament à action anesthésique locale.

**26.** Utilisation d'au moins un composé suivant les revendications 1 à 17, y compris des composés exceptés selon la revendication 1, pour la préparation d'un médicament à action anti-arythmique.

**27.** Utilisation d'au moins un composé suivant les revendications 1 à 17, y compris des composés exceptés selon la revendication 1, pour la préparation d'un médicament à action anti-émétique.

**28.** Utilisation d'au moins un composé suivant les revendications 1 à 17, y compris des composés exceptés selon la revendication 1, pour la préparation d'un médicament à action nootrope (neurotrope).

**29.** Utilisation d'au moins un composé suivant les revendications 1 à 17, y compris des composés exceptés selon la revendication 1, pour la préparation d'un médicament destiné au traitement de maladies cardiovasculaires.

**30.** Utilisation d'au moins un composé suivant les revendications 1 à 17, y compris des composés exceptés selon la revendication 1, pour la préparation d'un médicament destiné au traitement de l'incontinence urinaire.

**31.** Utilisation d'au moins un composé suivant les revendications 1 à 17, y compris des composés exceptés selon la revendication 1, pour la préparation d'un médicament destiné au traitement de la diarrhée.

**32.** Utilisation d'au moins un composé suivant les revendications 1 à 17, y compris des composés exceptés selon la revendication 1, pour la préparation d'un médicament destiné au traitement du prurit.

**33.** Utilisation d'au moins un composé suivant les revendications 1 à 17, y compris des composés exceptés selon la revendication 1, pour la préparation d'un médicament destiné au traitement de la dépendance à l'alcool.

**34.** Utilisation d'au moins un composé suivant les revendications 1 à 17, y compris des composés exceptés selon la revendication 1, pour la préparation d'un médicament destiné au traitement de la dépendance à la drogue.

**35.** Utilisation d'au moins un composé suivant les revendications 1 à 17, y compris des composés exceptés selon la revendication 1, pour la préparation d'un médicament destiné au traitement de la dépendance à l'égard de médicaments.

**36.** Utilisation d'un composé suivant les revendications 1 à 17, y compris des composés exceptés selon la revendication 1, pour la préparation d'un médicament destiné au traitement d'états inflammatoires.

**37.** Utilisation d'au moins un composé suivant les revendications 1 à 17, y compris des composés exceptés selon la revendication 1, pour la préparation d'un médicament destiné au traitement de dépressions.

**38.** Utilisation d'au moins un composé suivant les revendications 1 à 17, y compris des composés exceptés selon la revendication 1, pour la préparation d'un médicament destiné à accroître la libido.

**39.** Utilisation d'au moins un composé suivant les revendications 1 à 17, y compris des composés exceptés selon la revendication 1, pour la préparation d'un médicament destiné à accroître la vigilance.